# EUROPEAN PATENT SPECIFICATION

(11) **EP 1 457 564 B1**
(45) Date of publication and mention of the grant of the patent: **06.01.2010**
(21) Application number: 04006087.3
(22) Date of filing: 15.03.2004
(51) Int. Cl.: C12N 15/82, A01H 5/00, A01H 5/02, A01N 3/00

(54) **Production of plants having improved rooting efficiency and vase life using stress-resistance gene**
Herstellung von Pflanzen mit verbesserter Wurzelbildung und verbesserter Vasen-Lebensdauer durch Verwendung von Stressresistenzgenen
Production de plantes avec l'enracinement et la vie en vase améliorée par l'utilisation de gènes de résistance aux stress

(30) Priority: 14.03.2003 JP 2003071082
(43) Date of publication of application: 15.09.2004
(73) Proprietor: Japan International Research Center for Agricultural Sciences, Tsukuba-shi, Ibaraki 305-8686 (JP); Kirin Holdings Kabushiki Kaisha, Tokyo (JP)
(72) Inventor: Shinozaki, Kazuko, Tsukuba-shi Ibaraki 305-0031 (JP); Umemoto, Naoyuki, Kirin Holdings Kabushiki Kaisha, Saotome, Sakura-shi Tochigi 329-1414 (JP); Mamiya, Kanji, Kirin Holdings Kabushiki Kaisha, Saotome, Sakura-shi Tochigi 329-1414 (JP); Toguri, Toshihiro, Kirin Holdings Kabushiki Kaisha, Kanazawa-ku, Yokohama-shi, Kanagawa 236-0004 (JP)
(74) Representative: HOFFMANN EITLE

(56) References cited:
- WO-A-02/34034
- WO-A-99/53016
- CA-A- 2 269 105
- KASUGA MIE ET AL: "Improving plant drought, salt, and freezing tolerance by gene transfer of a single stress-inducible transcription factor" NATURE BIOTECHNOLOGY, NATURE PUB. CO, NEW YORK, NY, US, vol. 17, no. 3, March 1999 (1999-03), pages 287-291, XP002173128 ISSN: 1087-0156
- WINICOV ILGA: "Alfin1 transcription factor overexpression enhances plant root growth under normal and saline conditions and improves salt tolerance in alfalfa" PLANTA (BERLIN), vol. 210, no. 3, February 2000 (2000-02), pages 416-422, XP002290292 ISSN: 0032-0935
- LIU QIANG ET AL: "Two transcription factors, DREB1 and DREB2, with an EREBP/AP2 DNA binding domain separate two cellular signal transduction pathways in drought-and low-temperature-responsive gene expression, respectively, in Arabidopsis" PLANT CELL, AMERICAN SOCIETY OF PLANT PHYSIOLOGISTS, ROCKVILLE, MD, US, vol. 10, no. 8, August 1998 (1998-08), pages 1391-1406, XP002145075 ISSN: 1040-4651
- KANG JOUNG-YOUN ET AL: "Arabidopsis basic leucine zipper proteins that mediate stress-responsive abscisic acid signaling" PLANT CELL, AMERICAN SOCIETY OF PLANT PHYSIOLOGISTS, ROCKVILLE, MD, US, vol. 14, no. 2, February 2002 (2002-02), pages 343-357, XP002204934 ISSN: 1040-4651
- DUBOUZET J G ET AL: "OsDREB genes in rice, Oryza sativa L., encode transcription activators that function in drought-, high-salt- and cold-responsive gene expression" PLANT JOURNAL, BLACKWELL SCIENTIFIC PUBLICATIONS, OXFORD, GB, vol. 33, no. 4, February 2003 (2003-02), pages 751-763, XP002264239 ISSN: 0960-7412
- KOSUGI YUSUKE ET AL: "Senescence and gene expression of transgenic non-ethylene-producing carnation flowers" JOURNAL OF THE JAPANESE SOCIETY FOR HORTICULTURAL SCIENCE, vol. 71, no. 5, September 2002 (2002-09), pages 638-642, XP008033430 ISSN: 0013-7626

### SEQUENCE LISTING

<110> Kirin Beer Kabushiki Kaisha; Japan International Research Center f or Agricultural Sciences
<120> A production of plants having improved rooting efficiency and vase life by using environmental stress-resistant gene
<130> PH-2034
<150> JP 2003-071082
   <151> 2003-03-14
<160> 30
<210> 1
   <211> 933
   <212> DNA
   <213> Arabidopsis thaliana
<220>
   <221> CDS
   <222> (119) .. (766)
<400> 1
   cctgaactag aacagaaaga gagagaaact attatttcag caaaccatac caacaaaaaa 60
   gacagagatc ttttagttac cttatccagt ttcttgaaac agagtactct tctgatca 118
   atg aac tca ttt tct gct ttt tct gaa atg ttt ggc tcc gat tac gag 166
   Met Asn Ser Phe Ser Ala Phe Ser Glu Met Phe Gly Ser Asp Tyr Glu
   1 5 10 15
   tct tcg gtt tcc tca ggc ggt gat tat att ccg acg ctt gcg agc agc 214
   Ser Ser Val Ser Ser Gly Gly Asp Tyr Ile Pro Thr Leu Ala Ser Ser
                   20                  25                  30
   tgc ccc aag aaa ccg gcg ggt cgt aag aag ttt cgt gag act cgt cac      262
   Cys Pro Lys Lys Pro Ala Gly Arg Lys Lys Phe Arg Glu Thr Arg His
               35                  40                  45
   cca ata tac aga gga gtt cgt cgg aga aac tcc ggt aag tgg gtt tgt      310
   Pro Ile Tyr Arg Gly Val Arg Arg Arg Asn Ser Gly Lys Trp Val Cys
           50                  55                  60
   gag gtt aga gaa cca aac aag aaa aca agg att tgg ctc gga aca ttt      358
   Glu Val Arg Glu Pro Asn Lys Lys Thr Arg Ile Trp Leu Gly Thr Phe
   65                  70                  75                  80
   caa acc gct gag atg gca gct cga gct cac gac gtt gcc gct tta gcc      406
   Gln Thr Ala Glu Met Ala Ala Arg Ala His Asp Val Ala Ala Leu Ala
                   85                  90                  95
   ctt cgt ggc cga tca gcc tgt ctc aat ttc gct gac tcg gct tgg aga      454
   Leu Arg Gly Arg Ser Ala Cys Leu Asn Phe Ala Asp Ser Ala Trp Arg
               100                 105                 110
   ctc cga atc ccg gaa tca act tgc gct aag gac atc caa aag gcg gcg      502
   Leu Arg Ile Pro Glu Ser Thr Cys Ala Lys Asp Ile Gln Lys Ala Ala
           115                 120                 125
   gct gaa gct gcg ttg gcg ttt cag gat gag atg tgt gat gcg acg acg      550
   Ala Glu Ala Ala Leu Ala Phe Gln Asp Glu Met Cys Asp Ala Thr Thr
       130                 135                 140
   gat cat ggc ttc gac atg gag gag acg ttg gtg gag gct att tac acg      598
   Asp His Gly Phe Asp Met Glu Glu Thr Leu Val Glu Ala Ile Tyr Thr
   145                 150                 155                 160
   gcg gaa cag agc gaa aat gcg ttt tat atg cac gat gag gcg atg ttt      646
   Ala Glu Gln Ser Glu Asn Ala Phe Tyr Met His Asp Glu Ala Met Phe
                   165                 170                 175
   gag atg ccg agt ttg ttg gct aat atg gca gaa ggg atg ctt ttg ccg      694
   Glu Met Pro Ser Leu Leu Ala Asn Met Ala Glu Gly Met Leu Leu Pro
   180 185 190
   ctt ccg tcc gta cag tgg aat cat aat cat gaa gtc gac ggc gat gat 742
   Leu Pro Ser Val Gln Trp Asn His Asn His Glu Val Asp Gly Asp Asp
   195 200 205
   gac gac gta tcg tta tgg agt tat taaaactcag attattattt ccatttttag 796
   Asp Asp Val Ser Leu Trp Ser Tyr
   210 215
   tacgatactt tttattttat tattattttt agatcctttt ttagaatgga atcttcatta 856
   tgtttgtaaa actgagaaac gagtgtaaat taaattgatt cagtttcagt ataaaaaaaa 916
   aaaaaaaaaa aaaaaaa 933
<210> 2
   <211> 216
   <212> PRT
   <213> Arabidopsis thaliana
<400> 2
   Met Asn Ser Phe Ser Ala Phe Ser Glu Met Phe Gly Ser Asp Tyr Glu
   1 5 10 15
   Ser Ser Val Ser Ser Gly Gly Asp Tyr Ile Pro Thr Leu Ala Ser Ser
   20 25 30
   Cys Pro Lys Lys Pro Ala Gly Arg Lys Lys Phe Arg Glu Thr Arg His
   35 40 45
   Pro Ile Tyr Arg Gly Val Arg Arg Arg Asn Ser Gly Lys Trp Val Cys
   50 55 60
   Glu Val Arg Glu Pro Asn Lys Lys Thr Arg Ile Trp Leu Gly Thr Phe
   65 70 75 80
   Gln Thr Ala Glu Met Ala Ala Arg Ala His Asp Val Ala Ala Leu Ala
   85 90 95
   Leu Arg Gly Arg Ser Ala Cys Leu Asn Phe Ala Asp Ser Ala Trp Arg
   100 105 110
   Leu Arg Ile Pro Glu Ser Thr Cys Ala Lys Asp Ile Gln Lys Ala Ala
   115 120 125
   Ala Glu Ala Ala Leu Ala Phe Gln Asp Glu Met Cys Asp Ala Thr Thr
   130 135 140
   Asp His Gly Phe Asp Met Glu Glu Thr Leu Val Glu Ala Ile Tyr Thr
   145 150 155 160
   Ala Glu Gln Ser Glu Asn Ala Phe Tyr Met His Asp Glu Ala Met Phe
   165 170 175
   Glu Met Pro Ser Leu Leu Ala Asn Met Ala Glu Gly Met Leu Leu Pro
   180 185 190
   Leu Pro Ser Val Gln Trp Asn His Asn His Glu Val Asp Gly Asp Asp
   195 200 205
   Asp Asp Val Ser Leu Trp Ser Tyr
   210 215
<210> 3
   <211> 1437
   <212> DNA
   <213> Arabidopsis thaliana
<220>
   <221> CDS
   <222> (167) .. (1171)
   <400> 3
   gctgtctgat aaaaagaaga ggaaaactcg aaaaagctac acacaagaag aagaagaaaa 60
   gatacgagca agaagactaa acacgaaagc gatttatcaa ctcgaaggaa gagactttga 120
   ttttcaaatt tcgtccccta tagattgtgt tgtttctggg aaggag atg gca gtt 175
   Met Ala Val
   1
   tat gat cag agt gga gat aga aac aga aca caa att gat aca tcg agg 223
   Tyr Asp Gln Ser Gly Asp Arg Asn Arg Thr Gln Ile Asp Thr Ser Arg
   5 10 15
   aaa agg aaa tct aga agt aga ggt gac ggt act act gtg gct gag aga 271
   Lys Arg Lys Ser Arg Ser Arg Gly Asp Gly Thr Thr Val Ala Glu Arg
   20 25 30 35
   tta aag aga tgg aaa gag tat aac gag acc gta gaa gaa gtt tct acc 319
   Leu Lys Arg Trp Lys Glu Tyr Asn Glu Thr Val Glu Glu Val Ser Thr
   40 45 50
   aag aag agg aaa gta cct gcg aaa ggg tcg aag aag ggt tgt atg aaa 367
   Lys Lys Arg Lys Val Pro Ala Lys Gly Ser Lys Lys Gly Cys Met Lys
   55 60 65
   ggt aaa gga gga cca gag aat agc cga tgt agt ttc aga gga gtt agg 415
   Gly Lys Gly Gly Pro Glu Asn Ser Arg Cys Ser Phe Arg Gly Val Arg
   70 75 80
   caa agg att tgg ggt aaa tgg gtt gct gag atc aga gag cct aat cga 463
   Gln Arg Ile Trp Gly Lys Trp Val Ala Glu Ile Arg Glu Pro Asn Arg
   85 90 95
   ggt agc agg ctt tgg ctt ggt act ttc cct act gct caa gaa gct gct 511
   Gly Ser Arg Leu Trp Leu Gly Thr Phe Pro Thr Ala Gln Glu Ala Ala
   100 105 110 115
   tct gct tat gat gag gct gct aaa gct atg tat ggt cct ttg gct cgt 559
   Ser Ala Tyr Asp Glu Ala Ala Lys Ala Met Tyr Gly Pro Leu Ala Arg
   120 125 130
   ctt aat ttc cct cgg tct gat gcg tct gag gtt acg agt acc tca agt 607
   Leu Asn Phe Pro Arg Ser Asp Ala Ser Glu Val Thr Ser Thr Ser Ser
   135 140 145
   cag tct gag gtg tgt act gtt gag act cct ggt tgt gtt cat gtg aaa 655
   Gln Ser Glu Val Cys Thr Val Glu Thr Pro Gly Cys Val His Val Lys
   150 155 160
   aca gag gat cca gat tgt gaa tct aaa ccc ttc tcc ggt gga gtg gag 703
   Thr Glu Asp Pro Asp Cys Glu Ser Lys Pro Phe Ser Gly Gly Val Glu
   165 170 175
   ccg atg tat tgt ctg gag aat ggt gcg gaa gag atg aag aga ggt gtt 751
   Pro Met Tyr Cys Leu Glu Asn Gly Ala Glu Glu Met Lys Arg Gly Val
   180 185 190 195
   aaa gcg gat aag cat tgg ctg agc gag ttt gaa cat aac tat tgg agt 799
   Lys Ala Asp Lys His Trp Leu Ser Glu Phe Glu His Asn Tyr Trp Ser
   200 205 210
   gat att ctg aaa gag aaa gag aaa cag aag gag caa ggg att gta gaa 847
   Asp Ile Leu Lys Glu Lys Glu Lys Gln Lys Glu Gln Gly Ile Val Glu
   215 220 225
   acc tgt cag caa caa cag cag gat tcg cta tct gtt gca gac tat ggt 895
   Thr Cys Gln Gln Gln Gln Gln Asp Ser Leu Ser Val Ala Asp Tyr Gly
   230 235 240
   tgg ccc aat gat gtg gat cag agt cac ttg gat tct tca gac atg ttt 943
   Trp Pro Asn Asp Val Asp Gln Ser His Leu Asp Ser Ser Asp Met Phe
   245 250 255
   gat gtc gat gag ctt cta cgt gac cta aat ggc gac gat gtg ttt gca 991
   Asp Val Asp Glu Leu Leu Arg Asp Leu Asn Gly Asp Asp Val Phe Ala
   260 265 270 275
   ggc tta aat cag gac cgg tac ccg ggg aac agt gtt gcc aac ggt tca 1039
   Gly Leu Asn Gln Asp Arg Tyr Pro Gly Asn Ser Val Ala Asn Gly Ser
   280 285 290
   tac agg ccc gag agt caa caa agt ggt ttt gat ccg cta caa agc ctc 1087
   Tyr Arg Pro Glu Ser Gln Gln Ser Gly Phe Asp Pro Leu Gln Ser Leu
   295 300 305
   aac tac gga ata cct ccg ttt cag ctc gag gga aag gat ggt aat gga 1135
   Asn Tyr Gly Ile Pro Pro Phe Gln Leu Glu Gly Lys Asp Gly Asn Gly
   310 315 320
   ttc ttc gac gac ttg agt tac ttg gat ctg gag aac taaacaaaac 1181
   Phe Phe Asp Asp Leu Ser Tyr Leu Asp Leu Glu Asn
   325 330 335
   aatatgaagc tttttggatt tgatatttgc cttaatccca caacgactgt tgattctcta 1241
   tccgagtttt agtgatatag agaactacag aacacgtttt ttcttgttat aaaggtgaac 1301
   tgtatatatc gaaacagtga tatgacaata gagaagacaa ctatagtttg ttagtctgct 1361
   tctcttaagt tgttctttag atatgtttta tgttttgtaa caacaggaat gaataataca 1421
   cacttgtaaa aaaaaa 1437
<210> 4
   <211> 335
   <212> PRT
   <213> Arabidopsis thaliana
<400> 4
   Met Ala Val Tyr Asp Gln Ser Gly Asp Arg Asn Arg Thr Gln Ile Asp
   1 5 10 15
   Thr Ser Arg Lys Arg Lys Ser Arg Ser Arg Gly Asp Gly Thr Thr Val
   20 25 30
   Ala Glu Arg Leu Lys Arg Trp Lys Glu Tyr Asn Glu Thr Val Glu Glu
   35 40 45
   Val Ser Thr Lys Lys Arg Lys Val Pro Ala Lys Gly Ser Lys Lys Gly
   50 55 60
   Cys Met Lys Gly Lys Gly Gly Pro Glu Asn Ser Arg Cys Ser Phe Arg
   65 70 75 80
   Gly Val Arg Gln Arg Ile Trp Gly Lys Trp Val Ala Glu Ile Arg Glu
   85 90 95
   Pro Asn Arg Gly Ser Arg Leu Trp Leu Gly Thr Phe Pro Thr Ala Gln
   100 105 110
   Glu Ala Ala Ser Ala Tyr Asp Glu Ala Ala Lys Ala Met Tyr Gly Pro
   115 120 125
   Leu Ala Arg Leu Asn Phe Pro Arg Ser Asp Ala Ser Glu Val Thr Ser
   130 135 140
   Thr Ser Ser Gln Ser Glu Val Cys Thr Val Glu Thr Pro Gly Cys Val
   145 150 155 160
   His Val Lys Thr Glu Asp Pro Asp Cys Glu Ser Lys Pro Phe Ser Gly
   165 170 175
   Gly Val Glu Pro Met Tyr Cys Leu Glu Asn Gly Ala Glu Glu Met Lys
   180 185 190
   Arg Gly Val Lys Ala Asp Lys His Trp Leu Ser Glu Phe Glu His Asn
   195 200 205
   Tyr Trp Ser Asp Ile Leu Lys Glu Lys Glu Lys Gln Lys Glu Gln Gly
   210 215 220
   Ile Val Glu Thr Cys Gln Gln Gln Gln Gln Asp Ser Leu Ser Val Ala
   225 230 235 240
   Asp Tyr Gly Trp Pro Asn Asp Val Asp Gln Ser His Leu Asp Ser Ser
   245 250 255
   Asp Met Phe Asp Val Asp Glu Leu Leu Arg Asp Leu Asn Gly Asp Asp
   260 265 270
   Val Phe Ala Gly Leu Asn Gln Asp Arg Tyr Pro Gly Asn Ser Val Ala
   275 280 285
   Asn Gly Ser Tyr Arg Pro Glu Ser Gln Gln Ser Gly Phe Asp Pro Leu
   290 295 300
   Gln Ser Leu Asn Tyr Gly Ile Pro Pro Phe Gln Leu Glu Gly Lys Asp
   305 310 315 320
   Gly Asn Gly Phe Phe Asp Asp Leu Ser Tyr Leu Asp Leu Glu Asn
   325 330 335
<210> 5
   <211> 937
   <212> DNA
   <213> Arabidopsis thaliana
<220>
   <221> CDS
   <222> (164).. (802)
<400> 5
   cttgaaaaag aatctacctg aaaagaaaaa aaagagagag agatataaat agctttacca    60
   agacagatat actatctttt attaatccaa aaagactgag aactctagta actacgtact   120
   acttaaacct tatccagttt cttgaaacag agtactctga tca atg aac tca ttt     175
                                                   Met Asn Ser Phe
                                                   1
   tca gct ttt tct gaa atg ttt ggc tcc gat tac gag cct caa ggc gga     223
   Ser Ala Phe Ser Glu Met Phe Gly Ser Asp Tyr Glu Pro Gln Gly Gly
   5                   10                  15                  20
   gat tat tgt ccg acg ttg gcc acg agt tgt ccg aag aaa ccg gcg ggc     271
   Asp Tyr Cys Pro Thr Leu Ala Thr Ser Cys Pro Lys Lys Pro Ala Gly
                   25                  30                  35
   cgt aag aag ttt cgt gag act cgt cac cca att tac aga gga gtt cgt     319
   Arg Lys Lys Phe Arg Glu Thr Arg His Pro Ile Tyr Arg Gly Val Arg
               40                  45                  50
   caa aga aac tcc ggt aag tgg gtt tct gaa gtg aga gag cca aac aag     367
   Gln Arg Asn Ser Gly Lys Trp Val Ser Glu Val Arg Glu Pro Asn Lys
           55                  60                  65
   aaa acc agg att tgg ctc ggg act ttc caa acc gct gag atg gca gct     415
   Lys Thr Arg Ile Trp Leu Gly Thr Phe Gln Thr Ala Glu Met Ala Ala
       70                  75                  80
   cgt gct cac gac gtc gct gca tta gcc ctc cgt ggc cga tca gca tgt 463
   Arg Ala His Asp Val Ala Ala Leu Ala Leu Arg Gly Arg Ser Ala Cys
   85 90 95 100
   ctc aac ttc gct gac tcg gct tgg cgg cta cga atc ccg gag tca aca 511
   Leu Asn Phe Ala Asp Ser Ala Trp Arg Leu Arg Ile Pro Glu Ser Thr
   105 110 115
   tgc gcc aag gat atc caa aaa gcg gct gct gaa gcg gcg ttg gct ttt 559
   Cys Ala Lys Asp Ile Gln Lys Ala Ala Ala Glu Ala Ala Leu Ala Phe
   120 125 130
   caa gat gag acg tgt gat acg acg acc acg aat cat ggc ctg gac atg 607
   Gln Asp Glu Thr Cys Asp Thr Thr Thr Thr Asn His Gly Leu Asp Met
   135 140 145
   gag gag acg atg gtg gaa gct att tat aca ccg gaa cag agc gaa ggt 655
   Glu Glu Thr Met Val Glu Ala Ile Tyr Thr Pro Glu Gln Ser Glu Gly
   150 155 160
   gcg ttt tat atg gat gag gag aca atg ttt ggg atg ccg act ttg ttg 703
   Ala Phe Tyr Met Asp Glu Glu Thr Met Phe Gly Met Pro Thr Leu Leu
   165 170 175 180
   gat aat atg gct gaa ggc atg ctt tta ccg ccg ccg tct gtt caa tgg 751
   Asp Asn Met Ala Glu Gly Met Leu Leu Pro Pro Pro Ser Val Gln Trp
   185 190 195
   aat cat aat tat gac ggc gaa gga gat ggt gac gtg tcg ctt tgg agt 799
   Asn His Asn Tyr Asp Gly Glu Gly Asp Gly Asp Val Ser Leu Trp Ser
   200 205 210
   tac taatattcga tagtcgtttc catttttgta ctatagtttg aaaatattct 852
   Tyr
   agttcctttt tttagaatgg ttccttcatt ttattttatt ttattgttgt agaaacgagt 912
   ggaaaataat tcaatacaaa aaaaa 937
<210> 6
<211> 213
   <212> PRT
   <213> Arabidopsis thaliana
<400> 6
   Met Asn Ser Phe Ser Ala Phe Ser Glu Met Phe Gly Ser Asp Tyr Glu
   1 5 10 15
   Pro Gln Gly Gly Asp Tyr Cys Pro Thr Leu Ala Thr Ser Cys Pro Lys
   20 25 30
   Lys Pro Ala Gly Arg Lys Lys Phe Arg Glu Thr Arg His Pro Ile Tyr
   35 40 45
   Arg Gly Val Arg Gln Arg Asn Ser Gly Lys Trp Val Ser Glu Val Arg
   50 55 60
   Glu Pro Asn Lys Lys Thr Arg Ile Trp Leu Gly Thr Phe Gln Thr Ala
   65 70 75 80
   Glu Met Ala Ala Arg Ala His Asp Val Ala Ala Leu Ala Leu Arg Gly
   85 90 95
   Arg Ser Ala Cys Leu Asn Phe Ala Asp Ser Ala Trp Arg Leu Arg Ile
   100 105 110
   Pro Glu Ser Thr Cys Ala Lys Asp Ile Gln Lys Ala Ala Ala Glu Ala
   115 120 125
   Ala Leu Ala Phe Gln Asp Glu Thr Cys Asp Thr Thr Thr Thr Asn His
   130 135 140
   Gly Leu Asp Met Glu Glu Thr Met Val Glu Ala Ile Tyr Thr Pro Glu
   145 150 155 160
   Gln Ser Glu Gly Ala Phe Tyr Met Asp Glu Glu Thr Met Phe Gly Met
   165 170 175
   Pro Thr Leu Leu Asp Asn Met Ala Glu Gly Met Leu Leu Pro Pro Pro
   180 185 190
   Ser Val Gln Trp Asn His Asn Tyr Asp Gly Glu Gly Asp Gly Asp Val
   195 200 205
   Ser Leu Trp Ser Tyr
   210
<210> 7
   <211> 944
   <212> DNA
   <213> Arabidopsis thaliana
<220>
   <221> CDS
   <222> (135) .. (782)
<400> 7
   cctgaattag aaaagaaaga tagatagaga aataaatatt ttatcatacc atacaaaaaa 60
   agacagagat cttctactta ctctactctc ataaacctta tccagtttct tgaaacagag 120
   tactcttctg atca atg aac tca ttt tct gcc ttt tct gaa atg ttt ggc 170
   Met Asn Ser Phe Ser Ala Phe Ser Glu Met Phe Gly
   1 5 10
   tcc gat tac gag tct ccg gtt tcc tca ggc ggt gat tac agt ccg aag 218
   Ser Asp Tyr Glu Ser Pro Val Ser Ser Gly Gly Asp Tyr Ser Pro Lys
   15 20 25
   ctt gcc acg agc tgc ccc aag aaa cca gcg gga agg aag aag ttt cgt 266
   Leu Ala Thr Ser Cys Pro Lys Lys Pro Ala Gly Arg Lys Lys Phe Arg
   30 35 40
   gag act cgt cac cca att tac aga gga gtt cgt caa aga aac tcc ggt 314
   Glu Thr Arg His Pro Ile Tyr Arg Gly Val Arg Gln Arg Asn Ser Gly
   45 50 55 60
   aag tgg gtg tgt gag ttg aga gag cca aac aag aaa acg agg att tgg 362
   Lys Trp Val Cys Glu Leu Arg Glu Pro Asn Lys Lys Thr Arg Ile Trp
                   65                  70                  75
   ctc ggg act ttc caa acc gct gag atg gca gct cgt gct cac gac gtc     410
   Leu Gly Thr Phe Gln Thr Ala Glu Met Ala Ala Arg Ala His Asp Val
               80                  85                  90
   gcc gcc ata gct ctc cgt ggc aga tct gcc tgt ctc aat ttc gct gac     458
   Ala Ala Ile Ala Leu Arg Gly Arg Ser Ala Cys Leu Asn Phe Ala Asp
           95                  100                 105
   tcg gct tgg cgg cta cga atc ccg gaa tca acc tgt gcc aag gaa atc     506
   Ser Ala Trp Arg Leu Arg Ile Pro Glu Ser Thr Cys Ala Lys Glu Ile
       110                 115                 120
   caa aag gcg gcg gct gaa gcc gcg ttg aat ttt caa gat gag atg tgt     554
   Gln Lys Ala Ala Ala Glu Ala Ala Leu Asn Phe Gln Asp Glu Met Cys
   125                 130                 135                 140
   cat atg acg acg gat gct cat ggt ctt gac atg gag gag acc ttg gtg     602
   His Met Thr Thr Asp Ala His Gly Leu Asp Met Glu Glu Thr Leu Val
                   145                 150                 155
   gag gct att tat acg ccg gaa cag agc caa gat gcg ttt tat atg gat     650
   Glu Ala Ile Tyr Thr Pro Glu Gln Ser Gln Asp Ala Phe Tyr Met Asp
               160                 165                 170
   gaa gag gcg atg ttg ggg atg tct agt ttg ttg gat aac atg gcc gaa     698
   Glu Glu Ala Met Leu Gly Met Ser Ser Leu Leu Asp Asn Met Ala Glu
           175                 180                 185
   ggg atg ctt tta ccg tcg ccg tcg gtt caa tgg aac tat aat ttt gat     746
   Gly Met Leu Leu Pro Ser Pro Ser Val Gln Trp Asn Tyr Asn Phe Asp
       190                 195                 200
   gtc gag gga gat gat gac gtg tcc tta tgg agc tat taaaattcga          792
   Val Glu Gly Asp Asp Asp Val Ser Leu Trp Ser Tyr
   205                 210                 215
   tttttatttc catttttggt attatagctt tttatacatt tgatcctttt ttagaatgga   852
   tcttcttctt tttttggttg tgagaaacga atgtaaatgg taaaagttgt tgtcaaatgc   912
   aaatgttttt gagtgcagaa tatataatct tt 944
<210> 8
   <211> 216
   <212> PRT
   <213> Arabidopsis thaliana
<400> 8
   Met Asn Ser Phe Ser Ala Phe Ser Glu Met Phe Gly Ser Asp Tyr Glu
   1 5 10 15
   Ser Pro Val Ser Ser Gly Gly Asp Tyr Ser Pro Lys Leu Ala Thr Ser
   20 25 30
   Cys Pro Lys Lys Pro Ala Gly Arg Lys Lys Phe Arg Glu Thr Arg His
   35 40 45
   Pro Ile Tyr Arg Gly Val Arg Gln Arg Asn Ser Gly Lys Trp Val Cys
   50 55 60
   Glu Leu Arg Glu Pro Asn Lys Lys Thr Arg Ile Trp Leu Gly Thr Phe
   65 70 75 80
   Gln Thr Ala Glu Met Ala Ala Arg Ala His Asp Val Ala Ala Ile Ala
   85 90 95
   Leu Arg Gly Arg Ser Ala Cys Leu Asn Phe Ala Asp Ser Ala Trp Arg
   100 105 110
   Leu Arg Ile Pro Glu Ser Thr Cys Ala Lys Glu Ile Gln Lys Ala Ala
   115 120 125
   Ala Glu Ala Ala Leu Asn Phe Gln Asp Glu Met Cys His Met Thr Thr
   130 135 140
   Asp Ala His Gly Leu Asp Met Glu Glu Thr Leu Val Glu Ala Ile Tyr
   145 150 155 160
   Thr Pro Glu Gln Ser Gln Asp Ala Phe Tyr Met Asp Glu Glu Ala Met
   165 170 175
   Leu Gly Met Ser Ser Leu Leu Asp Asn Met Ala Glu Gly Met Leu Leu
   180 185 190
   Pro Ser Pro Ser Val Gln Trp Asn Tyr Asn Phe Asp Val Glu Gly Asp
   195 200 205
   Asp Asp Val Ser Leu Trp Ser Tyr
   210 215
<210> 9
   <211> 1513
   <212> DNA
   <213> Arabidopsis thaliana
<220>
   <221> CDS
   <222> (183) .. (1172)
<220>
   <221> misc_feature
   <222> (1443), (1444), (1447), (1450), (1459), (1472), (1495), (1508), (1 510)
   <223> n is A, C, G or T
<400> 9
   gagacgctag aaagaacgcg aaagcttgcg aagaagattt gcttttgatc gacttaacac 60
   gaacaacaaa caacatctgc gtgataaaga agagattttt gcctaaataa agaagagatt 120
   cgactctaat cctggagtta tcattcacga tagattctta gattgcgact ataaagaaga 180
   ag atg gct gta tat gaa caa acc gga acc gag cag ccg aag aaa agg 227
   Met Ala Val Tyr Glu Gln Thr Gly Thr Glu Gln Pro Lys Lys Arg
   1 5 10 15
   aaa tct agg gct cga gca ggt ggt tta acg gtg gct gat agg cta aag 275
   Lys Ser Arg Ala Arg Ala Gly Gly Leu Thr Val Ala Asp Arg Leu Lys
   20 25 30
   aag tgg aaa gag tac aac gag att gtt gaa gct tcg gct gtt aaa gaa 323
   Lys Trp Lys Glu Tyr Asn Glu Ile Val Glu Ala Ser Ala Val Lys Glu
   35 40 45
   gga gag aaa ccg aaa cgc aaa gtt cct gcg aaa ggg tcg aag aaa ggt 371
   Gly Glu Lys Pro Lys Arg Lys Val Pro Ala Lys Gly Ser Lys Lys Gly
   50 55 60
   tgt atg aag ggt aaa gga gga cca gat aat tct cac tgt agt ttt aga 419
   Cys Met Lys Gly Lys Gly Gly Pro Asp Asn Ser His Cys Ser Phe Arg
   65 70 75
   gga gtt aga caa agg att tgg ggt aaa tgg gtt gca gag att cga gaa 467
   Gly Val Arg Gln Arg Ile Trp Gly Lys Trp Val Ala Glu Ile Arg Glu
   80 85 90 95
   ccg aaa ata gga act aga ctt tgg ctt ggt act ttt cct acc gcg gaa 515
   Pro Lys Ile Gly Thr Arg Leu Trp Leu Gly Thr Phe Pro Thr Ala Glu
   100 105 110
   aaa gct gct tcc gct tat gat gaa gcg gct acc gct atg tac ggt tca 563
   Lys Ala Ala Ser Ala Tyr Asp Glu Ala Ala Thr Ala Met Tyr Gly Ser
   115 120 125
   ttg gct cgt ctt aac ttc cct cag tct gtt ggg tct gag ttt act agt 611
   Leu Ala Arg Leu Asn Phe Pro Gln Ser Val Gly Ser Glu Phe Thr Ser
   130 135 140
   acg tct agt caa tct gag gtg tgt acg gtt gaa aat aag gcg gtt gtt 659
   Thr Ser Ser Gln Ser Glu Val Cys Thr Val Glu Asn Lys Ala Val Val
   145 150 155
   tgt ggt gat gtt tgt gtg aag cat gaa gat act gat tgt gaa tct aat 707
   Cys Gly Asp Val Cys Val Lys His Glu Asp Thr Asp Cys Glu Ser Asn
   160 165 170 175
   cca ttt agt cag att tta gat gtt aga gaa gag tct tgt gga acc agg 755
   Pro Phe Ser Gln Ile Leu Asp Val Arg Glu Glu Ser Cys Gly Thr Arg
   180 185 190
   ccg gac agt tgc acg gtt gga cat caa gat atg aat tct tcg ctg aat 803
   Pro Asp Ser Cys Thr Val Gly His Gln Asp Met Asn Ser Ser Leu Asn
   195 200 205
   tac gat ttg ctg tta gag ttt gag cag cag tat tgg ggc caa gtt ttg 851
   Tyr Asp Leu Leu Leu Glu Phe Glu Gln Gln Tyr Trp Gly Gln Val Leu
   210 215 220
   cag gag aaa gag aaa ccg aag cag gaa gaa gag gag ata cag caa cag 899
   Gln Glu Lys Glu Lys Pro Lys Gln Glu Glu Glu Glu Ile Gln Gln Gln
   225 230 235
   caa cag gaa cag caa cag caa cag ctg caa ccg gat ttg ctt act gtt 947
   Gln Gln Glu Gln Gln Gln Gln Gln Leu Gln Pro Asp Leu Leu Thr Val
   240 245 250 255
   gca gat tac ggt tgg cct tgg tct aat gat att gta aat gat cag act 995
   Ala Asp Tyr Gly Trp Pro Trp Ser Asn Asp Ile Val Asn Asp Gln Thr
   260 265 270
   tct tgg gat cct aat gag tgc ttt gat att aat gaa ctc ctt gga gat 1043
   Ser Trp Asp Pro Asn Glu Cys Phe Asp Ile Asn Glu Leu Leu Gly Asp
   275 280 285
   ttg aat gaa cct ggt ccc cat cag agc caa gac caa aac cac gta aat 1091
   Leu Asn Glu Pro Gly Pro His Gln Ser Gln Asp Gln Asn His Val Asn
   290 295 300
   tct ggt agt tat gat ttg cat ccg ctt cat ctc gag cca cac gat ggt 1139
   Ser Gly Ser Tyr Asp Leu His Pro Leu His Leu Glu Pro His Asp Gly
   305 310 315
   cac gag ttc aat ggt ttg agt tct ctg gat att tgagagttct gaggcaatgg 1192
   His Glu Phe Asn Gly Leu Ser Ser Leu Asp Ile
   320 325 330
   tcctacaaga ctacaacata atctttggat tgatcatagg agaaacaaga aataggtgtt 1252
   aatgatctga ttcacaatga aaaaatattt aataactcta tagtttttgt tctttccttg 1312
   gatcatgaac tgttgcttct catctattga gttaatatag cgaatagcag agtttctctc 1372
   tttcttctct ttgtagaaaa aaaaaaaaaa aaaaaaaaaa aaaaaaaayh sakmabgcar 1432
   srcsdvsnaa nntrnatnar sarchcntrr agrctrascn csrcaswash tskbabarak 1492
   aantamaysa kmasrngnga c 1513
<210> 10
   <211> 330
   <212> PRT
   <213> Arabidopsis thaliana
<400> 10
   Met Ala Val Tyr Glu Gln Thr Gly Thr Glu Gln Pro Lys Lys Arg Lys
   1 5 10 15
   Ser Arg Ala Arg Ala Gly Gly Leu Thr Val Ala Asp Arg Leu Lys Lys
   20 25 30
   Trp Lys Glu Tyr Asn Glu Ile Val Glu Ala Ser Ala Val Lys Glu Gly
   35 40 45
   Glu Lys Pro Lys Arg Lys Val Pro Ala Lys Gly Ser Lys Lys Gly Cys
   50 55 60
   Met Lys Gly Lys Gly Gly Pro Asp Asn Ser His Cys Ser Phe Arg Gly
   65 70 75 80
   Val Arg Gln Arg Ile Trp Gly Lys Trp Val Ala Glu Ile Arg Glu Pro
   85 90 95
   Lys Ile Gly Thr Arg Leu Trp Leu Gly Thr Phe Pro Thr Ala Glu Lys
   100 105 110
   Ala Ala Ser Ala Tyr Asp Glu Ala Ala Thr Ala Met Tyr Gly Ser Leu
   115 120 125
   Ala Arg Leu Asn Phe Pro Gln Ser Val Gly Ser Glu Phe Thr Ser Thr
   130 135 140
   Ser Ser Gln Ser Glu Val Cys Thr Val Glu Asn Lys Ala Val Val Cys
   145 150 155 160
   Gly Asp Val Cys Val Lys His Glu Asp Thr Asp Cys Glu Ser Asn Pro
   165 170 175
   Phe Ser Gln Ile Leu Asp Val Arg Glu Glu Ser Cys Gly Thr Arg Pro
   180 185 190
   Asp Ser Cys Thr Val Gly His Gln Asp Met Asn Ser Ser Leu Asn Tyr
   195 200 205
   Asp Leu Leu Leu Glu Phe Glu Gln Gln Tyr Trp Gly Gln Val Leu Gln
   210 215 220
   Glu Lys Glu Lys Pro Lys Gln Glu Glu Glu Glu Ile Gln Gln Gln Gln
   225 230 235 240
   Gln Glu Gln Gln Gln Gln Gln Leu Gln Pro Asp Leu Leu Thr Val Ala
   245 250 255
   Asp Tyr Gly Trp Pro Trp Ser Asn Asp Ile Val Asn Asp Gln Thr Ser
   260 265 270
   Trp Asp Pro Asn Glu Cys Phe Asp Ile Asn Glu Leu Leu Gly Asp Leu
   275 280 285
   Asn Glu Pro Gly Pro His Gln Ser Gln Asp Gln Asn His Val Asn Ser
   290 295 300
   Gly Ser Tyr Asp Leu His Pro Leu His Leu Glu Pro His Asp Gly His
   305 310 315 320
   Glu Phe Asn Gly Leu Ser Ser Leu Asp Ile
   325 330
<210> 11
   <211> 675
   <212> DNA
   <213> Arabidopsis thaliana
<400> 11
   atgaatccat tttactctac attcccagac tcgtttctct caatctccga tcatagatct 60
   ccggtttcag acagtagtga gtgttcacca aagttagctt caagttgtcc aaagaaacga 120
   gctgggagga agaagtttcg tgagacacgt catccgattt acagaggagt tcgtcagagg 180
   aattctggta aatgggtttg tgaagttaga gagcctaata agaaatctag gatttggtta 240
   ggtacttttc cgacggttga aatggctgct cgtgctcatg atgttgctgc tttagctctt 300
   cgtggtcgct ctgcttgtct caatttcgct gattctgctt ggcggcttcg tattcctgag 360
   actacttgtc ctaaggagat tcagaaagct gcgtctgaag ctgcaatggc gtttcagaat 420
   gagactacga cggagggatc taaaactgcg gcggaggcag aggaggcggc aggggagggg 480
   gtgagggagg gggagaggag ggcggaggag cagaatggtg gtgtgtttta tatggatgat 540
   gaggcgcttt tggggatgcc caactttttt gagaatatgg cggaggggat gcttttgccg 600
   ccgccggaag ttggctggaa tcataacgac tttgacggag tgggtgacgt gtcactctgg 660
   agttttgacg agtaa 675
<210> 12
   <211> 224
   <212> PRT
   <213> Arabidopsis thaliana
<400> 12
   Met Asn Pro Phe Tyr Ser Thr Phe Pro Asp Ser Phe Leu Ser Ile Ser
   1 5 10 15
   Asp His Arg Ser Pro Val Ser Asp Ser Ser Glu Cys Ser Pro Lys Leu
   20 25 30
   Ala Ser Ser Cys Pro Lys Lys Arg Ala Gly Arg Lys Lys Phe Arg Glu
   35 40 45
   Thr Arg His Pro Ile Tyr Arg Gly Val Arg Gln Arg Asn Ser Gly Lys
   50 55 60
   Trp Val Cys Glu Val Arg Glu Pro Asn Lys Lys Ser Arg Ile Trp Leu
   65 70 75 80
   Gly Thr Phe Pro Thr Val Glu Met Ala Ala Arg Ala His Asp Val Ala
   85 90 95
   Ala Leu Ala Leu Arg Gly Arg Ser Ala Cys Leu Asn Phe Ala Asp Ser
   100 105 110
   Ala Trp Arg Leu Arg Ile Pro Glu Thr Thr Cys Pro Lys Glu Ile Gln
   115 120 125
   Lys Ala Ala Ser Glu Ala Ala Met Ala Phe Gln Asn Glu Thr Thr Thr
   130 135 140
   Glu Gly Ser Lys Thr Ala Ala Glu Ala Glu Glu Ala Ala Gly Glu Gly
   145 150 155 160
   Val Arg Glu Gly Glu Arg Arg Ala Glu Glu Gln Asn Gly Gly Val Phe
   165 170 175
   Tyr Met Asp Asp Glu Ala Leu Leu Gly Met Pro Asn Phe Phe Glu Asn
   180 185 190
   Met Ala Glu Gly Met Leu Leu Pro Pro Pro Glu Val Gly Trp Asn His
   195 200 205
   Asn Asp Phe Asp Gly Val Gly Asp Val Ser Leu Trp Ser Phe Asp Glu
   210 215 220
<210> 13
   <211> 546
   <212> DNA
   <213> Arabidopsis thaliana
<400> 13
   atggaaaacg acgatatcac cgtggcggag atgaagccaa agaagcgtgc tggacggagg 60
   attttcaagg agacacgtca cccaatctac agaggcgtgc ggcgtaggga cggcgacaaa 120
   tgggtatgcg aagtccgtga accgattcat cagcgtcgag tctggctcgg aacttatccg 180
   acggcagata tggccgcacg tgctcacgac gtggcggttc ttgctctgcg cgggagatcc 240
   gcgtgtttga atttctccga ttctgcttgg aggttgccgg tgccggcatc cactgatccg 300
   gacacgatca ggcgcacggc ggccgaagca gcggagatgt tcaggccgcc ggagtttagt 360
   acaggaatta cggttttacc ctcagccagt gagtttgaca cgtcggatga aggagtcgct 420
   ggaatgatga tgaggctcgc ggaggagccg ttgatgtcgc cgccaagatc gtacattgat 480
   atgaatacga gtgtgtacgt ggacgaagaa atgtgttacg aagatttgtc actttggagt 540
   tactaa 546
<210> 14
   <211> 181
   <212> PRT
   <213> Arabidopsis thaliana
<400> 14
   Met Glu Asn Asp Asp Ile Thr Val Ala Glu Met Lys Pro Lys Lys Arg
   1 5 10 15
   Ala Gly Arg Arg Ile Phe Lys Glu Thr Arg His Pro Ile Tyr Arg Gly
   20 25 30
   Val Arg Arg Arg Asp Gly Asp Lys Trp Val Cys Glu Val Arg Glu Pro
   35 40 45
   Ile His Gln Arg Arg Val Trp Leu Gly Thr Tyr Pro Thr Ala Asp Met
   50 55 60
   Ala Ala Arg Ala His Asp Val Ala Val Leu Ala Leu Arg Gly Arg Ser
   65 70 75 80
   Ala Cys Leu Asn Phe Ser Asp Ser Ala Trp Arg Leu Pro Val Pro Ala
   85 90 95
   Ser Thr Asp Pro Asp Thr Ile Arg Arg Thr Ala Ala Glu Ala Ala Glu
   100 105 110
   Met Phe Arg Pro Pro Glu Phe Ser Thr Gly Ile Thr Val Leu Pro Ser
   115 120 125
   Ala Ser Glu Phe Asp Thr Ser Asp Glu Gly Val Ala Gly Met Met Met
   130 135 140
   Arg Leu Ala Glu Glu Pro Leu Met Ser Pro Pro Arg Ser Tyr Ile Asp
   145 150 155 160
   Met Asn Thr Ser Val Tyr Val Asp Glu Glu Met Cys Tyr Glu Asp Leu
   165 170 175
   Ser Leu Trp Ser Tyr
   180
<210> 15
   <211> 630
   <212> DNA
   <213> Arabidopsis thaliana
<400> 15
   atgaataatg atgatattat tctggcggag atgaggccta agaagcgtgc gggaaggaga 60
   gtgtttaagg agacacgtca cccagtttac agaggcataa ggcggaggaa cggtgacaaa 120
   tgggtctgcg aagtcagaga accgacgcac caacgccgca tttggctcgg gacttatccc 180
   acagcagata tggcagcgcg tgcacacgac gtggcggttt tagctctgcg tgggagatcc 240
   gcatgtttga atttcgccga ctccgcttgg cggcttccgg tgccggaatc caatgatccg 300
   gatgtgataa gaagagttgc ggcggaagct gcggagatgt ttaggccggt ggatttagaa 360
   agtggaatta cggttttgcc ttgtgcggga gatgatgtgg atttgggttt tggttcgggt 420
   tccggctctg gttcgggatc ggaggagagg aattcttctt cgtatggatt tggagactac 480
   gaagaagtct caacgacgat gatgagactc gcggaggggc cactaatgtc gccgccgcga 540
   tcgtatatgg aagacatgac tcctactaat gtttacacgg aagaagagat gtgttatgaa 600
   gatatgtcat tgtggagtta cagatattaa 630
<210> 16
   <211> 209
   <212> PRT
   <213> Arabidopsis thaliana
<400> 16
   Met Asn Asn Asp Asp Ile Ile Leu Ala Glu Met Arg Pro Lys Lys Arg
   1 5 10 15
   Ala Gly Arg Arg Val Phe Lys Glu Thr Arg His Pro Val Tyr Arg Gly
   20 25 30
   Ile Arg Arg Arg Asn Gly Asp Lys Trp Val Cys Glu Val Arg Glu Pro
   35 40 45
   Thr His Gln Arg Arg Ile Trp Leu Gly Thr Tyr Pro Thr Ala Asp Met
   50 55 60
   Ala Ala Arg Ala His Asp Val Ala Val Leu Ala Leu Arg Gly Arg Ser
   65 70 75 80
   Ala Cys Leu Asn Phe Ala Asp Ser Ala Trp Arg Leu Pro Val Pro Glu
   85 90 95
   Ser Asn Asp Pro Asp Val Ile Arg Arg Val Ala Ala Glu Ala Ala Glu
   100 105 110
   Met Phe Arg Pro Val Asp Leu Glu Ser Gly Ile Thr Val Leu Pro Cys
   115 120 125
   Ala Gly Asp Asp Val Asp Leu Gly Phe Gly Ser Gly Ser Gly Ser Gly
   130 135 140
   Ser Gly Ser Glu Glu Arg Asn Ser Ser Ser Tyr Gly Phe Gly Asp Tyr
   145 150 155 160
   Glu Glu Val Ser Thr Thr Met Met Arg Leu Ala Glu Gly Pro Leu Met
   165 170 175
   Ser Pro Pro Arg Ser Tyr Met Glu Asp Met Thr Pro Thr Asn Val Tyr
   180 185 190
   Thr Glu Glu Glu Met Cys Tyr Glu Asp Met Ser Leu Trp Ser Tyr Arg
   195 200 205
   Tyr
<210> 17
   <211> 1026
   <212> DNA
   <213> Arabidopsis thaliana
<400> 17
   atgccgtcgg agattgttga caggaaaagg aagtctcgtg gaacacgaga tgtagctgag 60
   attctaaggc aatggagaga gtacaatgag cagattgagg cagaatcttg tatcgatggt 120
   ggtggtccaa aatcaatccg aaagcctcct ccaaaaggtt cgaggaaggg ttgtatgaaa 180
   ggtaaaggtg gacctgaaaa cgggatttgt gactatagag gagttagaca gaggagatgg 240
   ggtaaatggg ttgctgagat ccgtgagcca gacggaggtg ctaggttgtg gctcggtact 300
   ttctccagtt catatgaagc tgcattggct tatgacgagg cggccaaagc tatatatggt 360
   cagtctgcca gactcaatct tcccgagatc acaaatcgct cttcttcgac tgctgccact 420
   gccactgtgt caggctcggt tactgcattt tctgatgaat ctgaagtttg tgcacgtgag 480
   gatacaaatg caagttcagg ttttggtcag gtgaaactag aggattgtag cgatgaatat 540
   gttctcttag atagttctca gtgtattaaa gaggagctga aaggaaaaga ggaagtgagg 600
   gaagaacata acttggctgt tggttttgga attggacagg actcgaaaag ggagactttg 660
   gatgcttggt tgatgggaaa tggcaatgaa caagaaccat tggagtttgg tgtggatgaa 720
   acgtttgata ttaatgagct attgggtata ttaaacgaca acaatgtgtc tggtcaagag 780
   acaatgcagt atcaagtgga tagacaccca aatttcagtt accaaacgca gtttccaaat 840
   tctaacttgc tcgggagcct caaccctatg gagattgctc aaccaggagt tgattatgga 900
   tgtccttatg tgcagcccag tgatatggag aactatggta ttgatttaga ccatcgcagg 960
   ttcaatgatc ttgacataca ggacttggat tttggaggag acaaagatgt tcatggatct 1020
   acataa                                                            1026
<210> 18
   <211> 341
   <212> PRT
   <213> Arabidopsis thaliana
<400> 18
   Met Pro Ser Glu Ile Val Asp Arg Lys Arg Lys Ser Arg Gly Thr Arg
   1 5 10 15
   Asp Val Ala Glu Ile Leu Arg Gln Trp Arg Glu Tyr Asn Glu Gln Ile
   20 25 30
   Glu Ala Glu Ser Cys Ile Asp Gly Gly Gly Pro Lys Ser Ile Arg Lys
   35 40 45
   Pro Pro Pro Lys Gly Ser Arg Lys Gly Cys Met Lys Gly Lys Gly Gly
   50 55 60
   Pro Glu Asn Gly Ile Cys Asp Tyr Arg Gly Val Arg Gln Arg Arg Trp
   65 70 75 80
   Gly Lys Trp Val Ala Glu Ile Arg Glu Pro Asp Gly Gly Ala Arg Leu
   85 90 95
   Trp Leu Gly Thr Phe Ser Ser Ser Tyr Glu Ala Ala Leu Ala Tyr Asp
   100 105 110
   Glu Ala Ala Lys Ala Ile Tyr Gly Gln Ser Ala Arg Leu Asn Leu Pro
   115 120 125
   Glu Ile Thr Asn Arg Ser Ser Ser Thr Ala Ala Thr Ala Thr Val Ser
   130 135 140
   Gly Ser Val Thr Ala Phe Ser Asp Glu Ser Glu Val Cys Ala Arg Glu
   145 150 155 160
   Asp Thr Asn Ala Ser Ser Gly Phe Gly Gln Val Lys Leu Glu Asp Cys
   165 170 175
   Ser Asp Glu Tyr Val Leu Leu Asp Ser Ser Gln Cys Ile Lys Glu Glu
   180 185 190
   Leu Lys Gly Lys Glu Glu Val Arg Glu Glu His Asn Leu Ala Val Gly
   195 200 205
   Phe Gly Ile Gly Gln Asp Ser Lys Arg Glu Thr Leu Asp Ala Trp Leu
   210 215 220
   Met Gly Asn Gly Asn Glu Gln Glu Pro Leu Glu Phe Gly Val Asp Glu
   225 230 235 240
   Thr Phe Asp Ile Asn Glu Leu Leu Gly Ile Leu Asn Asp Asn Asn Val
   245 250 255
   Ser Gly Gln Glu Thr Met Gln Tyr Gln Val Asp Arg His Pro Asn Phe
   260 265 270
   Ser Tyr Gln Thr Gln Phe Pro Asn Ser Asn Leu Leu Gly Ser Leu Asn
   275 280 285
   Pro Met Glu Ile Ala Gln Pro Gly Val Asp Tyr Gly Cys Pro Tyr Val
   290 295 300
   Gln Pro Ser Asp Met Glu Asn Tyr Gly Ile Asp Leu Asp His Arg Arg
   305 310 315 320
   Phe Asn Asp Leu Asp Ile Gln Asp Leu Asp Phe Gly Gly Asp Lys Asp
   325 330 335
   Val His Gly Ser Thr
   340
<210> 19
   <211> 621
   <212> DNA
   <213> Arabidopsis thaliana
<400> 19
   atgtcatcca tagagccaaa agtaatgatg gttggtgcta ataagaaaca acgaaccgtc 60
   caagctagtt cgaggaaagg ttgtatgaga ggaaaaggtg gacccgataa cgcgtcttgc 120
   acttacaaag gtgttagaca acgcacttgg ggcaaatggg tcgctgagat ccgcgagcct 180
   aaccgaggag ctcgtctttg gctcggtacc ttcgacacct cccgtgaagc tgccttggct 240
   tatgactccg cagctcgtaa gctctatggg cctgaggctc atctcaacct ccctgagtcc 300
   ttaagaagtt accctaaaac ggcgtcgtct ccggcgtccc agactacacc aagcagcaac 360
   accggtggaa aaagcagcag cgactctgag tcgccgtgtt catccaacga gatgtcatca 420
   tgtggaagag tgacagagga gatatcatgg gagcatataa acgtggattt gccggtaatg 480
   gatgattctt caatatggga agaagctaca atgtcgttag gatttccatg ggttcatgaa 540
   ggagataatg atatttctcg gtttgatact tgtatttccg gtggctattc taattgggat 600
   tcctttcatt ccccactttg a 621
<210> 20
   <211> 206
   <212> PRT
   <213> Arabidopsis thaliana
<400> 20
   Met Ser Ser Ile Glu Pro Lys Val Met Met Val Gly Ala Asn Lys Lys
   1 5 10 15
   Gln Arg Thr Val Gln Ala Ser Ser Arg Lys Gly Cys Met Arg Gly Lys
   20 25 30
   Gly Gly Pro Asp Asn Ala Ser Cys Thr Tyr Lys Gly Val Arg Gln Arg
   35 40 45
   Thr Trp Gly Lys Trp Val Ala Glu Ile Arg Glu Pro Asn Arg Gly Ala
   50 55 60
   Arg Leu Trp Leu Gly Thr Phe Asp Thr Ser Arg Glu Ala Ala Leu Ala
   65 70 75 80
   Tyr Asp Ser Ala Ala Arg Lys Leu Tyr Gly Pro Glu Ala His Leu Asn
   85 90 95
   Leu Pro Glu Ser Leu Arg Ser Tyr Pro Lys Thr Ala Ser Ser Pro Ala
   100 105 110
   Ser Gln Thr Thr Pro Ser Ser Asn Thr Gly Gly Lys Ser Ser Ser Asp
   115 120 125
   Ser Glu Ser Pro Cys Ser Ser Asn Glu Met Ser Ser Cys Gly Arg Val
   130 135 140
   Thr Glu Glu Ile Ser Trp Glu His Ile Asn Val Asp Leu Pro Val Met
   145 150 155 160
   Asp Asp Ser Ser Ile Trp Glu Glu Ala Thr Met Ser Leu Gly Phe Pro
   165 170 175
   Trp Val His Glu Gly Asp Asn Asp Ile Ser Arg Phe Asp Thr Cys Ile
   180 185 190
   Ser Gly Gly Tyr Ser Asn Trp Asp Ser Phe His Ser Pro Leu
   195 200 205
<210> 21
   <211> 975
   <212> DNA
   <213> Arabidopsis thaliana
<400> 21
   atggaaaagg aagataacgg atcgaaacag agctcctctg cttctgttgt atcctcgaga 60
   agacgaagaa gagtggttga gccagtggaa gcgacgttac agagatggga ggaagaagga 120
   ttggcgagag ctcgtagggt tcaagccaaa ggttcgaaga aaggttgtat gagaggaaaa 180
   ggtggaccag agaatcctgt ttgtcggttt agaggtgttc gacaaagggt ttgggggaaa 240
   tgggttgctg agatacgtga accagtgagt caccgtggtg caaactctag tcgtagtaaa 300
   cggctttggc ttggcacgtt tgctactgca gctgaagctg ctttggctta cgacagagct 360
   gctagtgtca tgtacggacc ctatgccagg ttaaatttcc cggaagattt gggtggggga 420
   aggaagaagg acgaggaggc ggaaagttcg ggaggctatt ggttggaaac taacaaagcc 480
   ggtaatggcg tgattgaaac ggaaggtgga aaagactatg tagtctacaa tgaagacgct 540
   attgagcttg gccatgacaa gactcagaat cctgacatgt ttgatgtcga tgagcttcta 600
   cgtgacctaa atggcgacga tgtgtttgca ggcatgactg ataatgaaat agtgaaccca 660
   gcagttaaat caggaccggt acccggggaa cagtgttgcc aacggttcat acaggcccga 720
   gagttgaaat cagaggaagg ttacagctat gatcgattca aattggcaac aaagtggttt 780
   tgatccgcta caaagcctca actacggaat acctccgttt cagctcataa cggattgttg 840
   tataatgaac ctcaaagctc cagttatcac gagggaaagg atggtaatgg attcttcgac 900
   gacttgagtt acttggatct ggagaactaa cagggaggtg gattcgattc atattttgag 960
   tatttcagat tctag 975
<210> 22
   <211> 244
   <212> PRT
   <213> Arabidopsis thaliana
<400> 22
   Met Glu Lys Glu Asp Asn Gly Ser Lys Gln Ser Ser Ser Ala Ser Val
   1 5 10 15
   Val Ser Ser Arg Arg Arg Arg Arg Val Val Glu Pro Val Glu Ala Thr
   20 25 30
   Leu Gln Arg Trp Glu Glu Glu Gly Leu Ala Arg Ala Arg Arg Val Gln
   35 40 45
   Ala Lys Gly Ser Lys Lys Gly Cys Met Arg Gly Lys Gly Gly Pro Glu
   50 55 60
   Asn Pro Val Cys Arg Phe Arg Gly Val Arg Gln Arg Val Trp Gly Lys
   65 70 75 80
   Trp Val Ala Glu Ile Arg Glu Pro Val Ser His Arg Gly Ala Asn Ser
   85 90 95
   Ser Arg Ser Lys Arg Leu Trp Leu Gly Thr Phe Ala Thr Ala Ala Glu
   100 105 110
   Ala Ala Leu Ala Tyr Asp Arg Ala Ala Ser Val Met Tyr Gly Pro Tyr
   115 120 125
   Ala Arg Leu Asn Phe Pro Glu Asp Leu Gly Gly Gly Arg Lys Lys Asp
   130 135 140
   Glu Glu Ala Glu Ser Ser Gly Gly Tyr Trp Leu Glu Thr Asn Lys Ala
   145 150 155 160
   Gly Asn Gly Val Ile Glu Thr Glu Gly Gly Lys Asp Tyr Val Val Tyr
   165 170 175
   Asn Glu Asp Ala Ile Glu Leu Gly His Asp Lys Thr Gln Asn Pro Met
   180 185 190
   Thr Asp Asn Glu Ile Val Asn Pro Ala Val Lys Ser Glu Glu Gly Tyr
   195 200 205
   Ser Tyr Asp Arg Phe Lys Leu Asp Asn Gly Leu Leu Tyr Asn Glu Pro
   210 215 220
   Gln Ser Ser Ser Tyr His Gln Gly Gly Gly Phe Asp Ser Tyr Phe Glu
   225 230 235 240
   Tyr Phe Arg Phe
<210> 23
   <211> 834
   <212> DNA
   <213> Arabidopsis thaliana
<400> 23
   atggagaaat catcctcaat gaaacaatgg aagaagggtc ctgctcgggg taaaggcggt 60
   ccacaaaacg ctctttgtca gtaccgtgga gtcaggcaaa ggacttgggg caaatgggtg 120
   gctgagatca gagagcccaa gaagagggca agactttggc ttggctcttt cgctacagct 180
   gaagaagcag ctatggctta tgatgaggct gccttgaaac tctatgggca cgacgcatac 240
   ctcaacttac ctcatcttca gcggaataca agaccttctc tgagtaactc tcagaggttc 300
   aaatgggtac cttcaaggaa gtttatatct atgtttcctt catgtggtat gctaaacgtg 360
   aatgctcagc ctagtgttca cataatccag caaagactag aagaactcaa gaaaactgga 420
   cttttatctc aatcctattc ttctagttct tcctccaccg aatcaaaaac taatactagc 480
   tttcttgatg agaagaccag caagggagaa acagacaata tgttcgaagg tggtgatcag 540
   aagaaaccag agatcgacct gaccgagttt cttcagcaac taggaatctt gaaggatgaa 600
   aatgaagcag aaccaagtga ggtagcagag tgtcattccc ctccaccatg gaacgagcaa 660
   gaagaaactg gaagtccttt cagaactgag aatttcagct gggataccct gatcgagatg 720
   ccaagaagtg aaaccacaac tatgcaattt gactccagca acttcggaag ctatgatttt 780
   gaggatgatg tatccttccc ttccatctgg gactactacg gaagcttaga ttga 834
<210> 24
   <211> 277
   <212> PRT
   <213> Arabidopsis thaliana
<400> 24
   Met Glu Lys Ser Ser Ser Met Lys Gln Trp Lys Lys Gly Pro Ala Arg
   1 5 10 15
   Gly Lys Gly Gly Pro Gln Asn Ala Leu Cys Gln Tyr Arg Gly Val Arg
   20 25 30
   Gln Arg Thr Trp Gly Lys Trp Val Ala Glu Ile Arg Glu Pro Lys Lys
   35 40 45
   Arg Ala Arg Leu Trp Leu Gly Ser Phe Ala Thr Ala Glu Glu Ala Ala
   50 55 60
   Met Ala Tyr Asp Glu Ala Ala Leu Lys Leu Tyr Gly His Asp Ala Tyr
   65 70 75 80
   Leu Asn Leu Pro His Leu Gln Arg Asn Thr Arg Pro Ser Leu Ser Asn
   85 90 95
   Ser Gln Arg Phe Lys Trp Val Pro Ser Arg Lys Phe Ile Ser Met Phe
   100 105 110
   Pro Ser Cys Gly Met Leu Asn Val Asn Ala Gln Pro Ser Val His Ile
   115 120 125
   Ile Gln Gln Arg Leu Glu Glu Leu Lys Lys Thr Gly Leu Leu Ser Gln
   130 135 140
   Ser Tyr Ser Ser Ser Ser Ser Ser Thr Glu Ser Lys Thr Asn Thr Ser
   145 150 155 160
   Phe Leu Asp Glu Lys Thr Ser Lys Gly Glu Thr Asp Asn Met Phe Glu
   165 170 175
   Gly Gly Asp Gln Lys Lys Pro Glu Ile Asp Leu Thr Glu Phe Leu Gln
   180 185 190
   Gln Leu Gly Ile Leu Lys Asp Glu Asn Glu Ala Glu Pro Ser Glu Val
   195 200 205
   Ala Glu Cys His Ser Pro Pro Pro Trp Asn Glu Gln Glu Glu Thr Gly
   210 215 220
   Ser Pro Phe Arg Thr Glu Asn Phe Ser Trp Asp Thr Leu Ile Glu Met
   225 230 235 240
   Pro Arg Ser Glu Thr Thr Thr Met Gln Phe Asp Ser Ser Asn Phe Gly
   245 250 255
   Ser Tyr Asp Phe Glu Asp Asp Val Ser Phe Pro Ser Ile Trp Asp Tyr
   260 265 270
   Tyr Gly Ser Leu Asp
   275
<210> 25
   <211> 924
   <212> DNA
   <213> Arabidopsis thaliana
<400> 25
   atggaagaag agcaacctcc ggccaagaaa cgaaacatgg ggagatctag aaaaggttgc 60
   atgaaaggta aaggcggtcc agagaacgcc acgtgtactt tccgtggagt taggcaacgg 120
   acttggggta aatgggtggc tgagatccgt gagcctaacc gtgggactcg tctctggctc 180
   ggcacgttta atacctcggt cgaggccgcc atggcttacg atgaagccgc taagaaactc 240
   tatggacacg aggctaaact caacttggtg cacccacaac aacaacaaca agtagtagtg 300
   aacagaaact tgtctttttc tggccacggg tcgggttctt gggcttataa taagaagctc 360
   gatatggttc atgggttgga ccttggtctc ggccaggcaa gttgttcacg aggttcttgc 420
   tcagagagat cgagttttct acaagaagat gatgatcata gtcataatcg atgttcgtct 480
   tcaagtggtt cgaatctttg ttggttatta cctaaacaaa gtgattcaca agatcaagag 540
   accgttaatg ctacgactag ttatggcggt gaaggcggtg gtggctctac gttaacgttt 600
   tcgaccaatt tgaaaccaaa gaatttgatg agtcagaatt atggattata caatggagct 660
   tggtctaggt ttcttgtggg gcaagaaaag aagacggaac atgacgtgtc atcgtcgtgt 720
   ggatcgtcgg acaacaagga gagtatgttg gttcctagtt gcggcggaga gaggatgcat 780
   aggccggagt tggaagagcg aacaggatat ttggaaatgg atgatctttt ggagattgat 840
   gatttaggtt tgttgattgg caaaaatgga gatttcaaga attggtgttg tgaagagttt 900
   caacatccat ggaattggtt ctga 924
<210> 26
   <211> 306
   <212> PRT
   <213> Arabidopsis thaliana
<400> 26
   Glu Glu Glu Gln Pro Pro Ala Lys Lys Arg Asn Met Gly Arg Ser Arg
   1               5                   10                  15
   Lys Gly Cys Met Lys Gly Lys Gly Gly Pro Glu Asn Ala Thr Cys Thr
               20                  25                  30
   Phe Arg Gly Val Arg Gln Arg Thr Trp Gly Lys Trp Val Ala Glu Ile
           35                  40                  45
   Arg Glu Pro Asn Arg Gly Thr Arg Leu Trp Leu Gly Thr Phe Asn Thr
   50 55 60
   Ser Val Glu Ala Ala Met Ala Tyr Asp Glu Ala Ala Lys Lys Leu Tyr
   65 70 75 80
   Gly His Glu Ala Lys Leu Asn Leu Val His Pro Gln Gln Gln Gln Gln
   85 90 95
   Val Val Val Asn Arg Asn Leu Ser Phe Ser Gly His Gly Ser Gly Ser
   100 105 110
   Trp Ala Tyr Asn Lys Lys Leu Asp Met Val His Gly Leu Asp Leu Gly
   115 120 125
   Leu Gly Gln Ala Ser Cys Ser Arg Gly Ser Cys Ser Glu Arg Ser Ser
   130 135 140
   Phe Leu Gln Glu Asp Asp Asp His Ser His Asn Arg Cys Ser Ser Ser
   145 150 155 160
   Ser Gly Ser Asn Leu Cys Trp Leu Leu Pro Lys Gln Ser Asp Ser Gln
   165 170 175
   Asp Gln Glu Thr Val Asn Ala Thr Thr Ser Tyr Gly Gly Glu Gly Gly
   180 185 190
   Gly Gly Ser Thr Leu Thr Phe Ser Thr Asn Leu Lys Pro Lys Asn Leu
   195 200 205
   Met Ser Gln Asn Tyr Gly Leu Tyr Asn Gly Ala Trp Ser Arg Phe Leu
   210 215 220
   Val Gly Gln Glu Lys Lys Thr Glu His Asp Val Ser Ser Ser Cys Gly
   225 230 235 240
   Ser Ser Asp Asn Lys Glu Ser Met Leu Val Pro Ser Cys Gly Gly Glu
   245 250 255
   Arg Met His Arg Pro Glu Leu Glu Glu Arg Thr Gly Tyr Leu Glu Met
   260 265 270
   Asp Asp Leu Leu Glu Ile Asp Asp Leu Gly Leu Leu Ile Gly Lys Asn
   275 280 285
   Gly Asp Phe Lys Asn Trp Cys Cys Glu Glu Phe Gln His Pro Trp Asn
   290 295 300
   Trp Phe
   305
<210> 27
   <211> 534
   <212> DNA
   <213> Arabidopsis thaliana
<400> 27
   atgcccagga aacggaagtc tcgtggaaca cgagatgtag ctgagattct aaggaaatgg 60
   agagagtaca atgagcagac cgaggcagat tcttgcatcg atggtggtgg ttcaaaacca 120
   atccgaaagg ctcctccaaa acgttcgagg aagggttgta tgaaaggtaa aggtggacct 180
   gaaaatggga tttgtgacta tacaggagtt agacagagga catggggtaa atgggttgct 240
   gagatccgtg agccaggccg aggtgctaag ttatggctcg gtactttctc tagttcatat 300
   gaagctgcat tggcttatga tgaggcttcc aaagctattt acggtcagtc tgcccgactc 360
   aatcttccac tgctgccact gtgtcaggct cggttactgc attttctgat gaatctgaag 420
   tttgtgcacg tgaggataca aatgcaagat ctggttttgg tcagatctct aacttctcgc 480
   atttccaaaa tgttaagtcc aataactgca ttggttaagt tggggcgtta ctag 534
<210> 28
   <211> 177
   <212> PRT
   <213> Arabidopsis thaliana
<400> 28
   Met Pro Arg Lys Arg Lys Ser Arg Gly Thr Arg Asp Val Ala Glu Ile
   1 5 10 15
   Leu Arg Lys Trp Arg Glu Tyr Asn Glu Gln Thr Glu Ala Asp Ser Cys
   20 25 30
   Ile Asp Gly Gly Gly Ser Lys Pro Ile Arg Lys Ala Pro Pro Lys Arg
   35 40 45
   Ser Arg Lys Gly Cys Met Lys Gly Lys Gly Gly Pro Glu Asn Gly Ile
   50 55 60
   Cys Asp Tyr Thr Gly Val Arg Gln Arg Thr Trp Gly Lys Trp Val Ala
   65 70 75 80
   Glu Ile Arg Glu Pro Gly Arg Gly Ala Lys Leu Trp Leu Gly Thr Phe
   85 90 95
   Ser Ser Ser Tyr Glu Ala Ala Leu Ala Tyr Asp Glu Ala Ser Lys Ala
   100 105 110
   Ile Tyr Gly Gln Ser Ala Arg Leu Asn Leu Pro Leu Leu Pro Leu Cys
   115 120 125
   Gln Ala Arg Leu Leu His Phe Leu Met Asn Leu Lys Phe Val His Val
   130 135 140
   Arg Ile Gln Met Gln Asp Leu Val Leu Val Arg Ser Leu Thr Ser Arg
   145 150 155 160
   Ile Ser Lys Met Leu Ser Pro Ile Thr Ala Leu Val Lys Leu Gly Arg
   165 170 175
   Tyr
<210> 29
   <211> 18
   <212> DNA
   <213> Artificial Sequence
   <220>
   <223> Description of Artificial Sequence: Primer
<400> 29
   gagtcttcgg tttcctca 18
<210> 30
   <211> 18
   <212> DNA
   <213> Artificial Sequence
<220>
   <223> Description of Artificial Sequence: Primer
<400> 30
   cgatacgtcg tcatcatc 18

## Description

### BACKGROUND OF THE INVENTION

### 1. FIELD OF THE INVENTION

The present invention relates to a method of producing a transformed plant having improved rooting efficiency and/or prolonged vase life, which comprises transforming a plant with a gene as defined in claim 1.

### 2. DESCRIPTION OF RELATED ART

Cultivated plants are grown by natural plant mechanisms such as breeding by seeds and bulbs, and by cloning technique such as cutting (herbaceous cutting and scion) and tissue culture. Particularly, concerning 3 important cut flowers, chrysanthemum, carnation, and rose, when a good variety is produced, its branches and buds are propagated by cutting (herbaceous cutting and scion), and the propagated plants are used for the production of cut flowers and the maintenance of the plant variety. To raise the productivity of this variety, the propagation efficiency of cutting should be raised. To raise the productivity to the highest, the rooting ability of cutting should be improved. To address the problem, treatment with a chemical such as auxins represented by the trade name of Rooton or the like has been conducted. However, it is never sufficient, it costs much, and it takes time under current conditions. In the meantime, it goes without saying that the property of keeping the quality of flowers (prolonged vase life) is a very important character of cut flowers. Biochemical examinations regarding vase life have been conducted, so that, for example, a technique of physically absorbing ethylene, which is an aging hormone, has been developed. However, in this method, the vase life controlled by ethylene does not represent a substantial improvement with regard to cut flowers, but rather only a partial improvement. Furthermore, the varieties of plants that can be improved by absorption of ethylene or suppression of ethylene generation are limited, so that improvement in applicability to more various plant varieties and in plants' own conditions has been expected. Besides, there has been no known means for improving rooting ability and prolonging the vase life of cut flowers at the same time.

To date, in the case of artificially producing a plant having improved propagation ability in terms of clonal productivity or vase life, techniques such as selection and crossing of lines showing excellent characters relating to each of these properties have been employed. However, while the selection method requires long term, the crossing method can be used only between related species. Thus, it has been difficult to produce plants having improved propagation efficiency with reference to cutting and improved vase life.

With the progress of biotechnology in recent years, the production of various plants has been attempted using techniques such as transformation technology, whereby a specific gene derived from an organism of a different species is introduced into a plant. To date, regarding the promotion of rooting, there has been a case involving the introduction of a rolC gene into a carnation. However, since the rolC gene itself has been known to promote dwarfing or to enhance branching in a various plants, the practical application thereof may be difficult [J. Amer. Soc. Hort. Sci. 126: 13-18 (2001)]. Suppressing the generation of ethylene or making the ethylene-receptive mechanism insensitive has been attempted by genetic modification. It has been reported that the produced plant so far could have partially improved vase life (suppressed aging of flower petals and the like) [HortScience 30: 970-972 (1995); Mol. Breed. 5: 301-308 (1999)].

In the meantime, plants inhabit naturally exposing themselves to various environmental stresses such as drought, high temperature, low temperature, or salinity. Since plants are unable to take action to protect themselves from stress by moving as animals do, they have acquired various stress-resistance mechanisms in the process of evolution. For example, low-temperature-resistant plants (e.g., Arabidopsis, spinach, lettuce, pea, barley, and beet) have a lower content of unsaturated fatty acid in biological membrane lipids compared with the case of low-temperature-sensitive plants (e.g., corn, rice, pumpkin, cucumber, banana, and tomato), so that when the low-temperature-resistant plants are exposed to low temperature, the phase transition of biological membrane lipids occurs with difficulty and thus low temperature injuries are not easily caused. To date, in the case of artificially producing environmental stress-resistant plants, techniques such as selection and crossing of lines with resistance against drought, low temperatures, or salinity have been employed. However, while the selection method requires long term, the crossing method can be used only between related species. Thus, it has been difficult to produce plants having strong resistance against various environmental stress.

With the progress of biotechnology in recent years, the production of plants resistant to drought, low temperature, salinity, or the like has been attempted using techniques such as transformation technology, whereby a specific gene derived from an organism of a different species is introduced into a plant. An example of a plant that is thought to be the most practical use is an environmental-stress-resistant transformed plant [JP Patent Nos. 3178672 see also CA 2,269,105, and JP 3183458] that has been produced by introducing a gene, wherein a DNA (referred to as DREB gene) encoding a transcription factor having functions to bind to dehydration responsive element (DRE) so as to activate the transcription of a gene located downstream of DRE is ligated downstream of a stress-responsive promoter. By the use of this method, transformed plants having improved resistance against forms of environmental stresses (e.g., drought stress, low temperature stress, and salinity stress) and exhibiting no dwarfing are generated. However, such stress resistance has been conferred when plants are assumed to be cultivated under special conditions (e.g., cultivated continuously in desert areas, areas damaged by salinity, and low temperature areas), or when plants are exposed temporarily to extreme forms of environmental stress. It has not been reported that the thus-conferred resistance against stress has a favorable effect on the rooting efficiency for propagation by cutting, the ordinary form of cultivation, the vase life of cut flowers (prolonged life of cut flowers), the ordinary form of the distribution of products, or that of consumption.

### SUMMARY OF THE INVENTION

An object of the present invention is to provide a method of producing transformed plants having better propagation efficiency by cutting that is improved by increasing rooting efficiency, and having improved vase life.

We have conducted experiments as a result of intensive studies to achieve the above object. We have obtained chrysanthemum transformed with a plant transformation plasmid pBI29AP:DREB1A (produced for the purpose of conferring stress resistance as disclosed in Example 5 of Japanese Patent No. 3178672), propagated the plant by cloning technique, produced cut flowers from the plant, examined the vase life of this plant, and compared the chrysanthemum without gene transformation. We found clear superiority of this transformed plant in rooting efficiency, propagation ability by cutting, and vase life (prolonged life of cut flowers). That is, the present invention is as follows
1. A method of producing a transformed plant having improved rooting efficiency and/or prolonged vase life,
   comprising transforming a plant
   using a gene wherein a DNA encoding a protein that binds to a stress-responsive element contained in a stress-responsive promoter and activates the transcription of a gene located downstream of the element is ligated downstream of the stress-responsive promoter, and
   wherein the DNA encoding a protein that binds to a stress-responsive element and activates the transcription of a gene located downstream of the element is at least one gene selected from the group consisting of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene.
2. The method of producing a transformed plant of item 1, wherein the DNA encoding a protein that binds to a stress-responsive element and regulates the transcription of a gene located downstream of the element is at least one DNA selected from the group consisting of:
   (a) a DNA comprising a nucleotide sequence having at least 80% or more homology with the nucleotide sequence of a DNA of at least one of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene, and encoding a protein having activity to bind to a stress-responsive element and regulate the transcription of a gene located downstream of the element; and
   (b) a DNA hybridizing under stringent conditions to a DNA complementary to a DNA of at least one of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene, and encoding a protein having activity to bind to a stress-responsive element and regulate the transcription of a gene located downstream of the element.
3. The method of producing a transformed plant of item 1 or 2, wherein the stress-responsive promoter is at least one promoter selected from the group consisting of rd29A gene promoter, rd29B gene promoter, rd17 gene promoter, rd22 gene promoter, DREB1A gene promoter, cor6.6 gene promoter, cor15a gene promoter, erd 1 gene promoter, and kin 1 gene promoter.
4. The method of producing a transformed plant of any one of items 1 to 3, wherein the DNA of a stress-responsive promoter is at least one DNA selected from the group consisting of:
   (a) a DNA comprising a nucleotide sequence having at least 80% or more homology with the nucleotide sequence of a DNA of at least one of rd29A gene promoter, rd29B gene promoter, rd17 gene promoter, rd22 gene promoter, DREB1A gene promoter, cor6.6 gene promoter, cor15a gene promoter, erd1 gene promoter, and kin1 gene promoter, and having activity as the DNA of the stress-responsive promoter; and
   (b) a DNA hybridizing under stringent conditions to a DNA complementary to a DNA of at least one of rd29A gene promoter, rd29B gene promoter, rd17 gene promoter, rd22 gene promoter, DREB1A gene promoter, cor6.6 gene promoter, cor15a gene promoter, erd1 gene promoter, and kin1 gene promoter, and having activity as the DNA of the stress-responsive promoter.

### BRIEF DESCRIPTION OF THE DRAWINGS

Fig. 1 shows the structure between RB and LB of a rd29A-DREB1A vector.
Fig. 2-1 shows 1 to 1 alignment, common sequences and homology % at the nucleotide sequence level between DREB1A as a standard and each one of DREB1B to DREB1F.
Fig. 2-2 shows 1 to 1 alignment, common sequences and homology % at the nucleotide sequence level between DREB1A as a standard and each one of DREB1B to DREB1F (continued from Fig. 2-1).

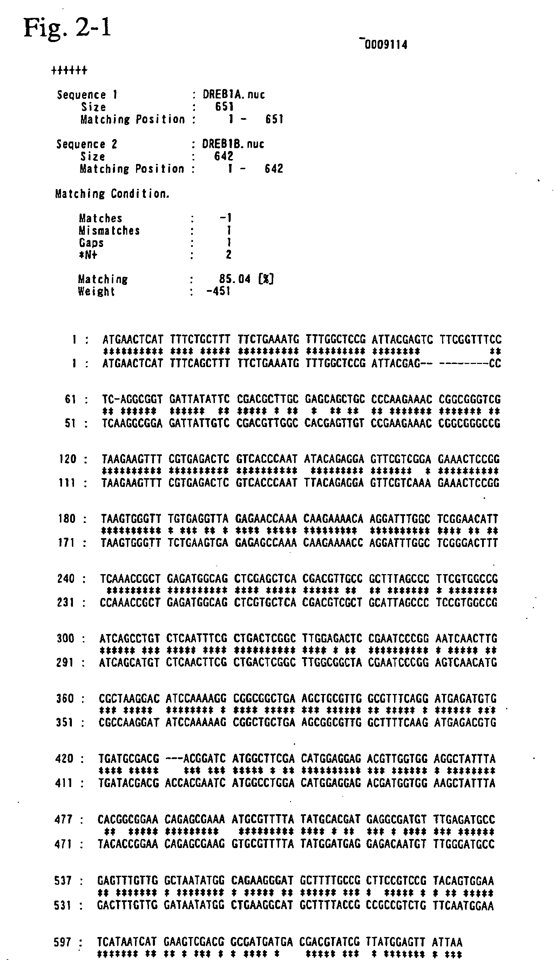

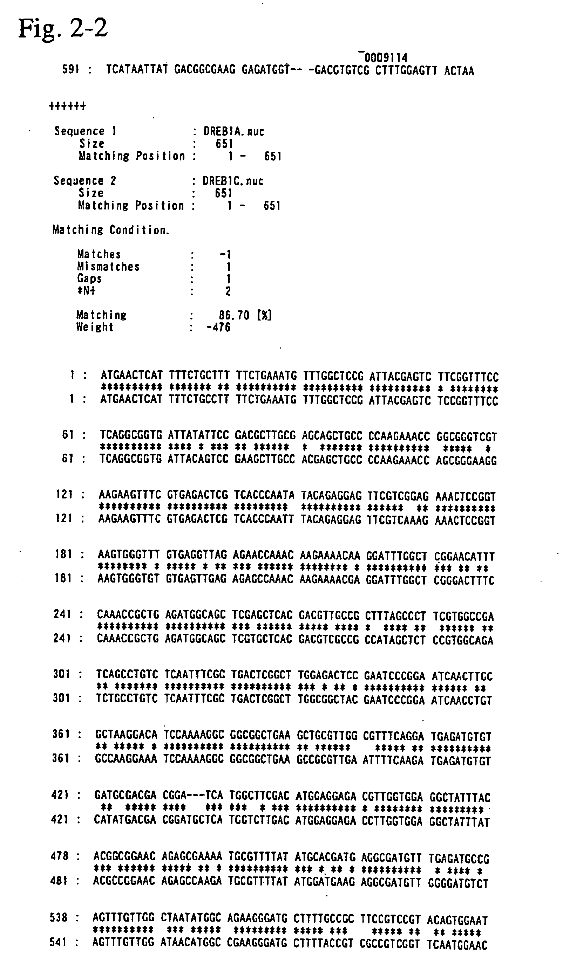

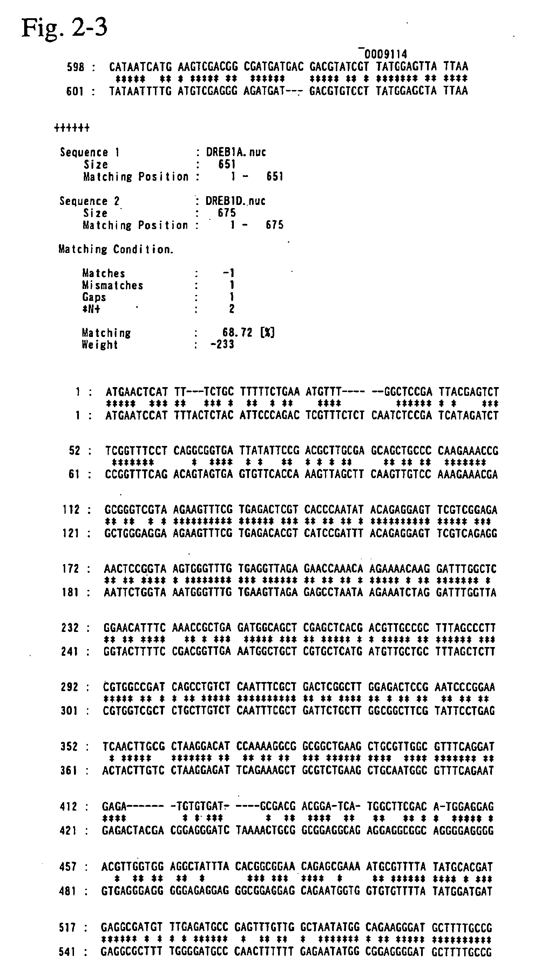

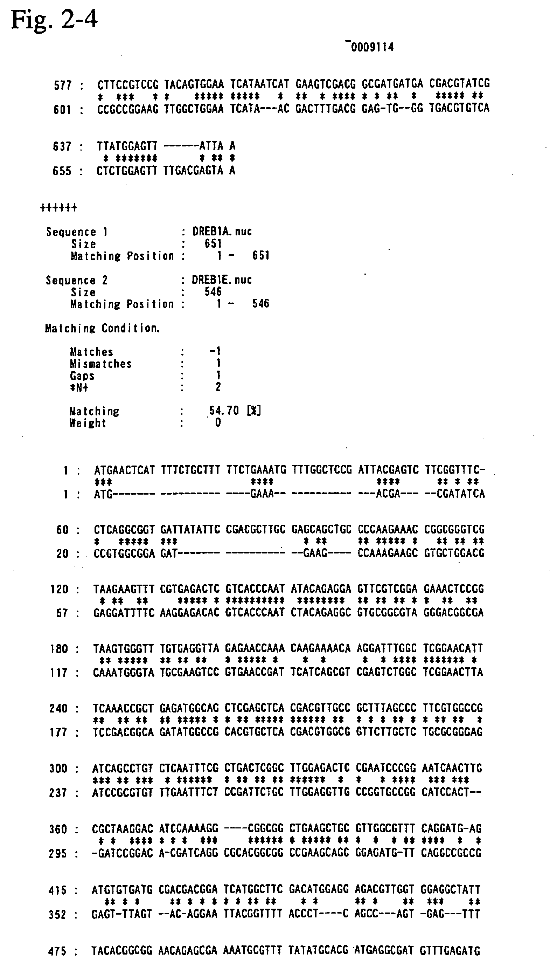

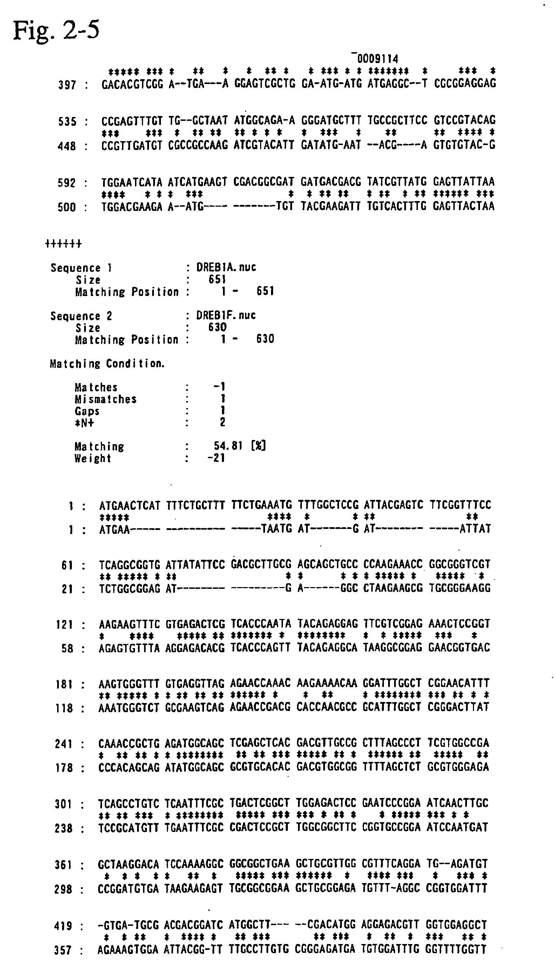

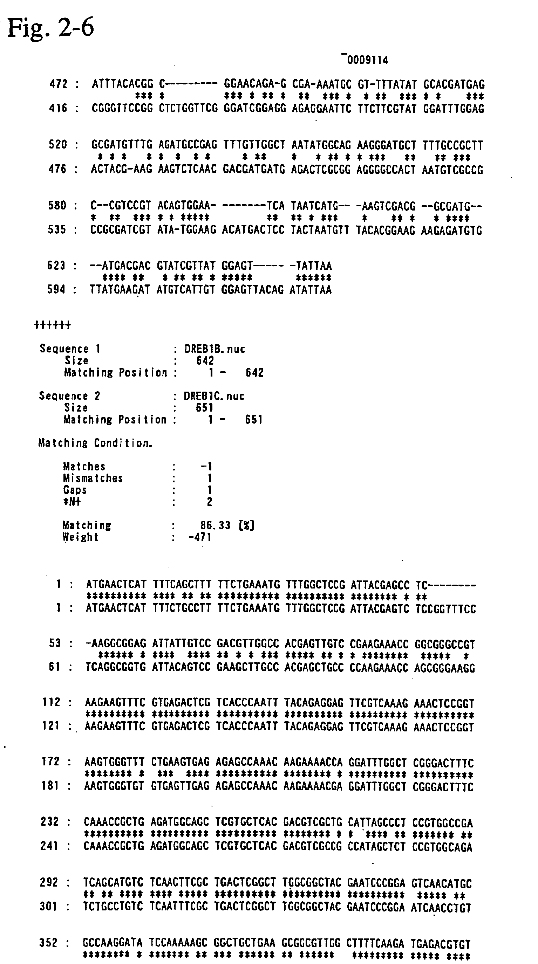

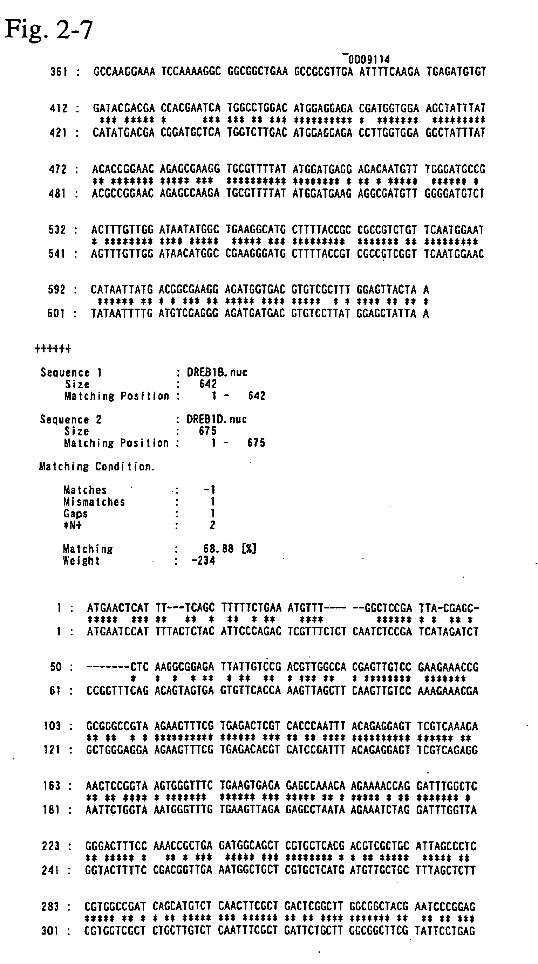

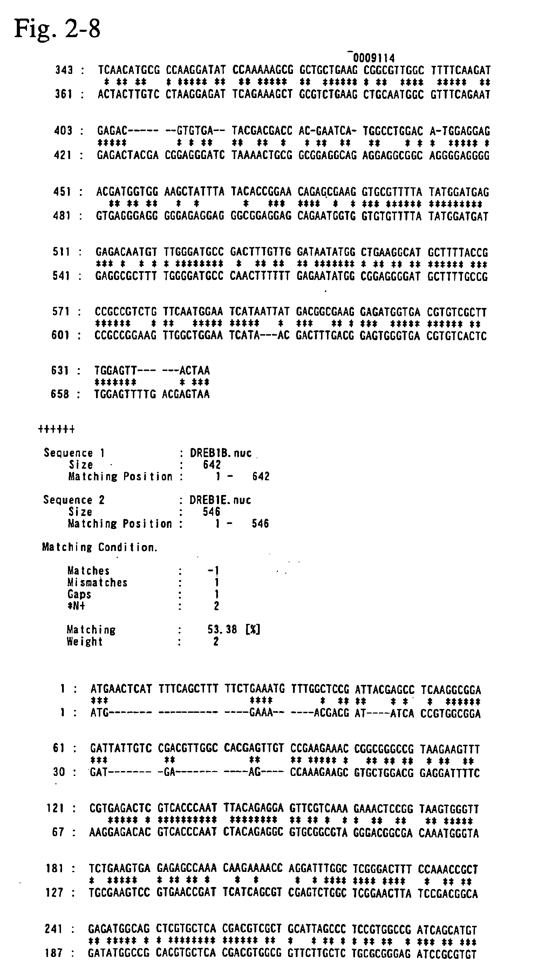

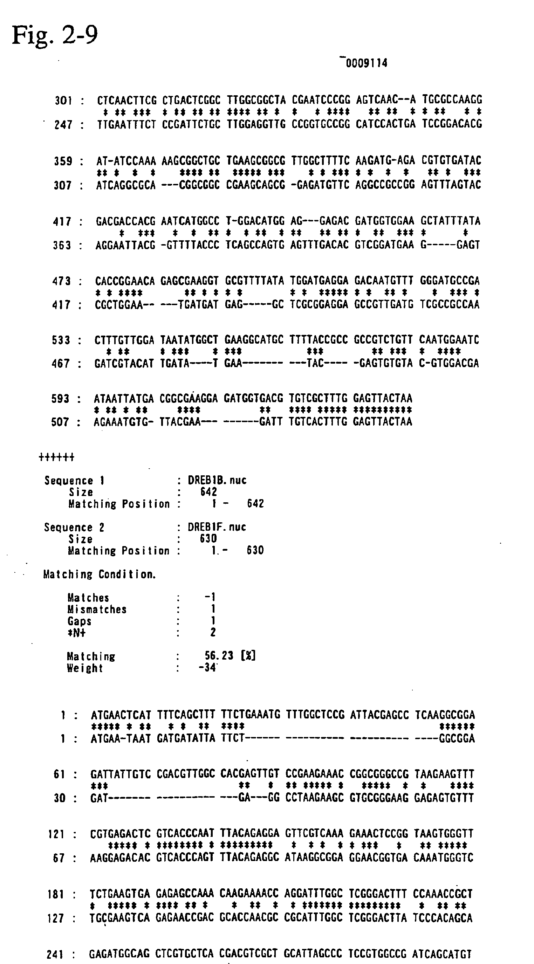

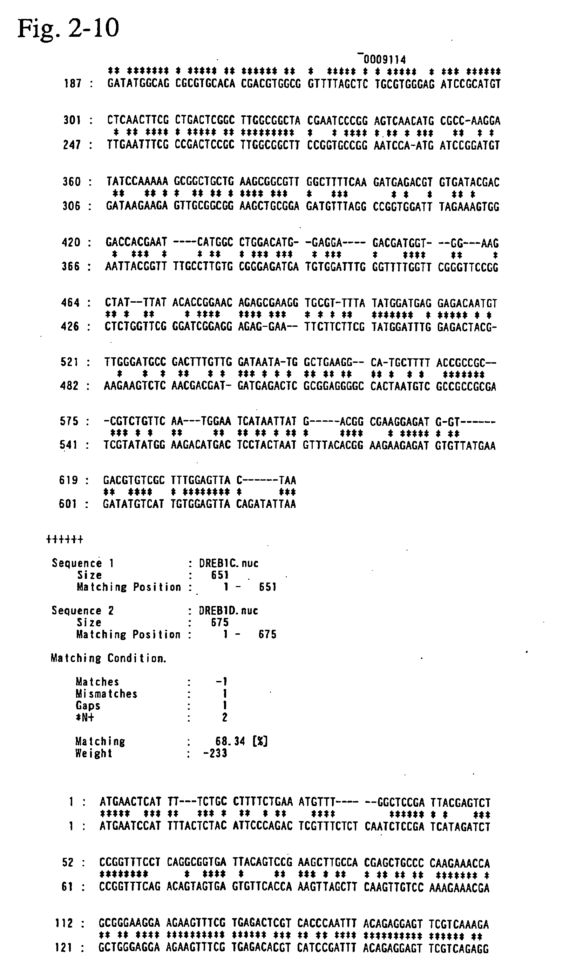

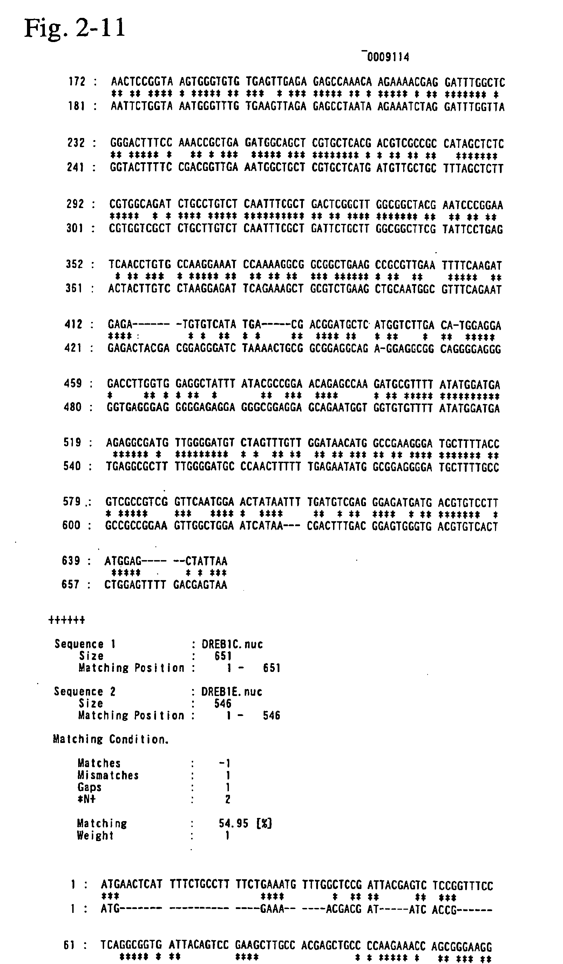

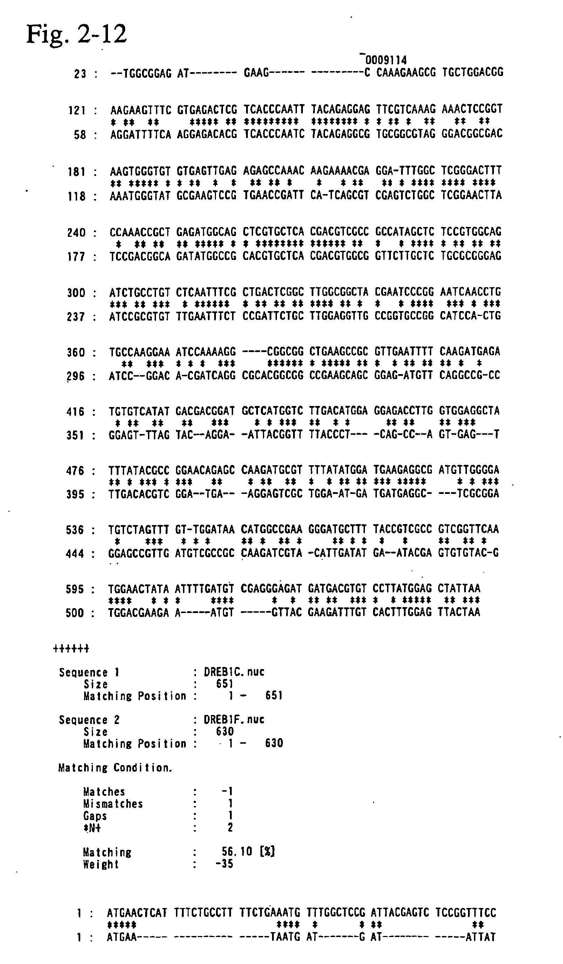

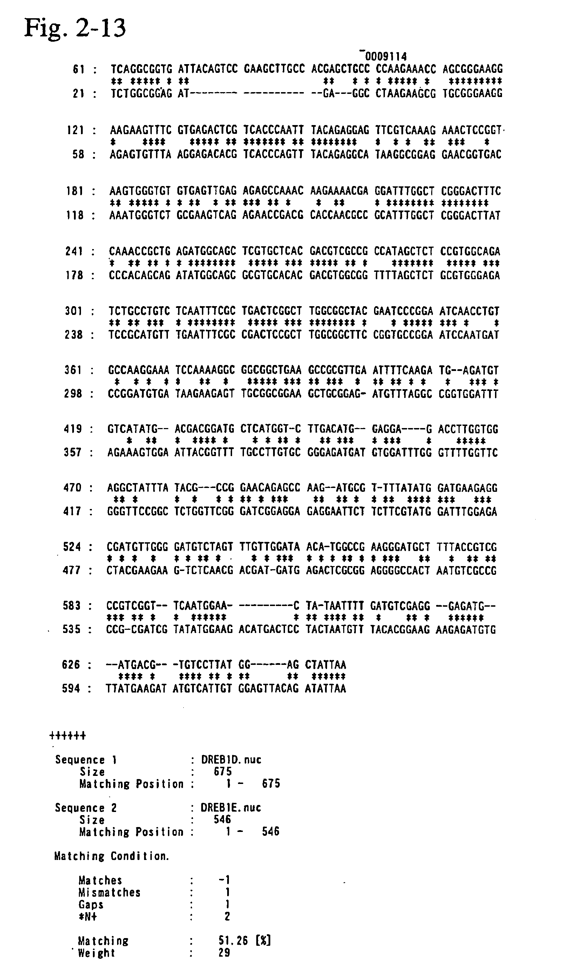

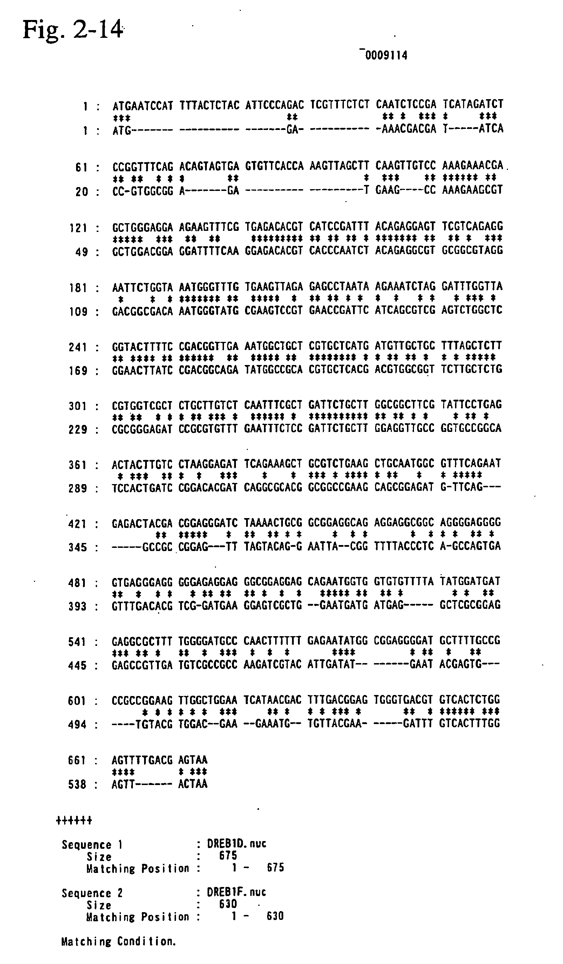

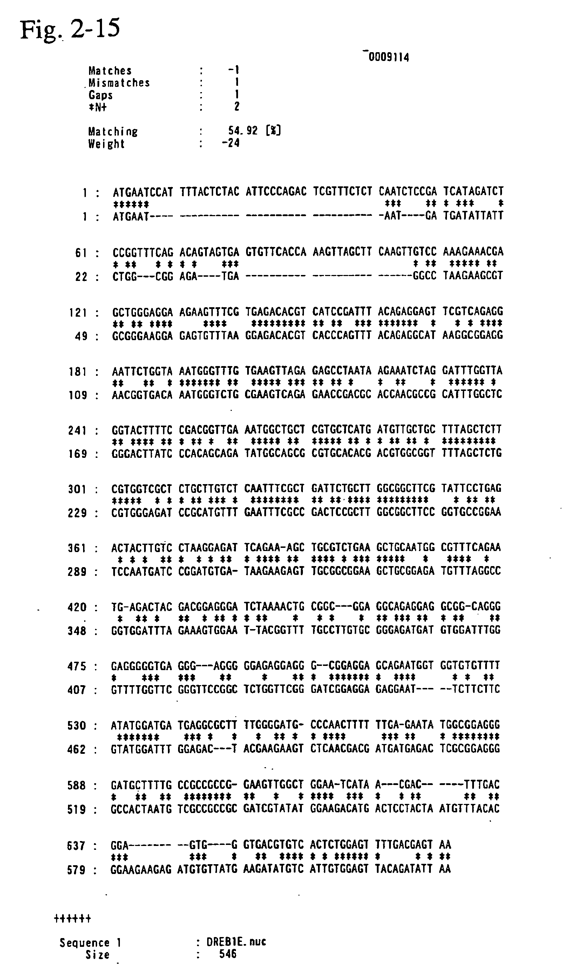

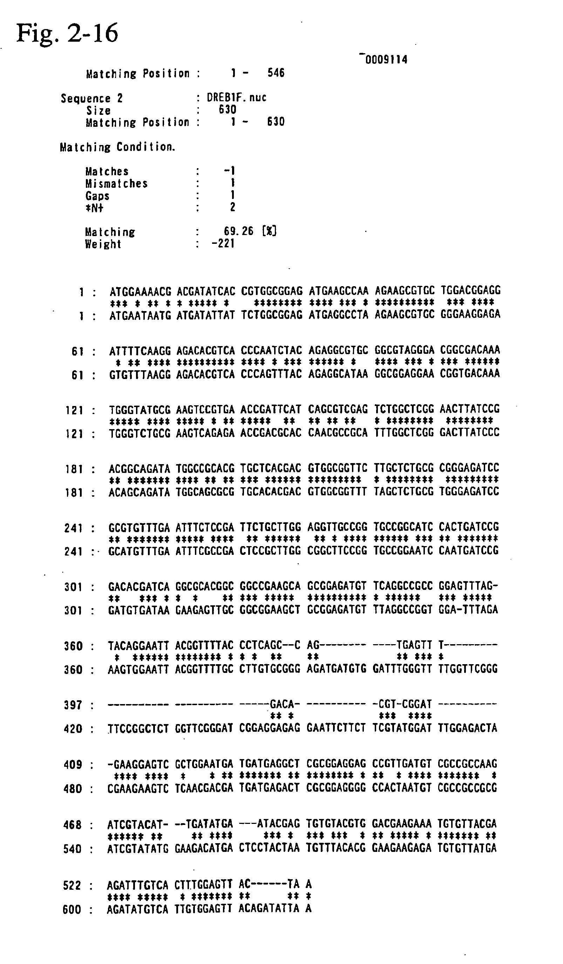

Fig. 2-3 shows 1 to 1 alignment, common sequences and homology % at the nucleotide sequence level between DREB1A as a standard and each one of DREB 1 B to DREB1F (continued from Fig. 2-2).
Fig. 2-4 shows 1 to 1 alignment, common sequences and homology % at the nucleotide sequence level between DREB1A as a standard and each one of DREB1B to DREB1F (continued from Fig. 2-3).
Fig. 2-5 shows 1 to 1 alignment, common sequences and homology % at the nucleotide sequence level between DREB1A as a standard and each one of DREB1B to DREB1F (continued from Fig. 2-4).
Fig. 2-6 shows 1 to 1 alignment, common sequences and homology % at the nucleotide sequence level between DREB1A as a standard and each one of DREB1B to DREB1F (continued from Fig. 2-5).
Fig. 2-7 shows 1 to 1 alignment, common sequences and homology % at the nucleotide sequence level between DREB1A as a standard and each one of DREB1B to DREB1F (continued from Fig. 2-6).
Fig. 2-8 shows 1 to 1 alignment, common sequences and homology % at the nucleotide sequence level between DREB1A as a standard and each one of DREB1B to DREB1F (continued from Fig. 2-7).
Fig. 2-9 shows 1 to 1 alignment, common sequences and homology % at the nucleotide sequence level between DREB1A as a standard and each one of DREB1B to DREB1F (continued from Fig. 2-8).
Fig. 2-10 shows 1 to 1 alignment, common sequences and homology % at the nucleotide sequence level between DREB1A as a standard and each one of DREB1B to DREB1F (continued from Fig. 2-9).
Fig. 2-11 shows 1 to 1 alignment, common sequences and homology % at the nucleotide sequence level between DREB1A as a standard and each one of DREB1B to DREB1F (continued from Fig. 2-10).
Fig. 2-12 shows 1 to 1 alignment, common sequences and homology % at the nucleotide sequence level between DREB1A as a standard and each one of DREB1B to DREB1F (continued from Fig. 2-11).
Fig. 2-13 shows 1 to 1 alignment, common sequences and homology % at the nucleotide sequence level between DREB1A as a standard and each one of DREB1B to DREB1F (continued from Fig. 2-12).
Fig. 2-14 shows 1 to 1 alignment, common sequences and homology % at the nucleotide sequence level between DREB1A as a standard and each one of DREB1B to DREB1F (continued from Fig. 2-13).
Fig. 2-15 shows 1 to 1 alignment, common sequences and homology % at the nucleotide sequence level between DREB1A as a standard and each one of DREB1B to DREB1F (continued from Fig. 2-14).
Fig. 2-16 shows 1 to 1 alignment, common sequences and homology % at the nucleotide sequence level between DREB1A as a standard and each one of DREB1B to DREB1F (continued from Fig. 2-15).

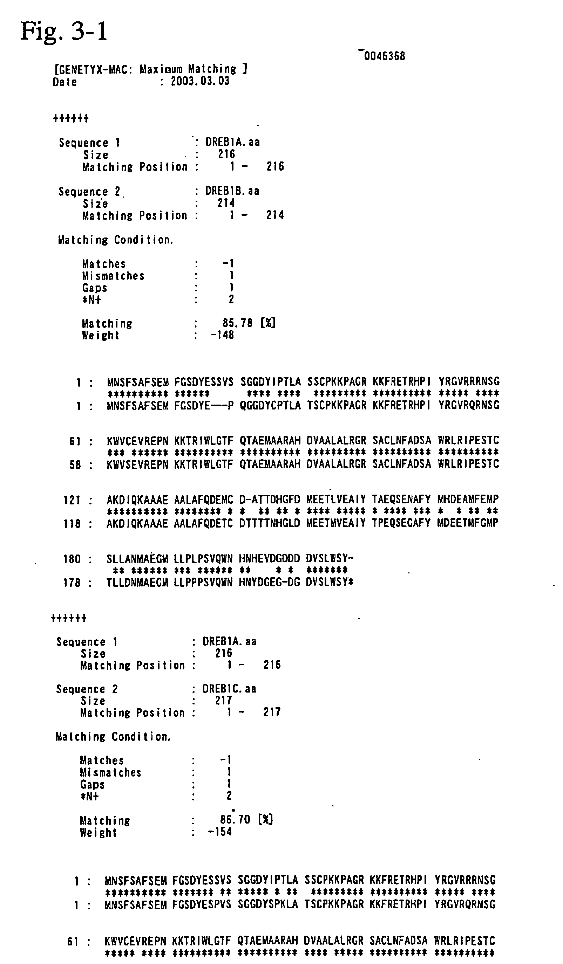

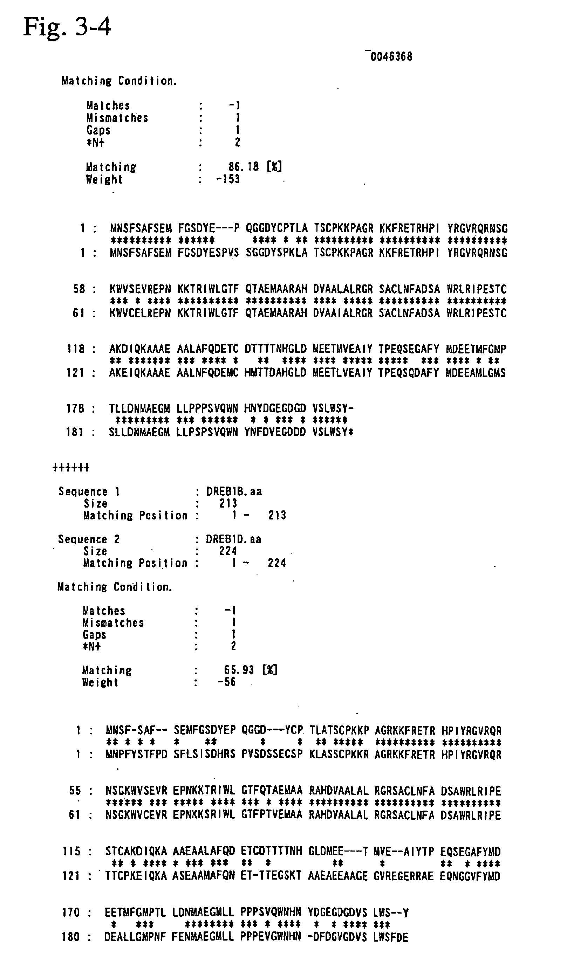

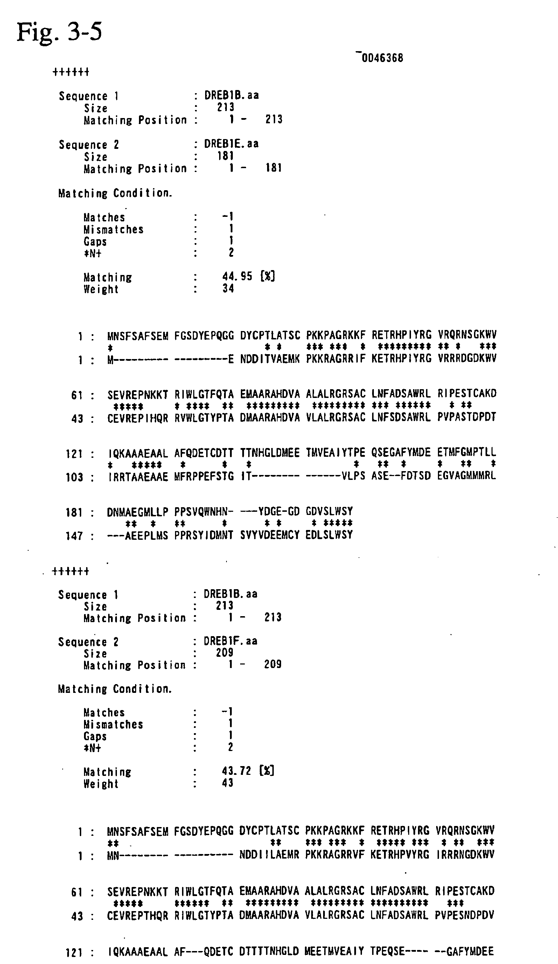

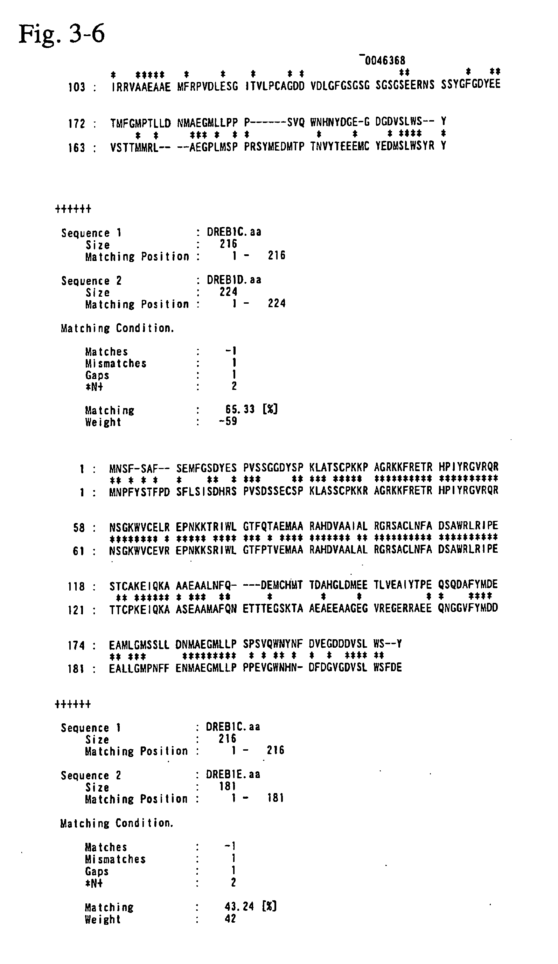

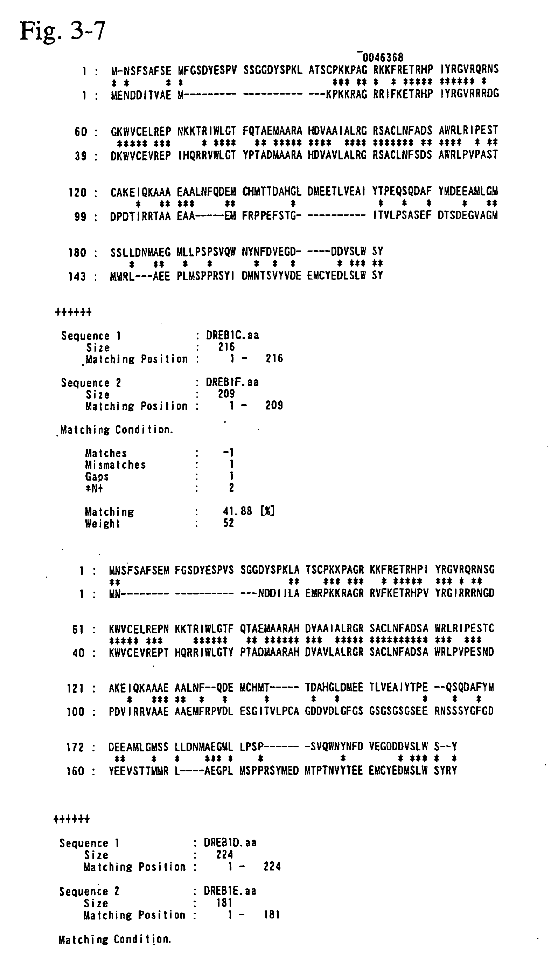

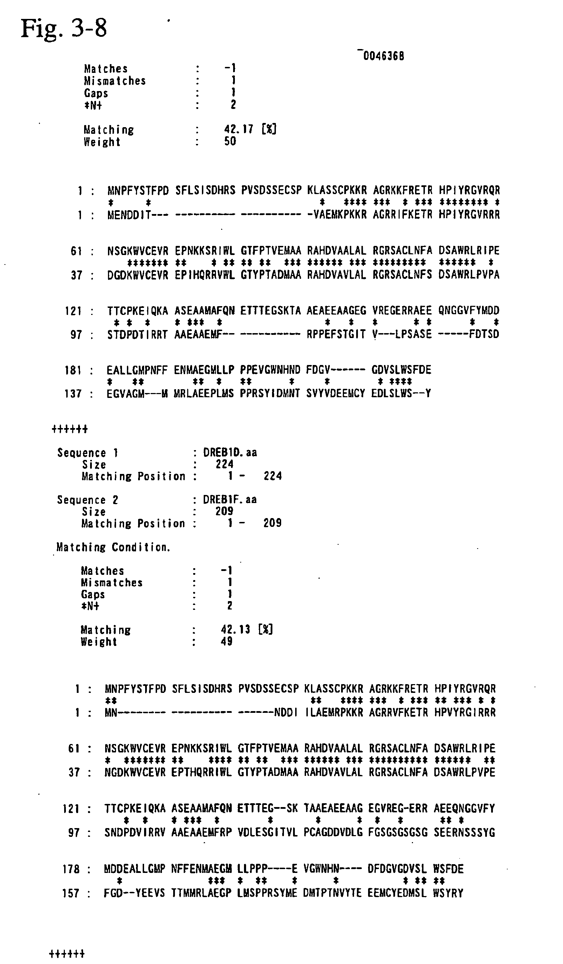

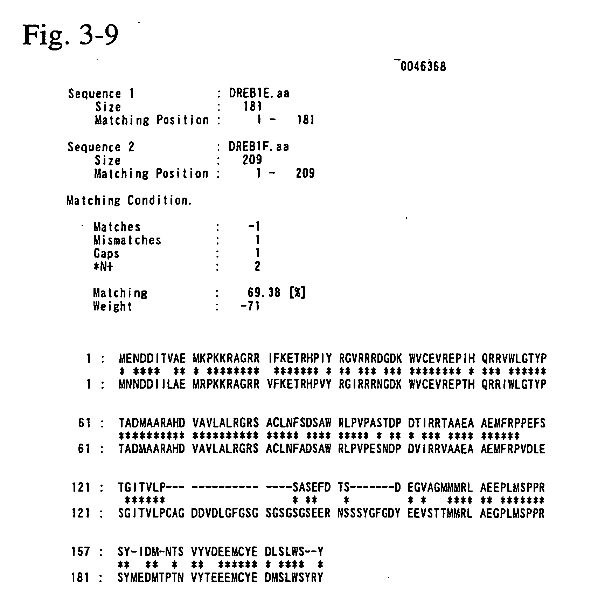

Fig. 3-1 shows 1 to 1 alignment, common sequences and homology % at the amino acid sequence level between DREB1A as a standard and each one of DREB1B to DREB1F.

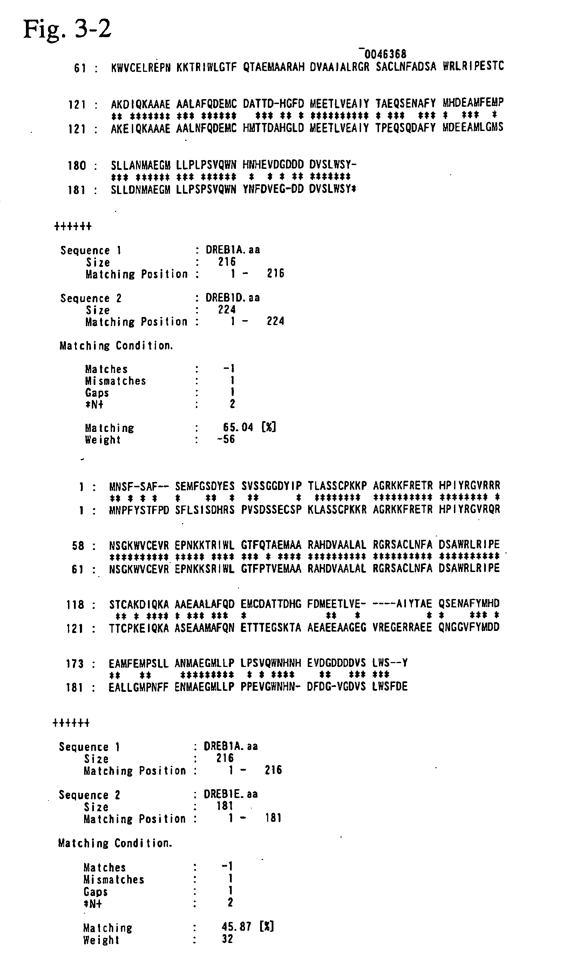

Fig. 3-2 shows 1 to 1 alignment, common sequences and homology % at the amino acid sequence level between DREB1A as a standard and each one of DREB1B to DREB1F (continued from Fig. 3-1).

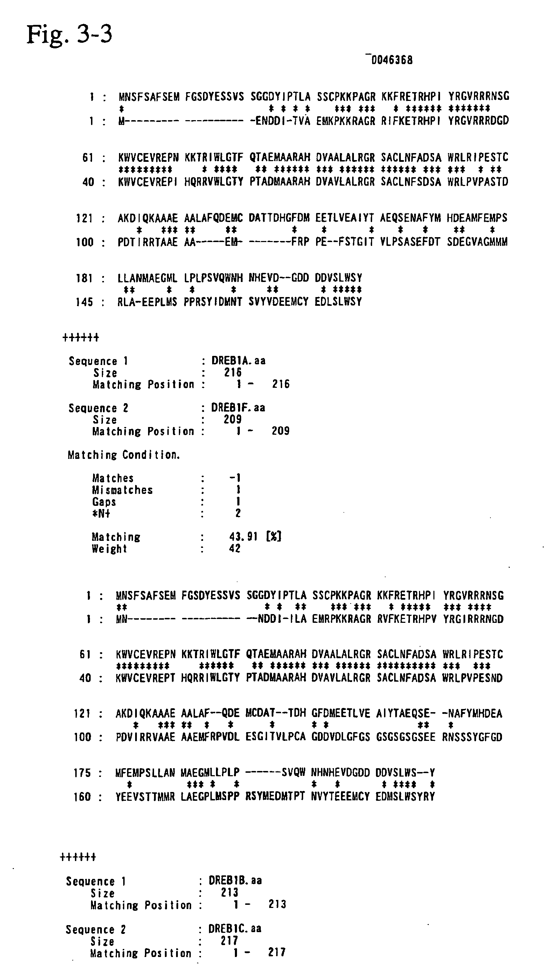

Fig. 3-3 shows 1 to 1 alignment, common sequences and homology % at the amino acid sequence level between DREB1A as a standard and each one of DREB1B to DREB1F (continued from Fig. 3-2).
Fig. 3-4 shows 1 to 1 alignment, common sequences and homology % at the amino acid sequence level between DREB1A as a standard and each one of DREB1B to DREB1F (continued from Fig. 3-3).
Fig. 3-5 shows 1 to 1 alignment, common sequences and homology % at the amino acid sequence level between DREB1A as a standard and each one of DREB1B to DREB1F (continued from Fig. 3-4).
Fig. 3-6 shows 1 to 1 alignment, common sequences and homology % at the amino acid sequence level between DREB1A as a standard and each one of DREB1B to DREB1F (continued from Fig. 3-5).
Fig. 3-7 shows 1 to 1 alignment, common sequences and homology % at the amino acid sequence level between DREB1A as a standard and each one of DREB1B to DREB1F (continued from Fig. 3-6).
Fig. 3-8 shows 1 to 1 alignment, common sequences and homology % at the amino acid sequence level between DREB1A as a standard and each one of DREB1B to DREB1F (continued from Fig. 3-7).
Fig. 3-9 shows 1 to 1 alignment, common sequences and homology % at the amino acid sequence level between DREB1A as a standard and each one of DREB1B to DREB1F (continued from Fig. 3-8).

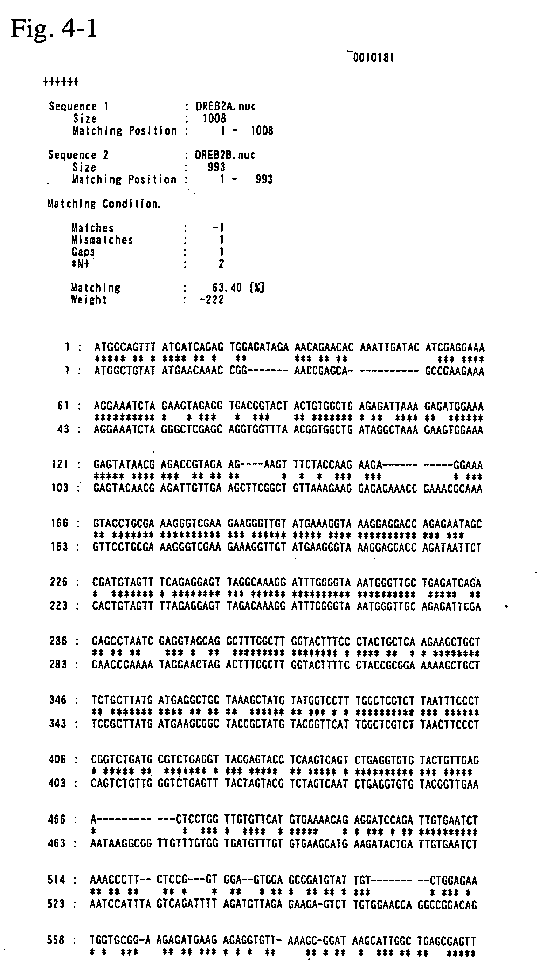

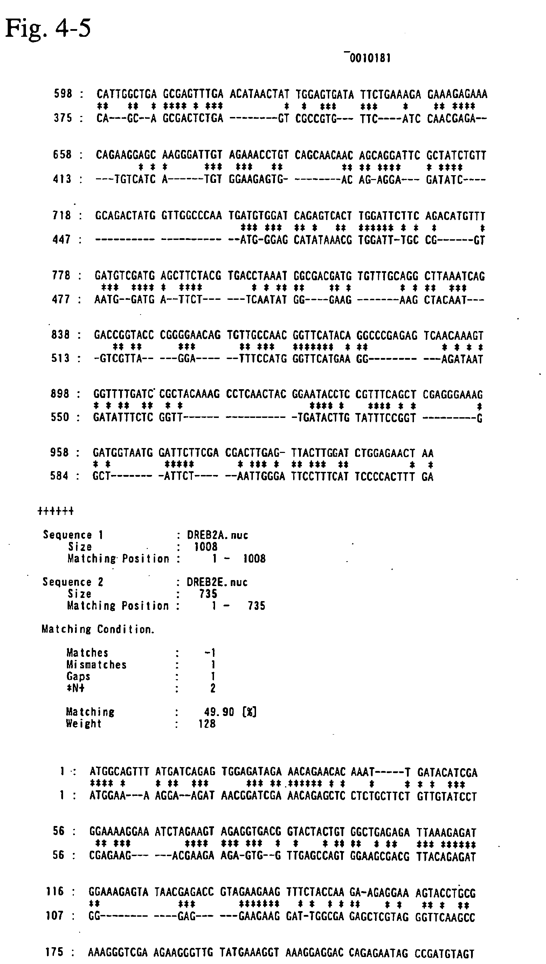

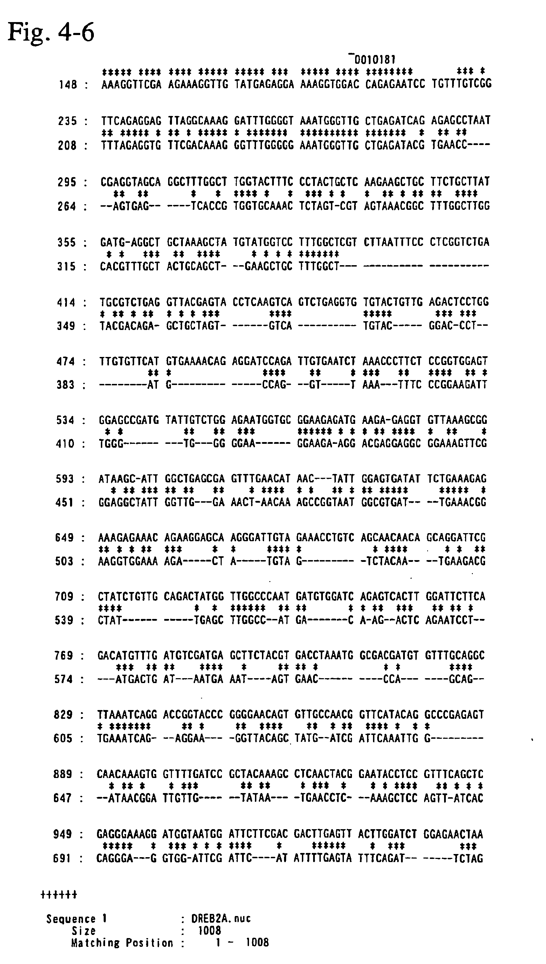

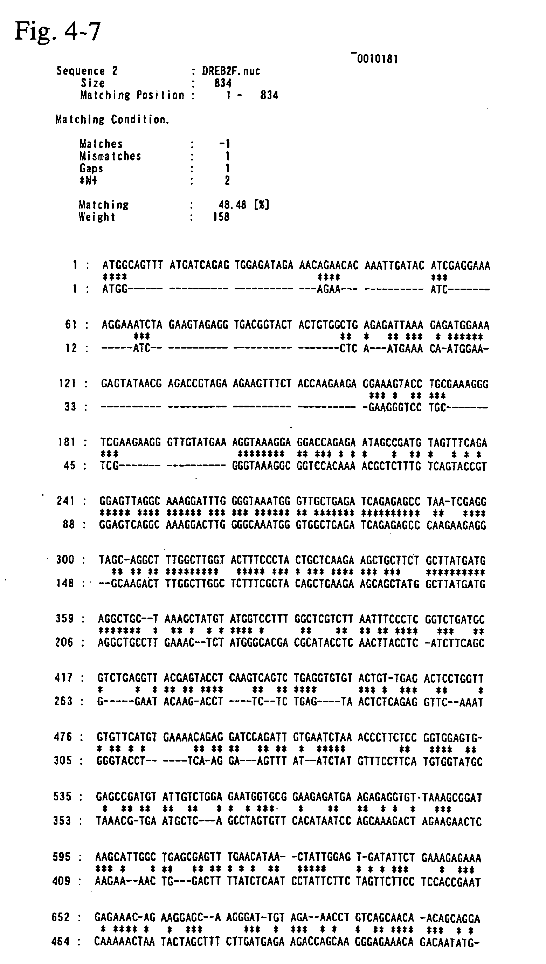

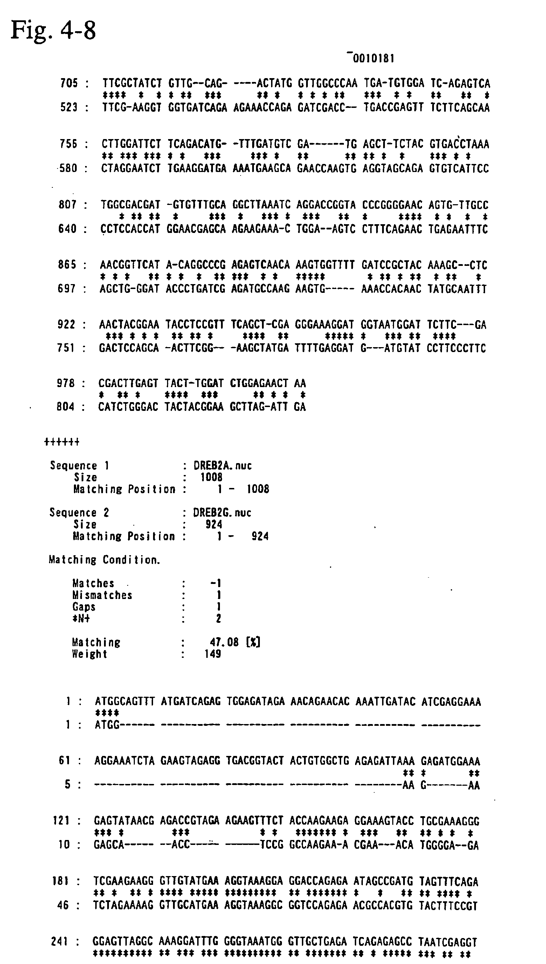

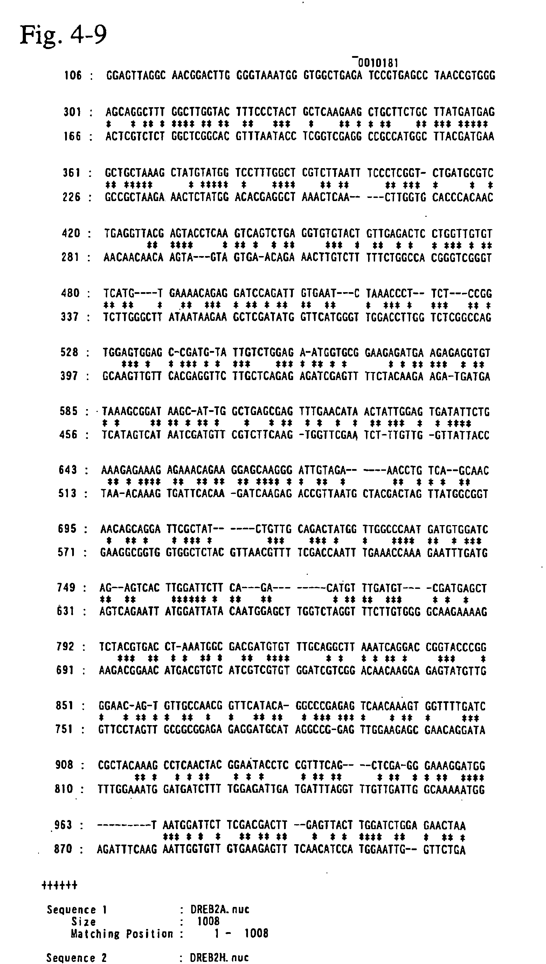

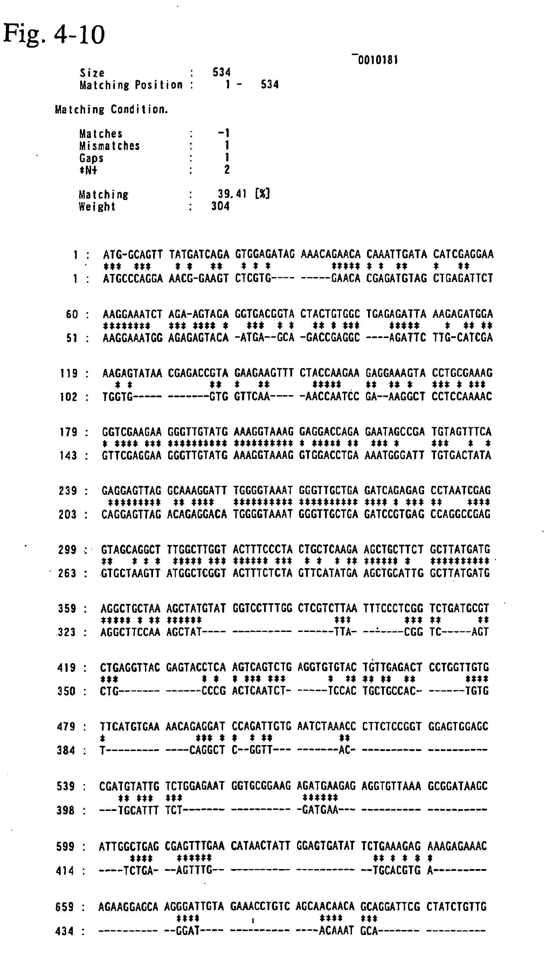

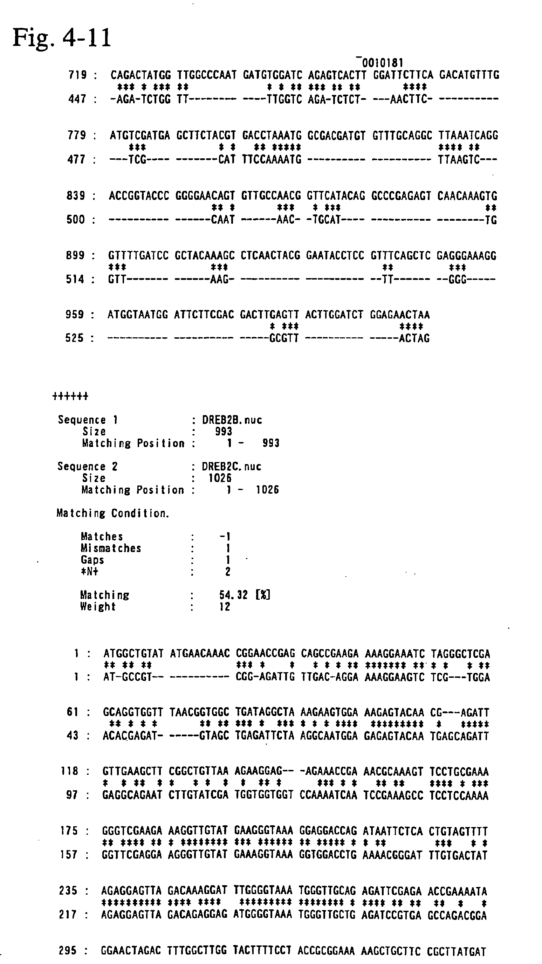

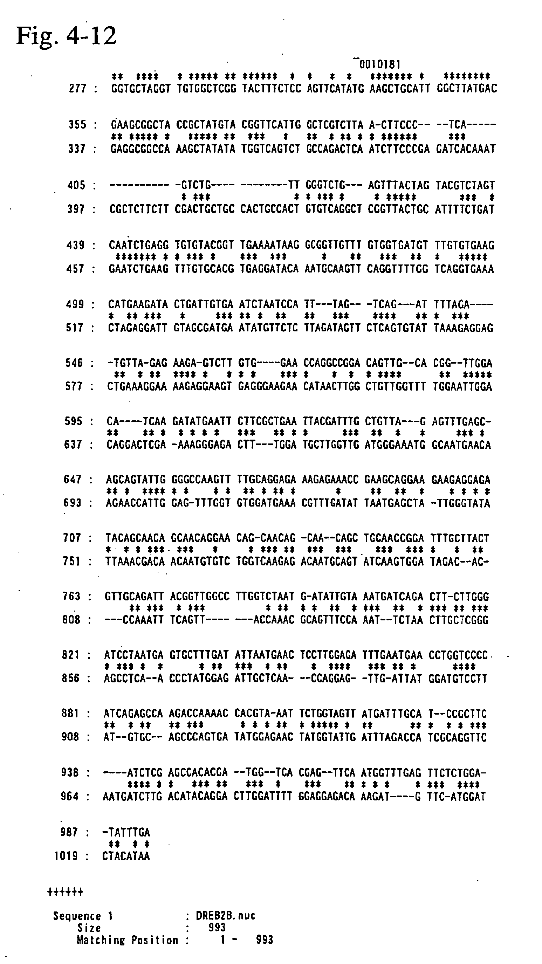

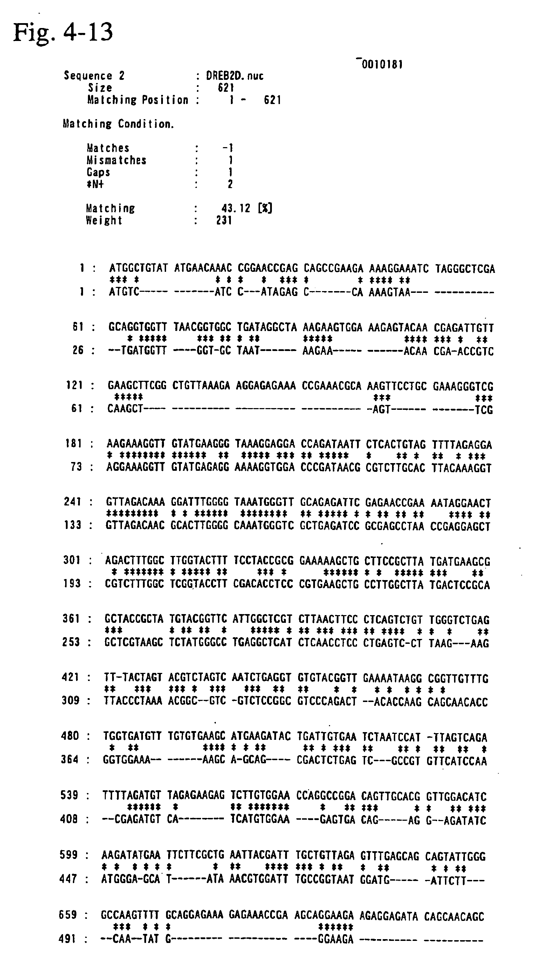

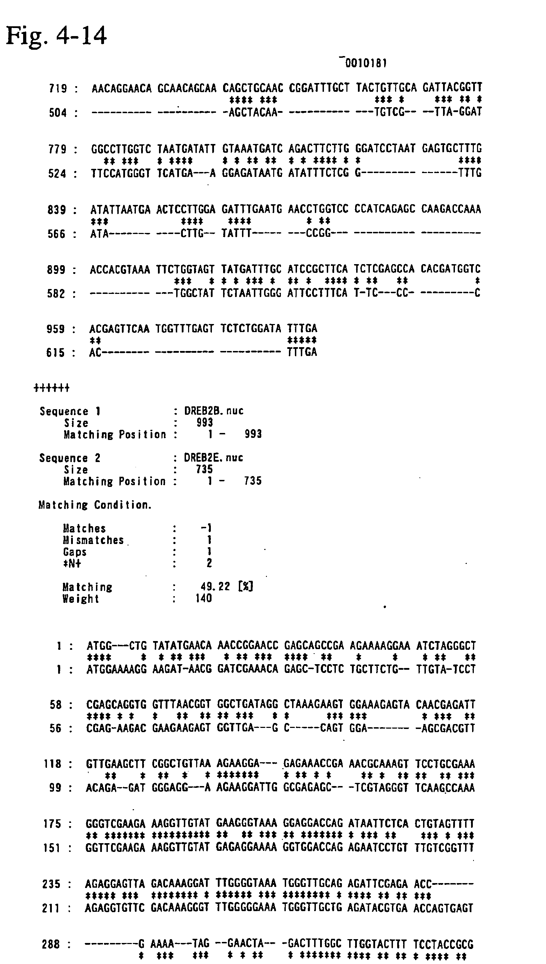

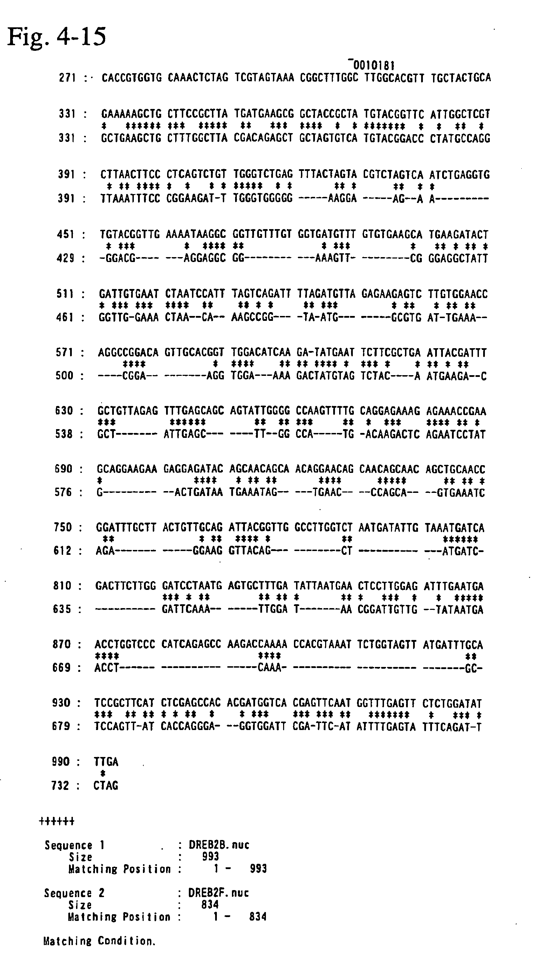

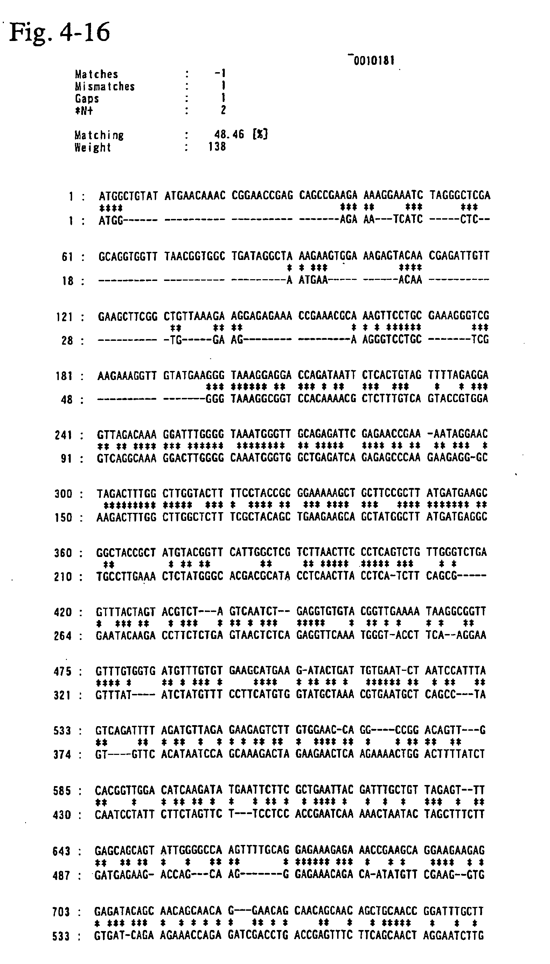

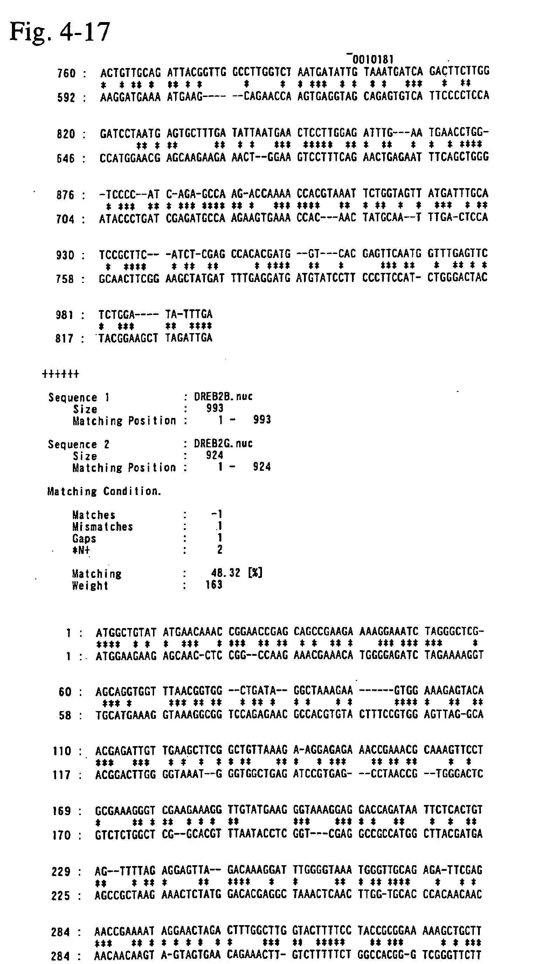

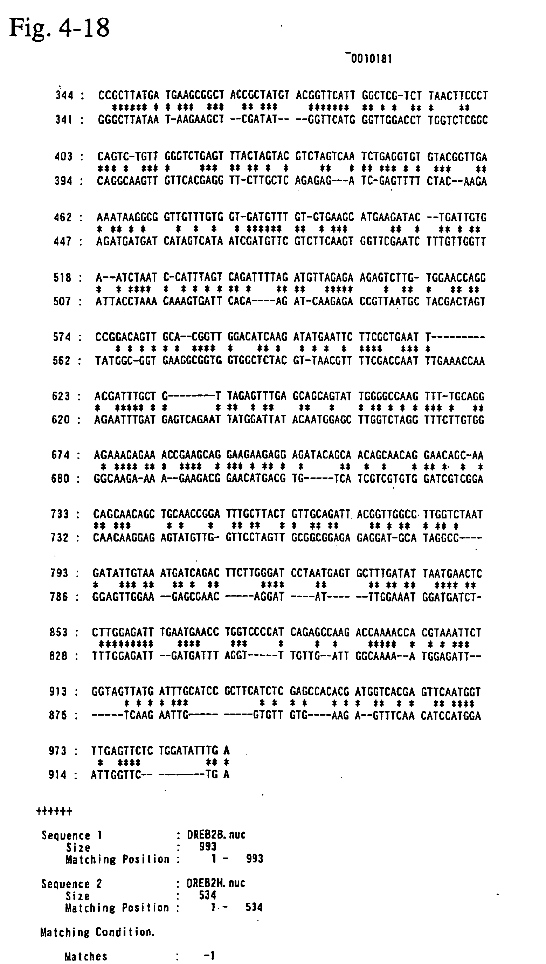

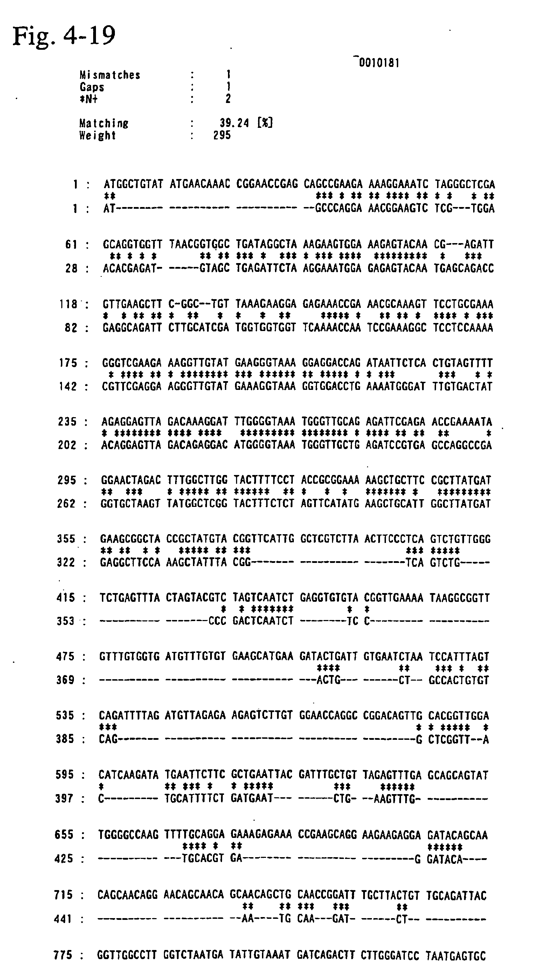

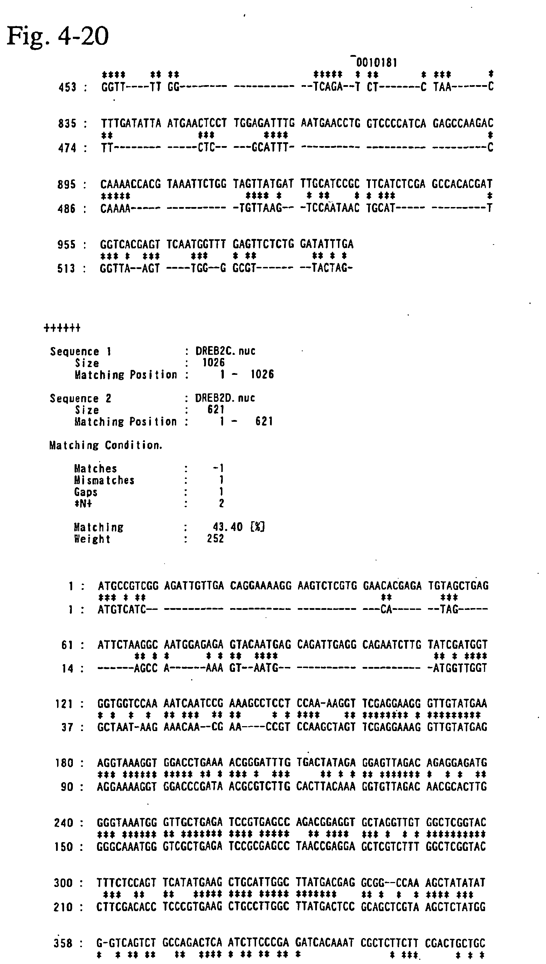

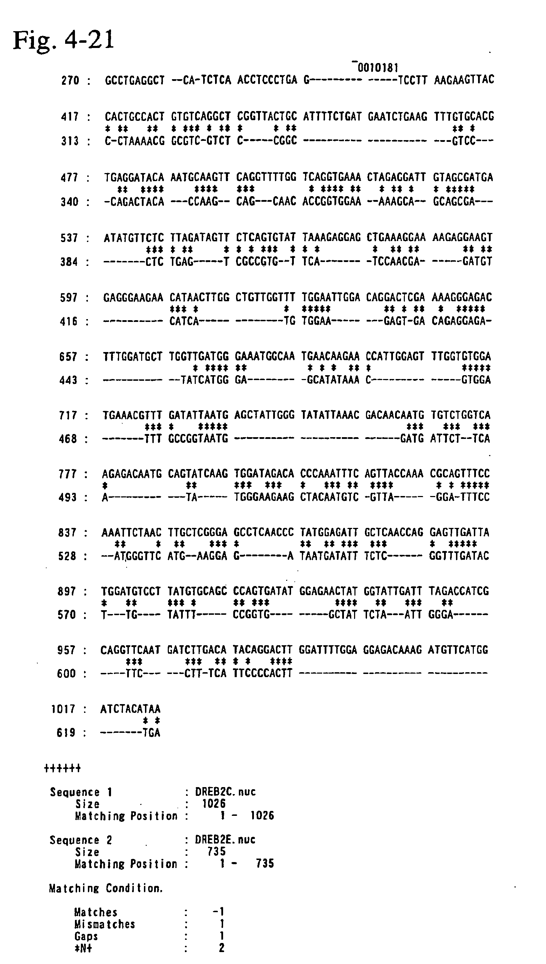

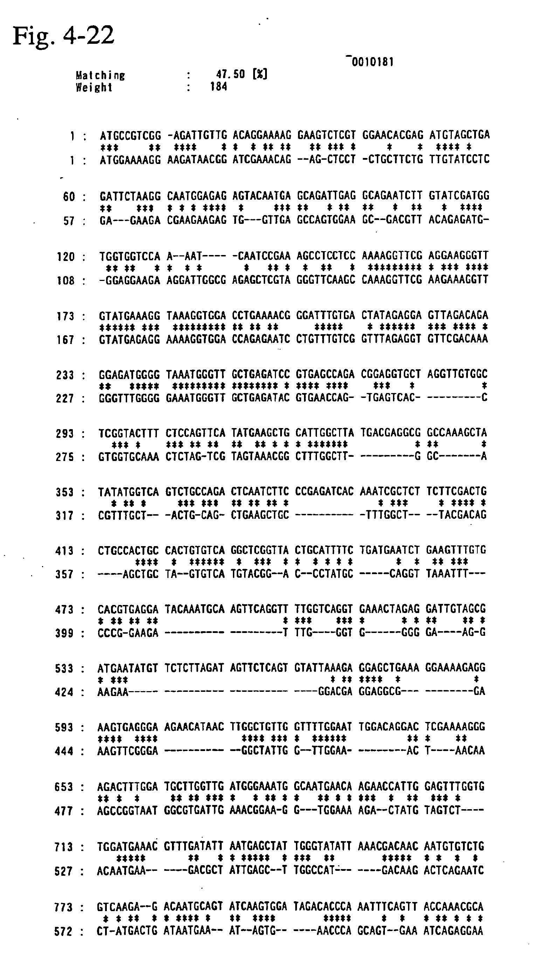

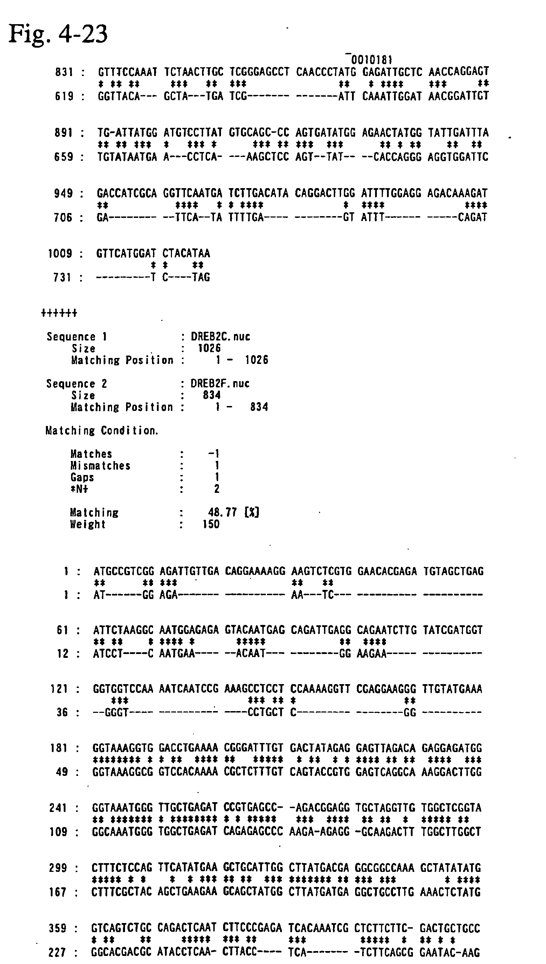

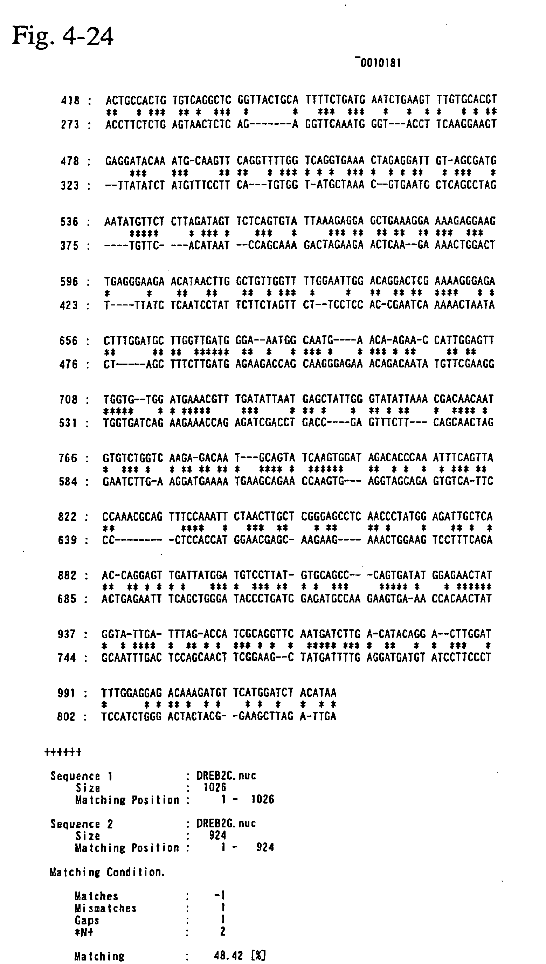

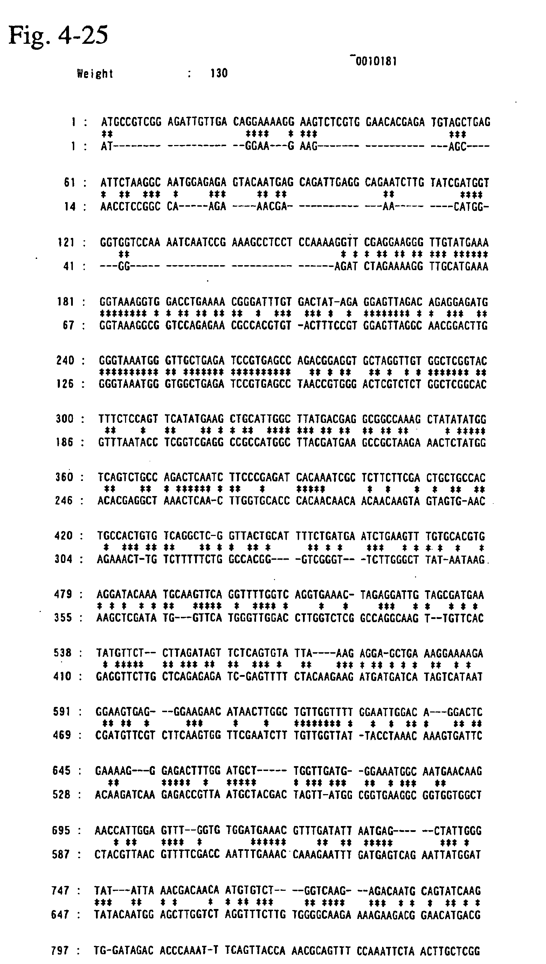

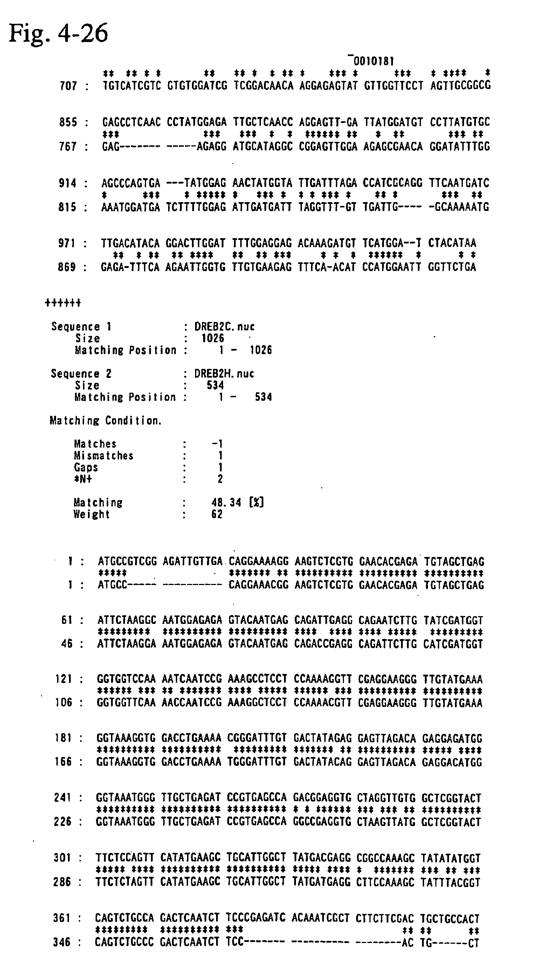

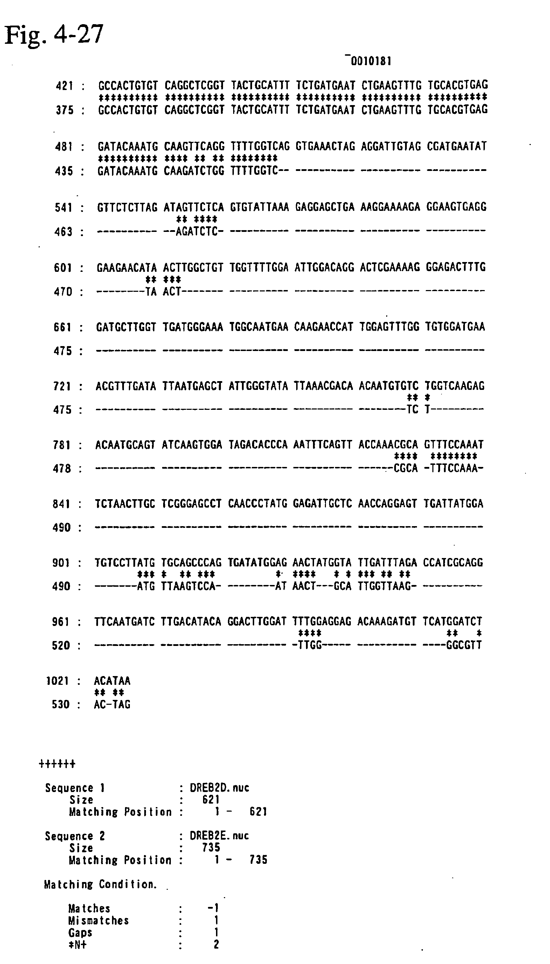

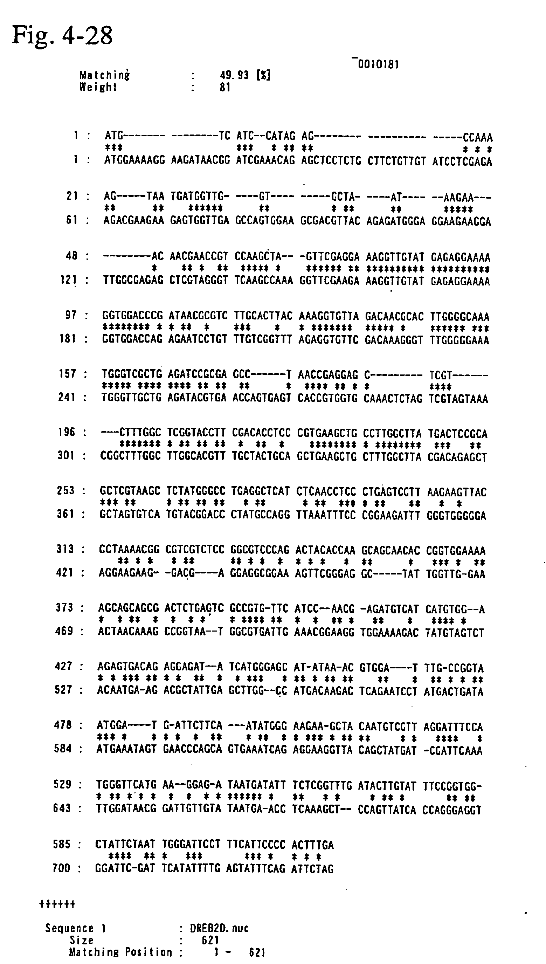

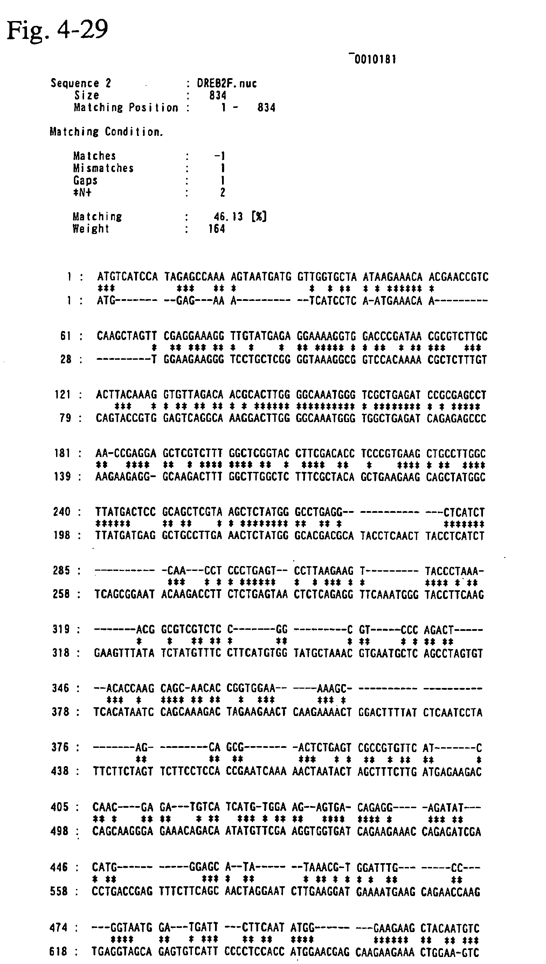

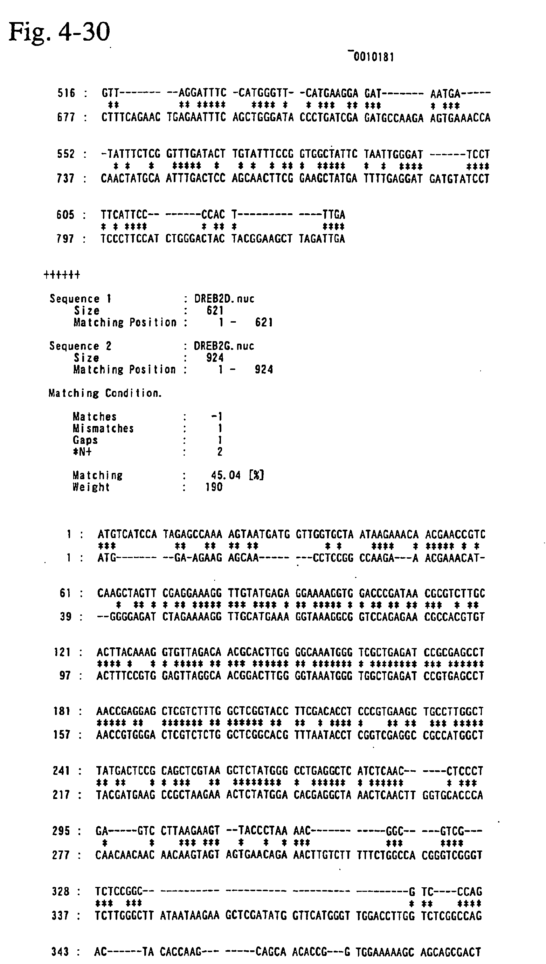

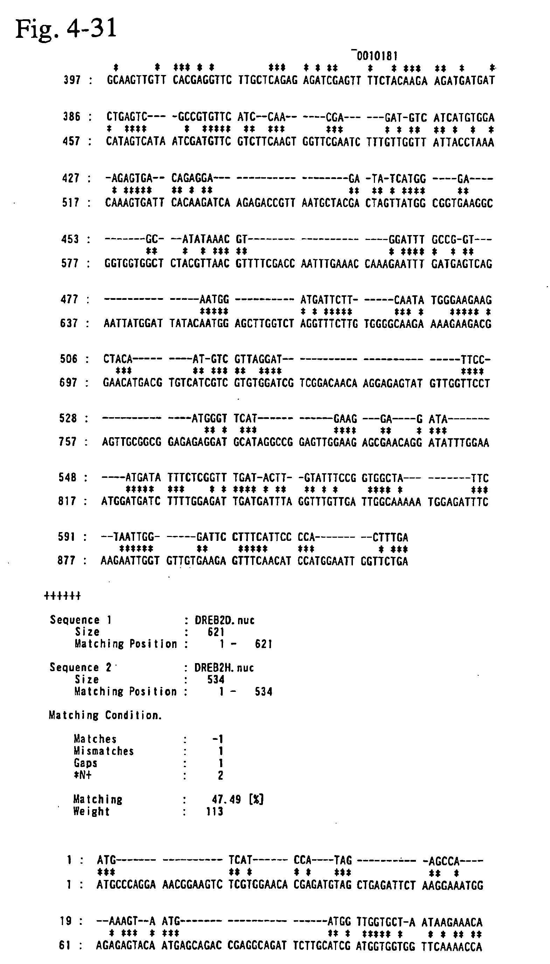

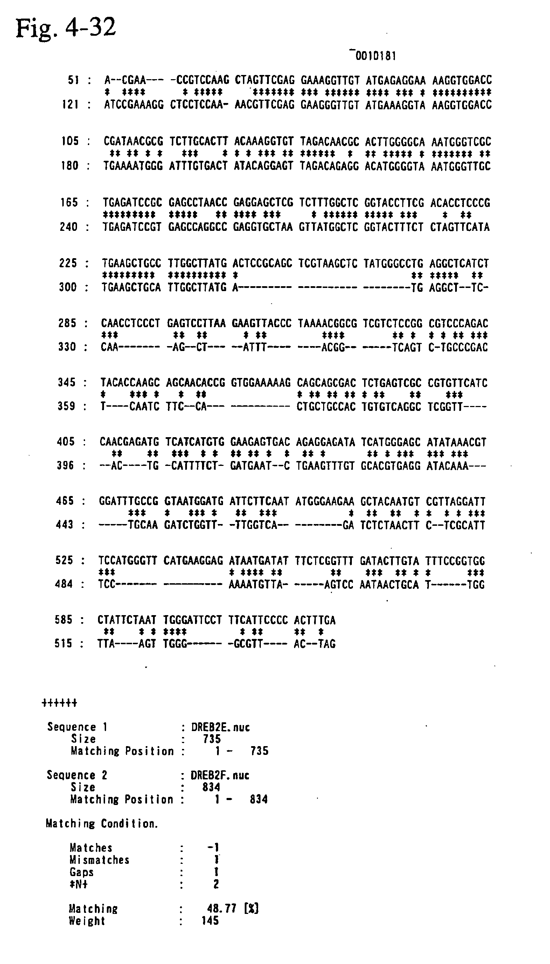

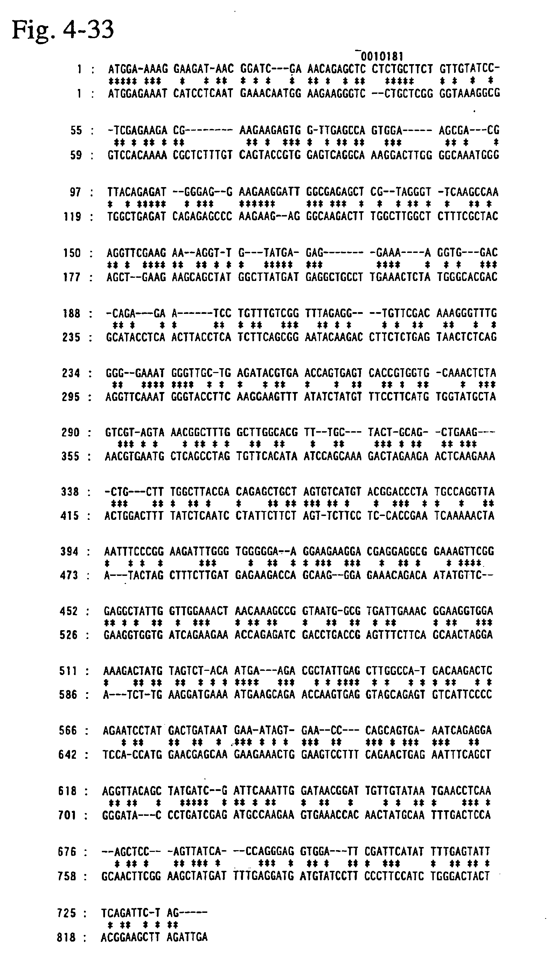

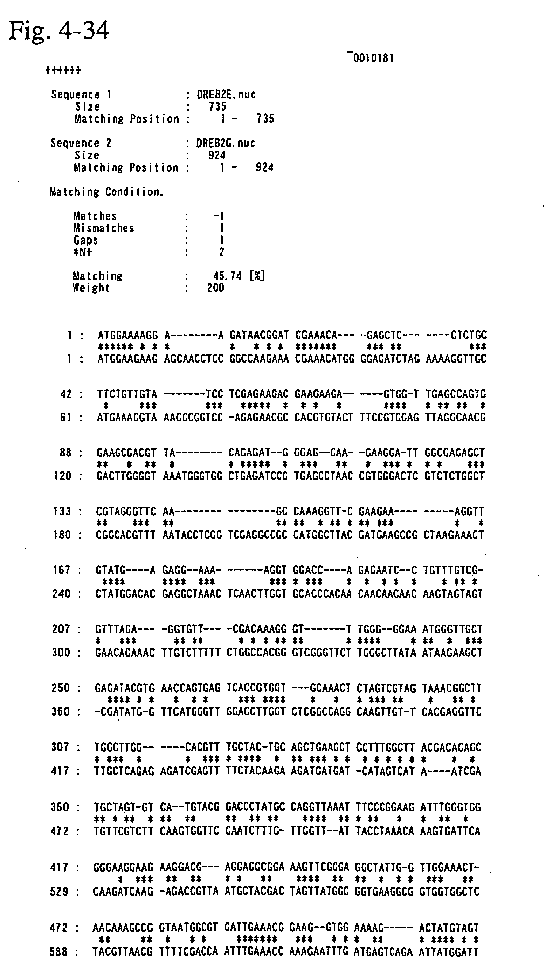

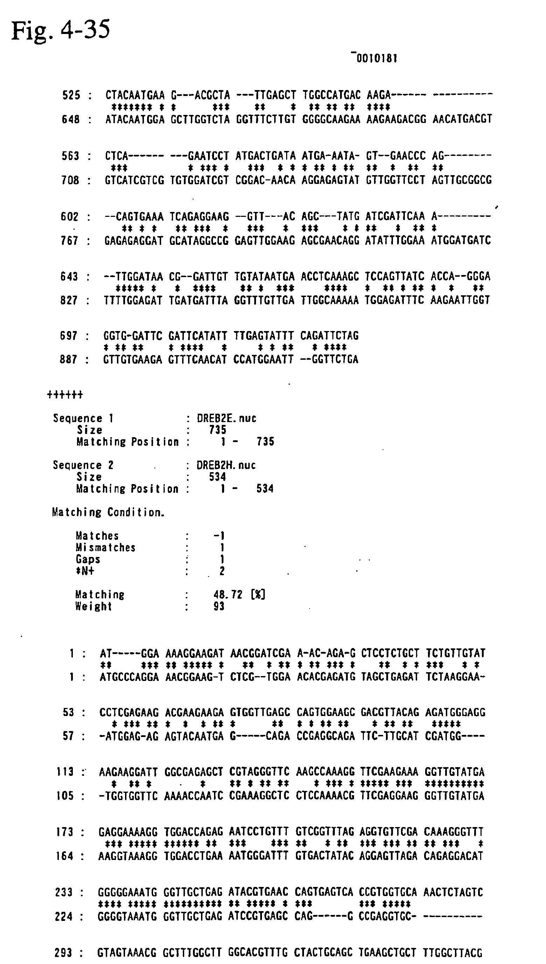

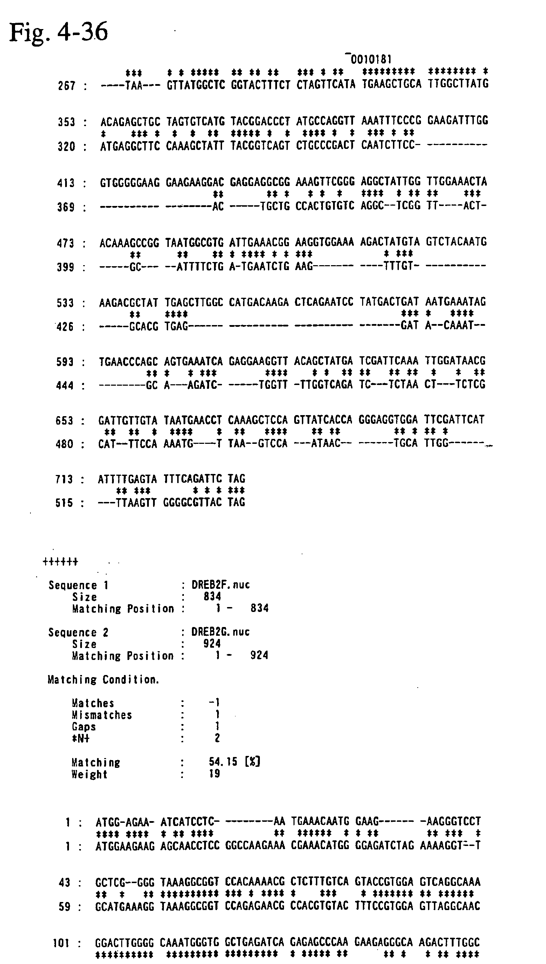

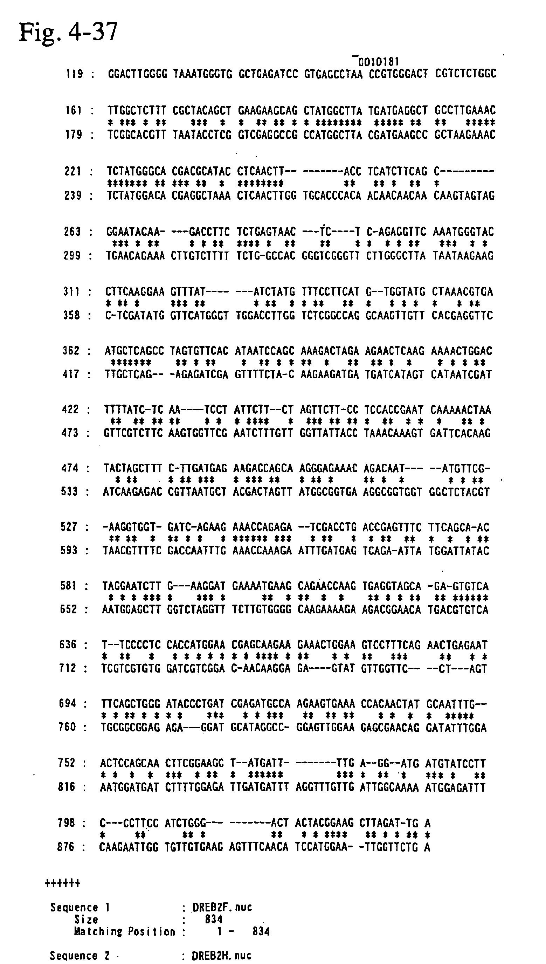

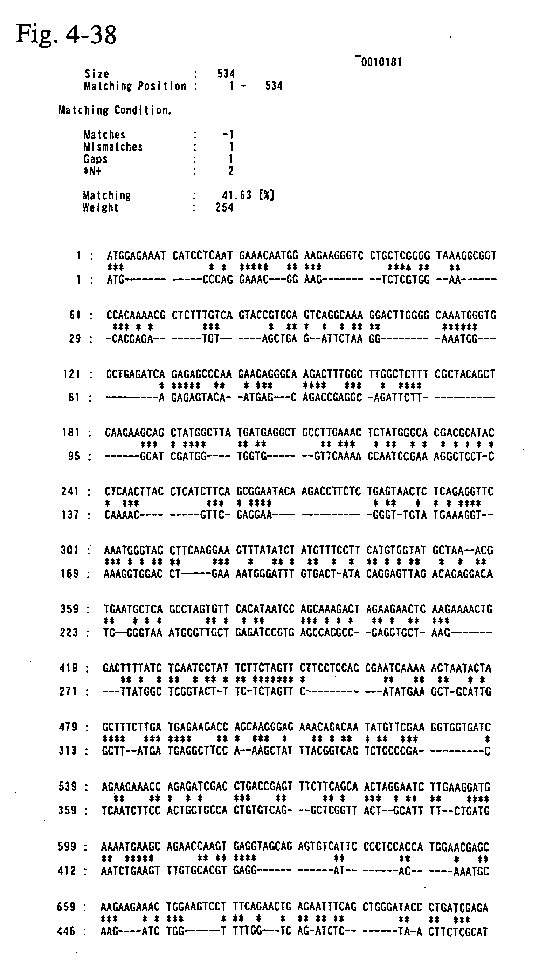

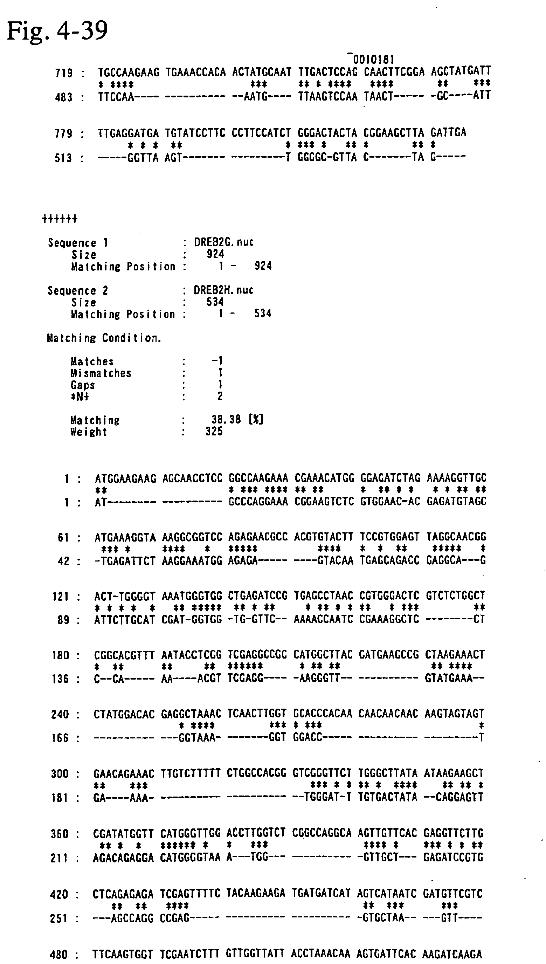

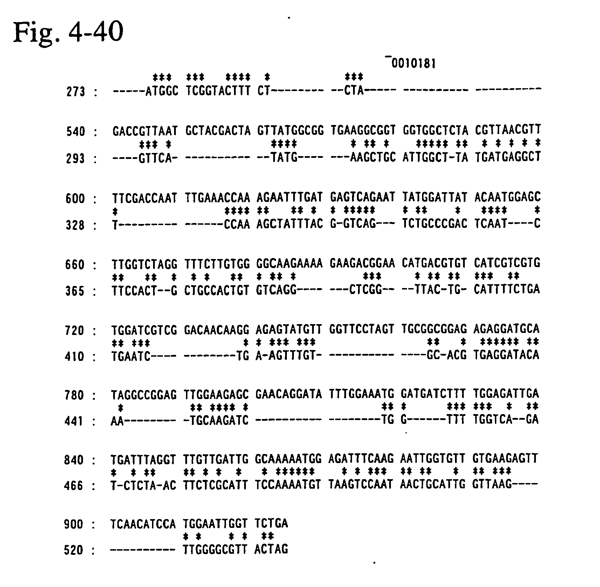

Fig. 4-1 shows 1 to 1 alignment, common sequences and homology % at the nucleotide sequence level between DREB2A as a standard and each one of DREB2B to DREB2H.

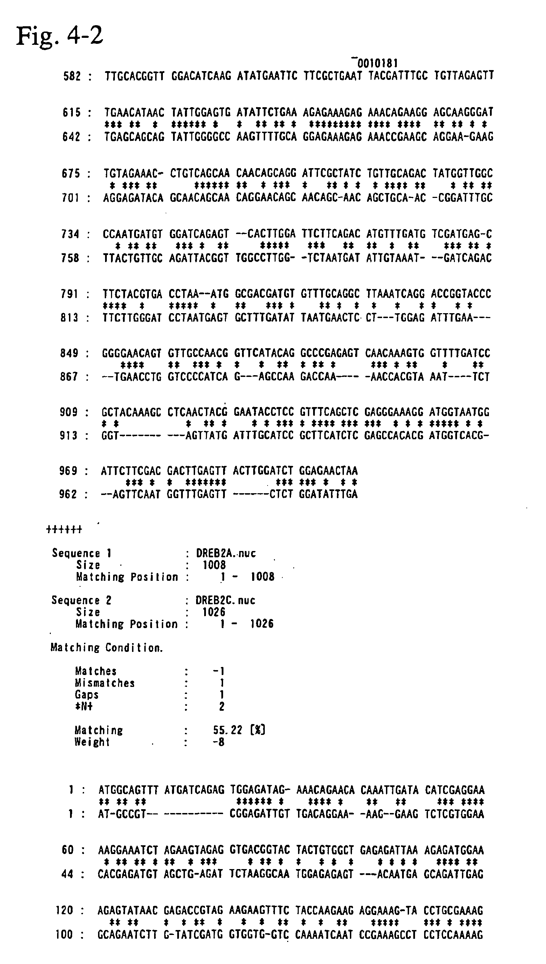

Fig. 4-2 shows 1 to 1 alignment, common sequences and homology % at the nucleotide sequence level between DREB2A as a standard and each one of DREB2B to DREB2H (continued from Fig. 4-1).

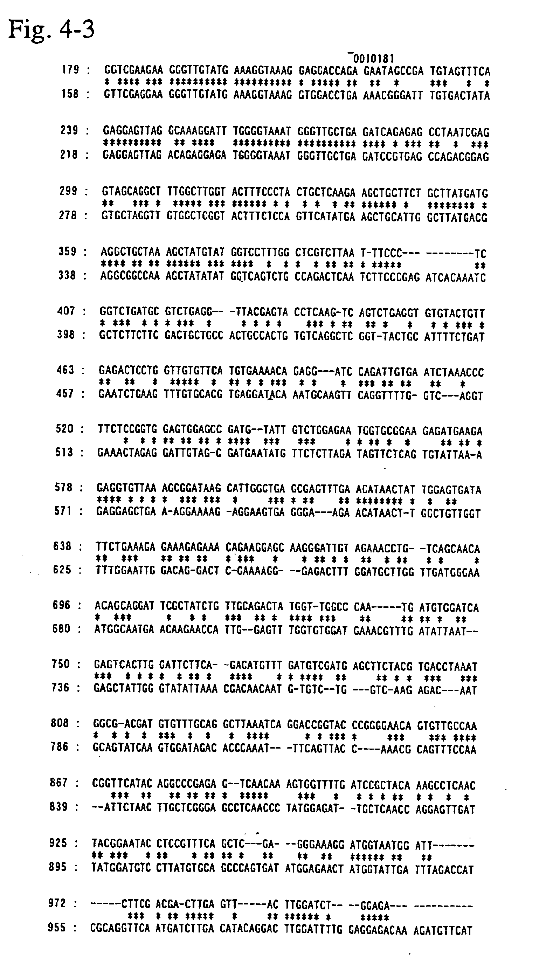

Fig. 4-3 shows 1 to 1 alignment, common sequences and homology % at the nucleotide sequence level between DREB2A as a standard and each one of DREB2B to DREB2H (continued from Fig. 4-2).

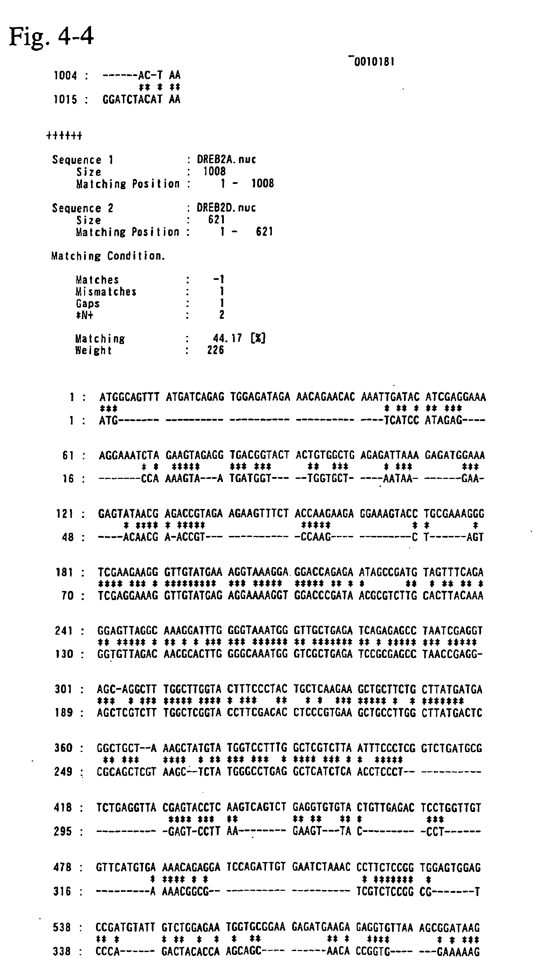

Fig. 4-4 shows 1 to 1 alignment, common sequences and homology % at the nucleotide sequence level between DREB2A as a standard and each one of DREB2B to DREB2H (continued from Fig. 4-3).
Fig. 4-5 shows 1 to 1 alignment, common sequences and homology % at the nucleotide sequence level between DREB2A as a standard and each one of DREB2B to DREB2H (continued from Fig. 4-4).
Fig. 4-6 shows 1 to 1 alignment, common sequences and homology % at the nucleotide sequence level between DREB2A as a standard and each one of DREB2B to DREB2H (continued from Fig. 4-5).
Fig. 4-7 shows 1 to 1 alignment, common sequences and homology % at the nucleotide sequence level between DREB2A as a standard and each one of DREB2B to DREB2H (continued from Fig. 4-6).
Fig. 4-8 shows 1 to 1 alignment, common sequences and homology % at the nucleotide sequence level between DREB2A as a standard and each one of DREB2B to DREB2H (continued from Fig. 4-7).
Fig. 4-9 shows 1 to 1 alignment, common sequences and homology % at the nucleotide sequence level between DREB2A as a standard and each one of DREB2B to DREB2H (continued from Fig. 4-8).
Fig. 4-10 shows 1 to 1 alignment, common sequences and homology % at the nucleotide sequence level between DREB2A as a standard and each one of DREB2B to DREB2H (continued from Fig. 4-9).
Fig. 4-11 shows 1 to 1 alignment, common sequences and homology % at the nucleotide sequence level between DREB2A as a standard and each one of DREB2B to DREB2H (continued from Fig. 4-10).
Fig. 4-12 shows 1 to 1 alignment, common sequences and homology % at the nucleotide sequence level between DREB2A as a standard and each one of DREB2B to DREB2H (continued from Fig. 4-11).
Fig. 4-13 shows 1 to 1 alignment, common sequences and homology % at the nucleotide sequence level between DREB2A as a standard and each one of DREB2B to DREB2H (continued from Fig. 4-12).
Fig. 4-14 shows 1 to 1 alignment, common sequences and homology % at the nucleotide sequence level between DREB2A as a standard and each one of DREB2B to DREB2H (continued from Fig. 4-13).
Fig. 4-15 shows 1 to 1 alignment, common sequences and homology % at the nucleotide sequence level between DREB2A as a standard and each one of DREB2B to DREB2H (continued from Fig. 4-14).
Fig. 4-16 shows 1 to 1 alignment, common sequences and homology % at the nucleotide sequence level between DREB2A as a standard and each one of DREB2B to DREB2H (continued from Fig. 4-15).
Fig. 4-17 shows 1 to 1 alignment, common sequences and homology % at the nucleotide sequence level between DREB2A as a standard and each one of DREB2B to DREB2H (continued from Fig. 4-16).
Fig. 4-18 shows 1 to 1 alignment, common sequences and homology % at the nucleotide sequence level between DREB2A as a standard and each one of DREB2B to DREB2H (continued from Fig. 4-17).
Fig. 4-19 shows 1 to 1 alignment, common sequences and homology % at the nucleotide sequence level between DREB2A as a standard and each one of DREB2B to DREB2H (continued from Fig. 4-18).
Fig. 4-20 shows 1 to 1 alignment, common sequences and homology % at the nucleotide sequence level between DREB2A as a standard and each one of DREB2B to DREB2H (continued from Fig. 4-19).
Fig. 4-21 shows 1 to 1 alignment, common sequences and homology % at the nucleotide sequence level between DREB2A as a standard and each one of DREB2B to DREB2H (continued from Fig. 4-20).
Fig. 4-22 shows 1 to 1 alignment, common sequences and homology % at the nucleotide sequence level between DREB2A as a standard and each one of DREB2B to DREB2H (continued from Fig. 4-21).
Fig. 4-23 shows 1 to 1 alignment, common sequences and homology % at the nucleotide sequence level between DREB2A as a standard and each one of DREB2B to DREB2H (continued from Fig. 4-22).
Fig. 4-24 shows 1 to 1 alignment, common sequences and homology % at the nucleotide sequence level between DREB2A as a standard and each one of DREB2B to DREB2H (continued from Fig. 4-23).
Fig. 4-25 shows 1 to 1 alignment, common sequences and homology % at the nucleotide sequence level between DREB2A as a standard and each one of DREB2B to DREB2H (continued from Fig. 4-24).
Fig. 4-26 shows 1 to 1 alignment, common sequences and homology % at the nucleotide sequence level between DREB2A as a standard and each one of DREB2B to DREB2H (continued from Fig. 4-25).
Fig. 4-27 shows 1 to 1 alignment, common sequences and homology % at the nucleotide sequence level between DREB2A as a standard and each one of DREB2B to DREB2H (continued from Fig. 4-26).
Fig. 4-28 shows 1 to 1 alignment, common sequences and homology % at the nucleotide sequence level between DREB2A as a standard and each one of DREB2B to DREB2H (continued from Fig. 4-27).
Fig. 4-29 shows 1 to 1 alignment, common sequences and homology % at the nucleotide sequence level between DREB2A as a standard and each one of DREB2B to DREB2H (continued from Fig. 4-28).
Fig. 4-30 shows 1 to 1 alignment, common sequences and homology % at the nucleotide sequence level between DREB2A as a standard and each one of DREB2B to DREB2H (continued from Fig. 4-29).
Fig. 4-31 shows 1 to 1 alignment, common sequences and homology % at the nucleotide sequence level between DREB2A as a standard and each one of DREB2B to DREB2H (continued from Fig. 4-30).
Fig. 4-32 shows 1 to 1 alignment, common sequences and homology % at the nucleotide sequence level between DREB2A as a standard and each one of DREB2B to DREB2H (continued from Fig. 4-31).
Fig. 4-33 shows 1 to 1 alignment, common sequences and homology % at the nucleotide sequence level between DREB2A as a standard and each one of DREB2B to DREB2H (continued from Fig. 4-32).
Fig. 4-34 shows 1 to 1 alignment, common sequences and homology % at the nucleotide sequence level between DREB2A as a standard and each one of DREB2B to DREB2H (continued from Fig. 4-33).
Fig. 4-35 shows 1 to 1 alignment, common sequences and homology % at the nucleotide sequence level between DREB2A as a standard and each one of DREB2B to DREB2H (continued from Fig. 4-34).
Fig. 4-36 shows 1 to 1 alignment, common sequences and homology % at the nucleotide sequence level between DREB2A as a standard and each one of DREB2B to DREB2H (continued from Fig. 4-35).
Fig. 4-37 shows 1 to 1 alignment, common sequences and homology % at the nucleotide sequence level between DREB2A as a standard and each one of DREB2B to DREB2H (continued from Fig. 4-36).
Fig. 4-38 shows 1 to 1 alignment, common sequences and homology % at the nucleotide sequence level between DREB2A as a standard and each one of DREB2B to DREB2H (continued from Fig. 4-37).
Fig. 4-39 shows 1 to 1 alignment, common sequences and homology % at the nucleotide sequence level between DREB2A as a standard and each one of DREB2B to DREB2H (continued from Fig. 4-38).
Fig. 4-40 shows 1 to 1 alignment, common sequences and homology % at the nucleotide sequence level between DREB2A as a standard and each one of DREB2B to DREB2H (continued from Fig. 4-39).

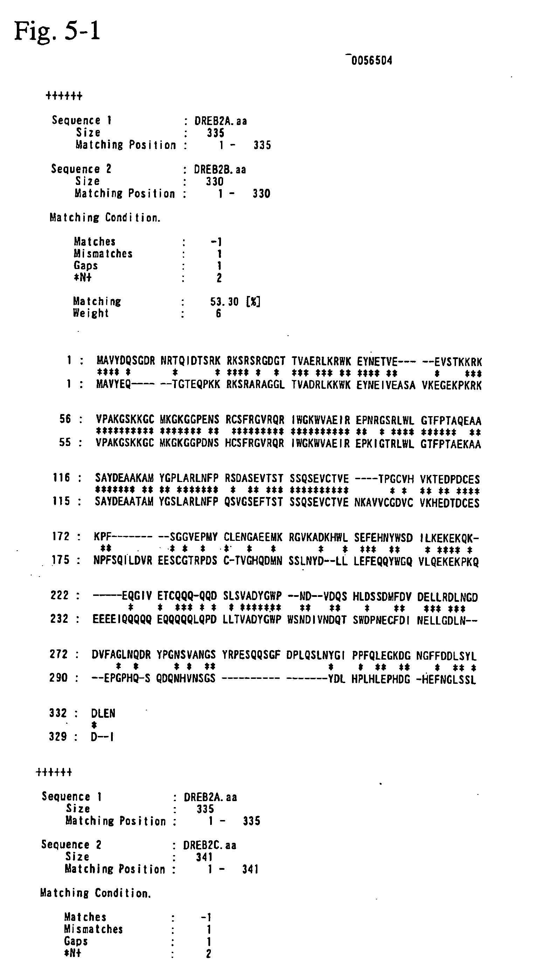

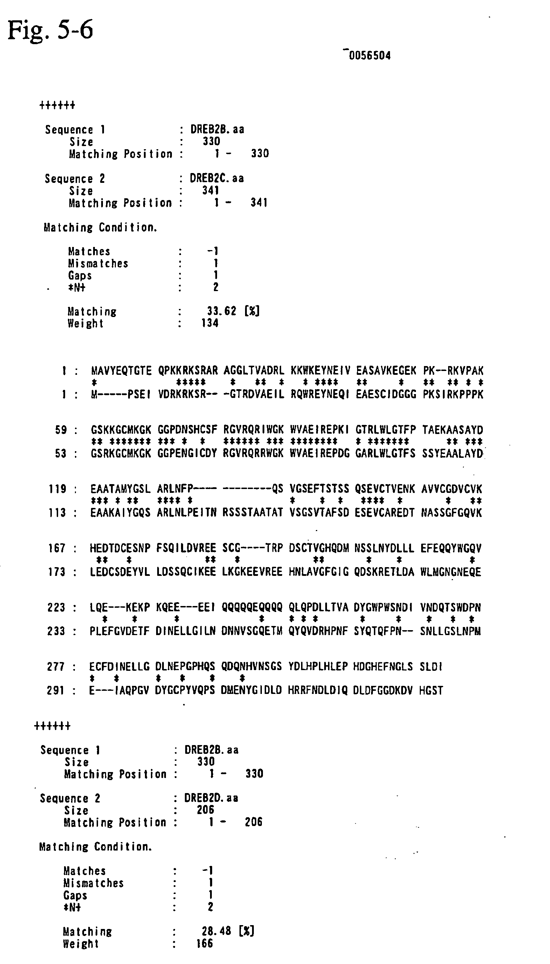

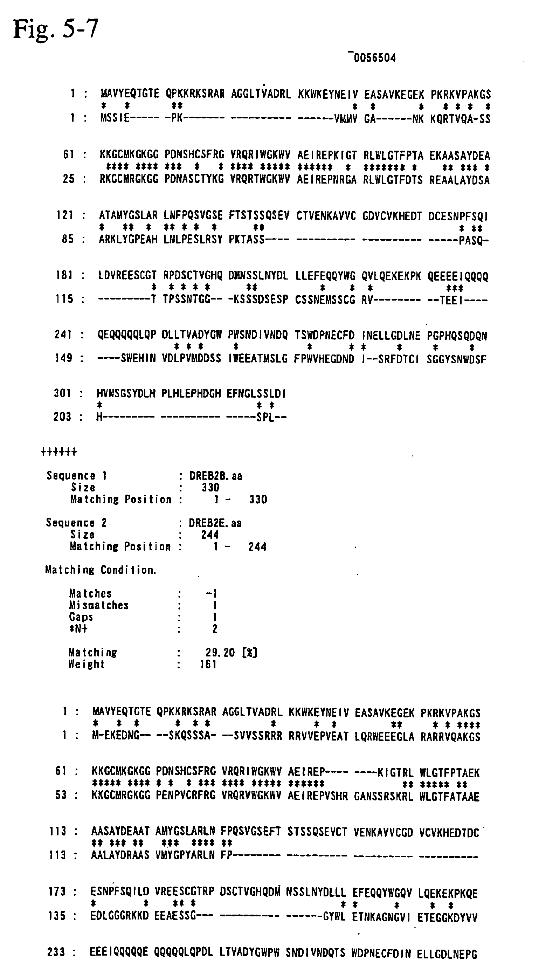

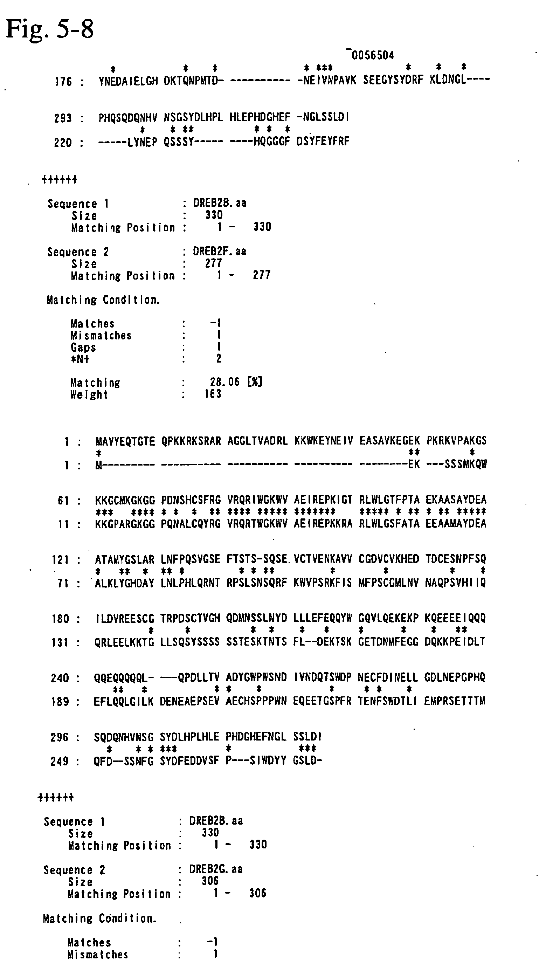

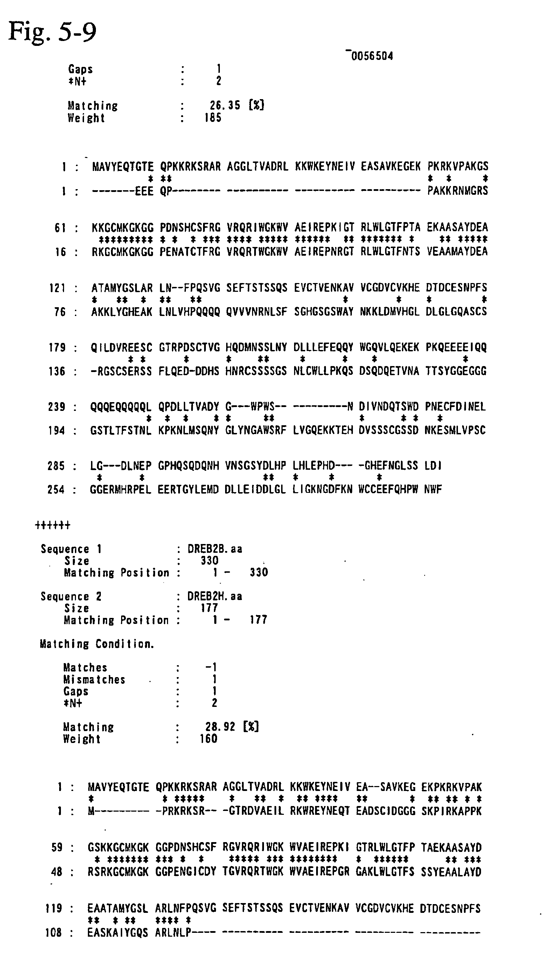

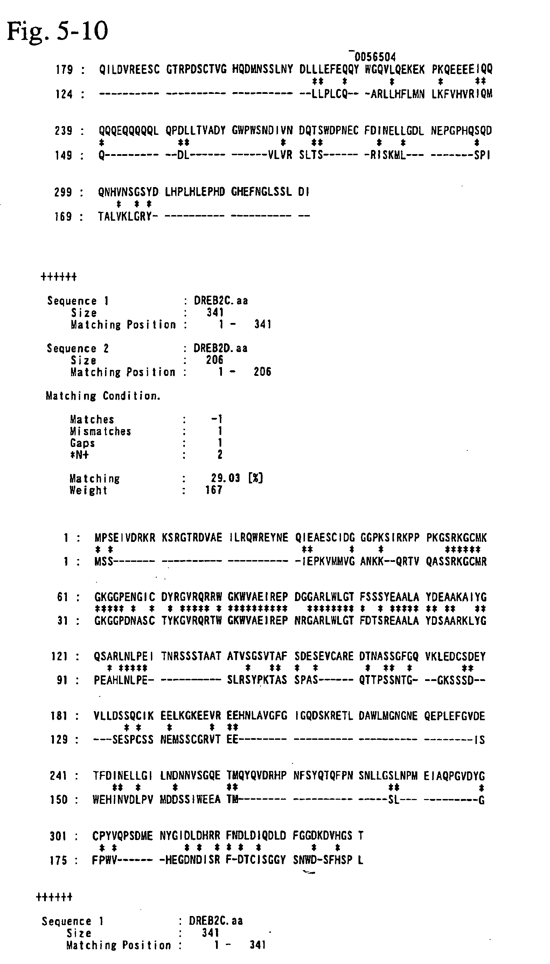

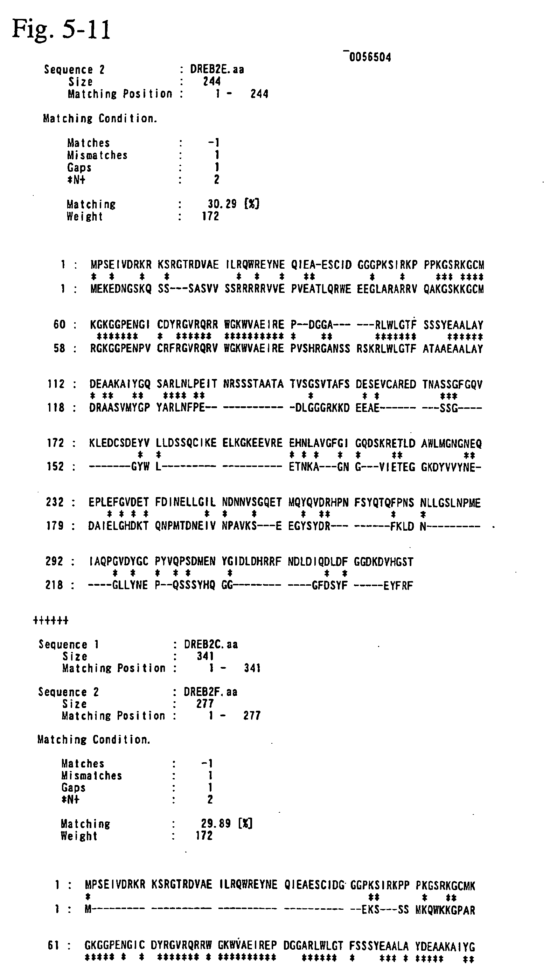

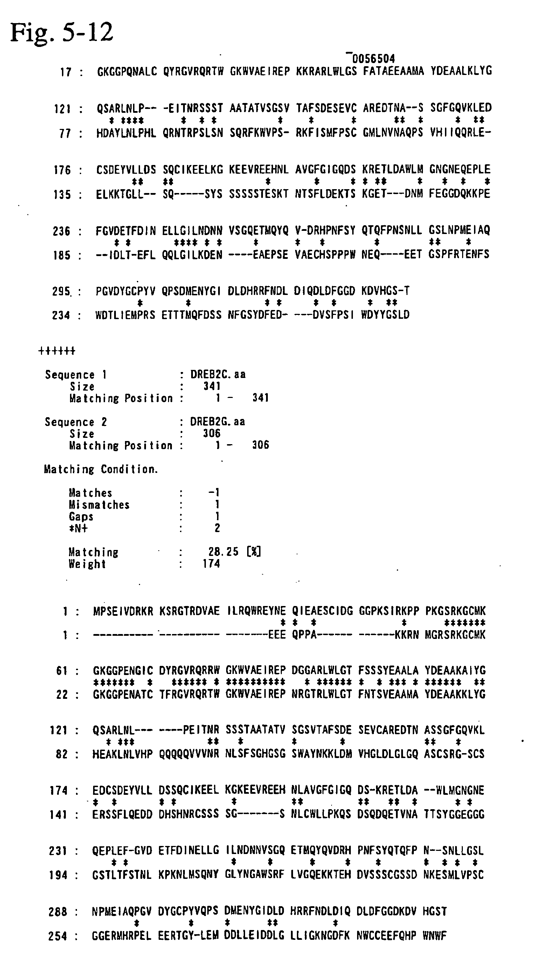

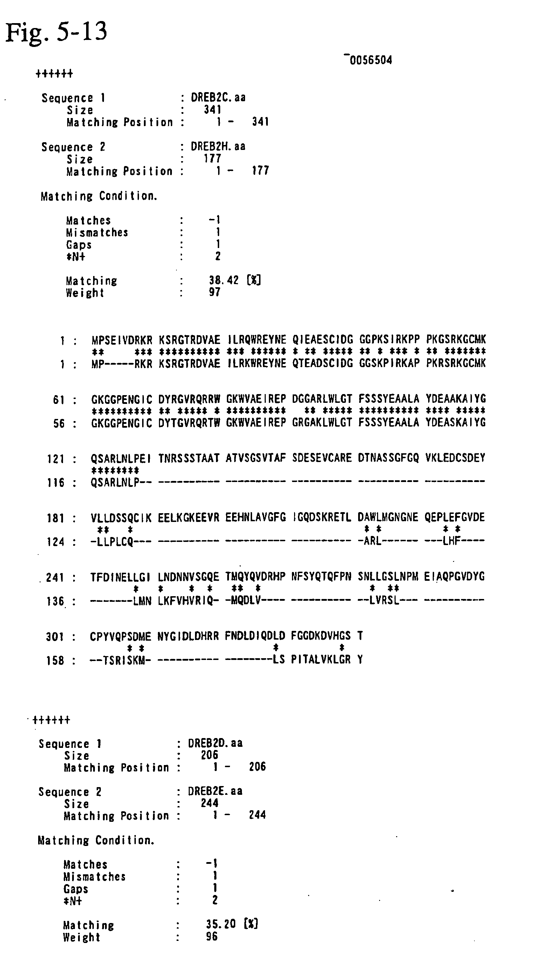

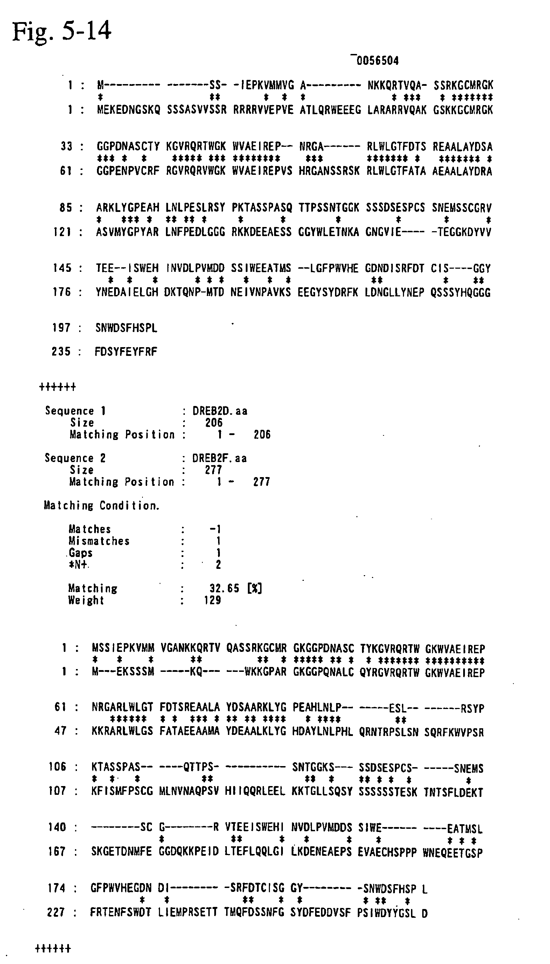

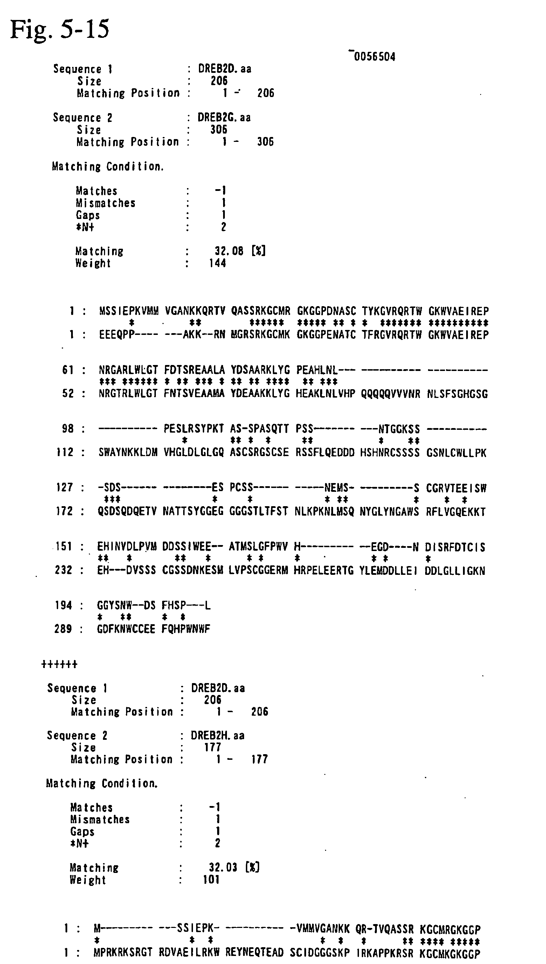

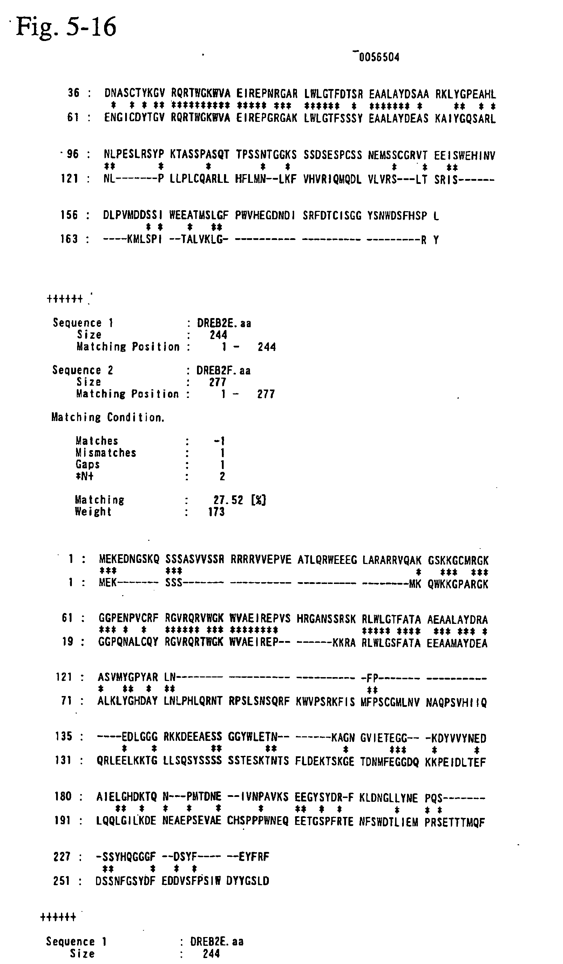

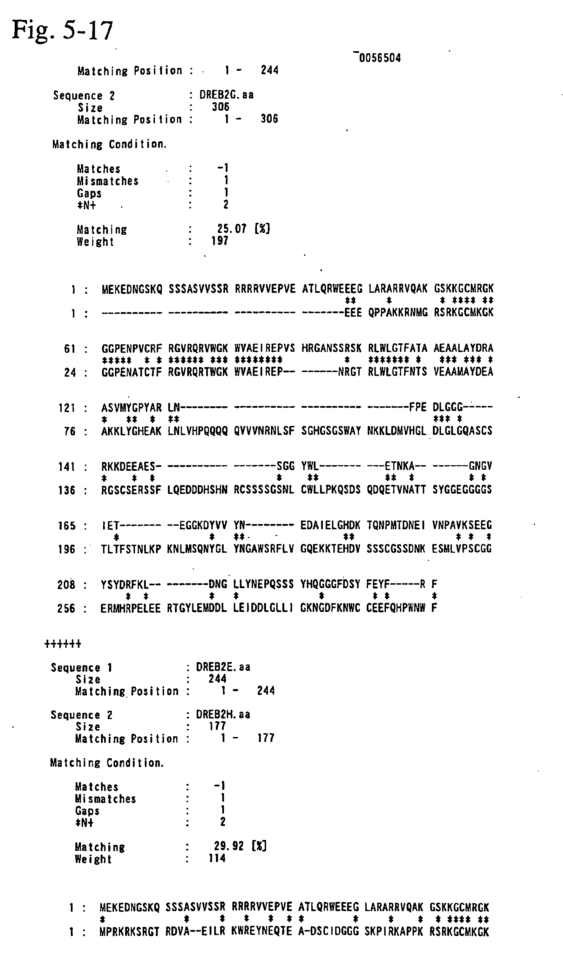

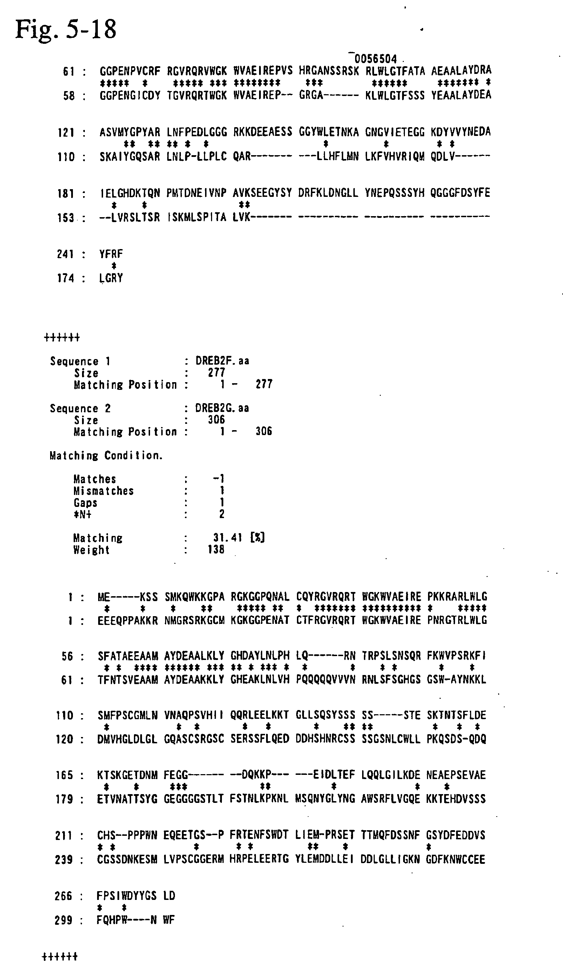

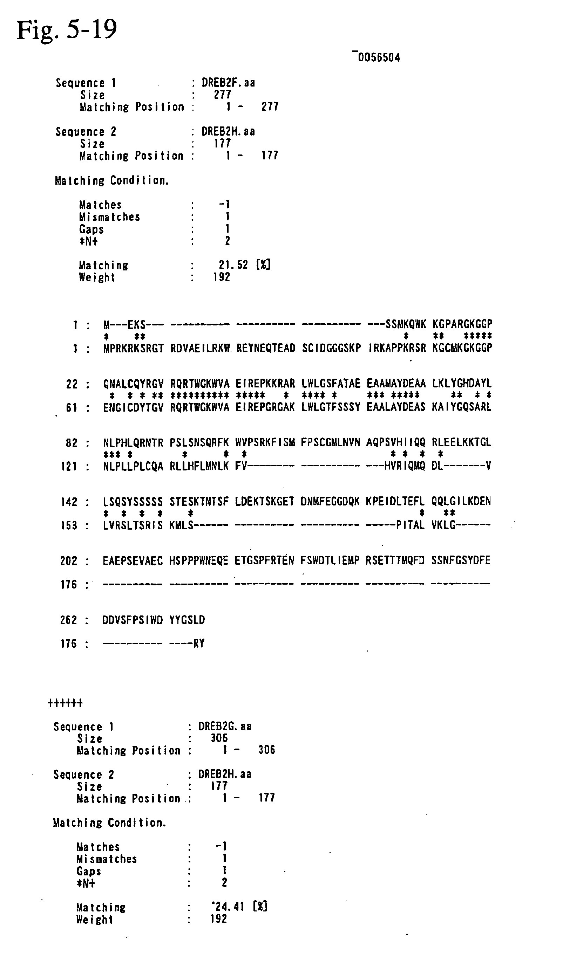

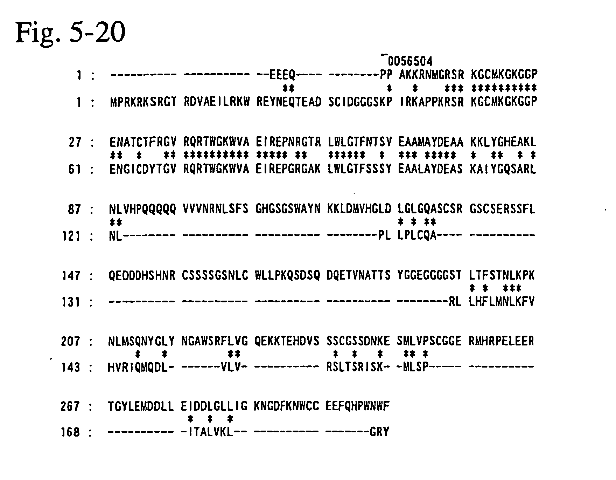

Fig. 5-1 shows 1 to 1 alignment, common sequences and homology % at the amino acid sequence level between DREB2A as a standard and each one of DREB2B to DREB2H.

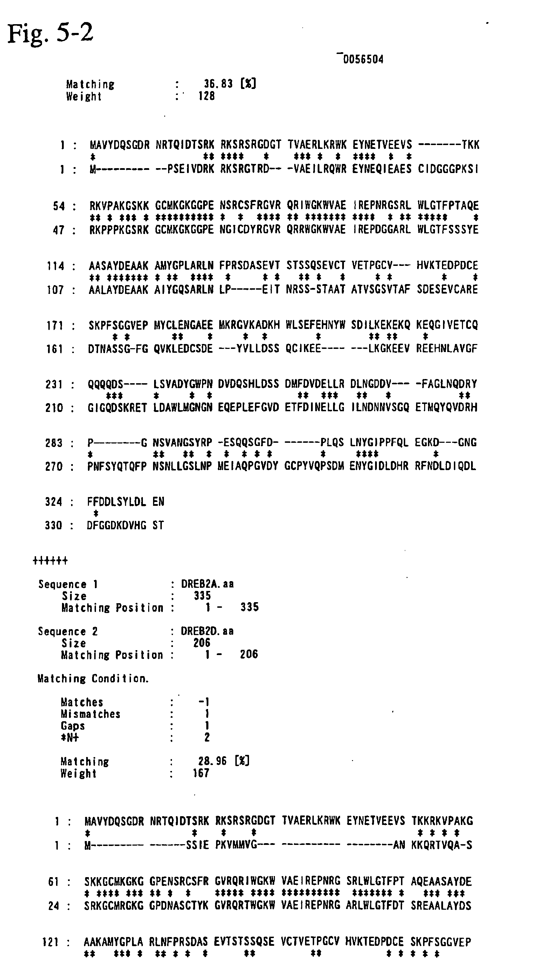

Fig. 5-2 shows 1 to 1 alignment, common sequences and homology % at the amino acid sequence level between DREB2A as a standard and each one of DREB2B to DREB2H (continued from Fig. 5-1).

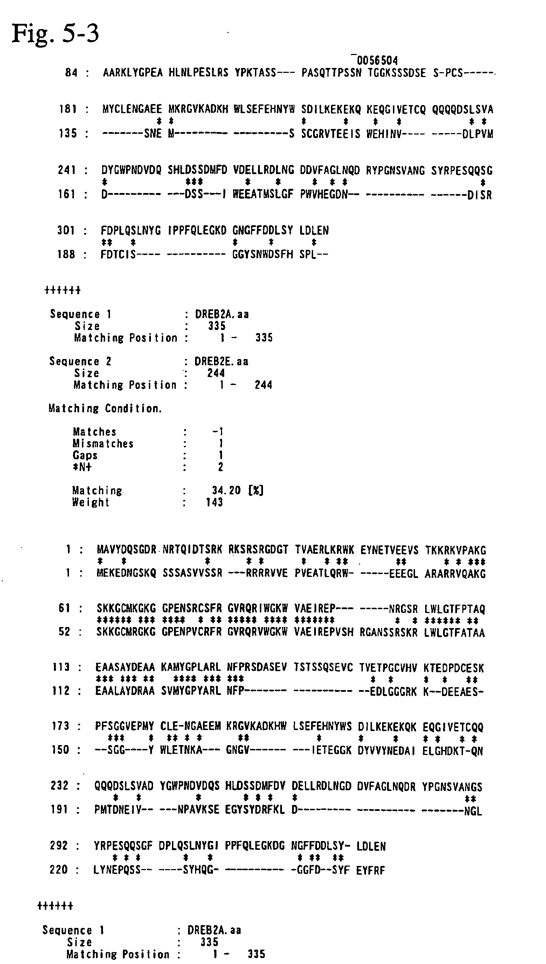

Fig. 5-3 shows 1 to 1 alignment, common sequences and homology % at the amino acid sequence level between DREB2A as a standard and each one of DREB2B to DREB2H (continued from Fig. 5-2).

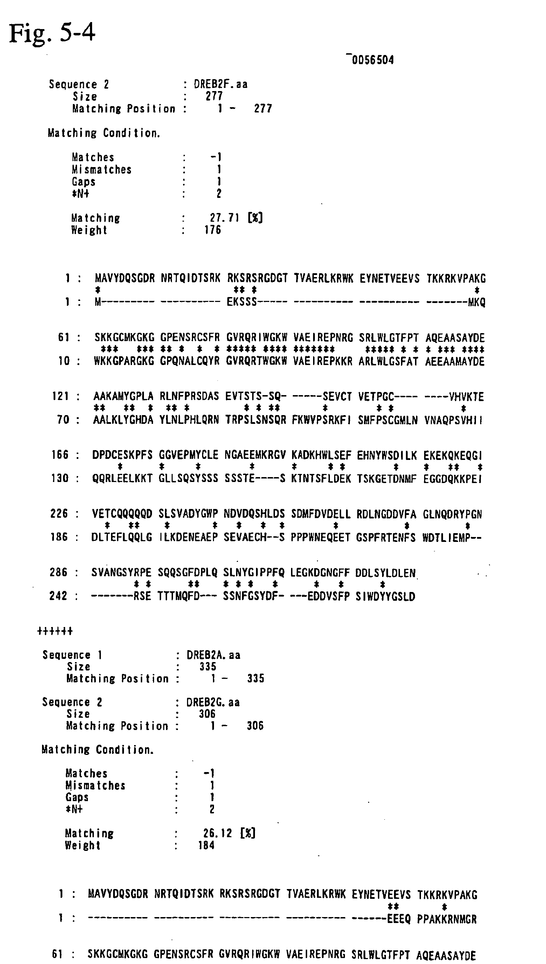

Fig. 5-4 shows 1 to 1 alignment, common sequences and homology % at the amino acid sequence level between DREB2A as a standard and each one of DREB2B to DREB2H (continued from Fig. 5-3).

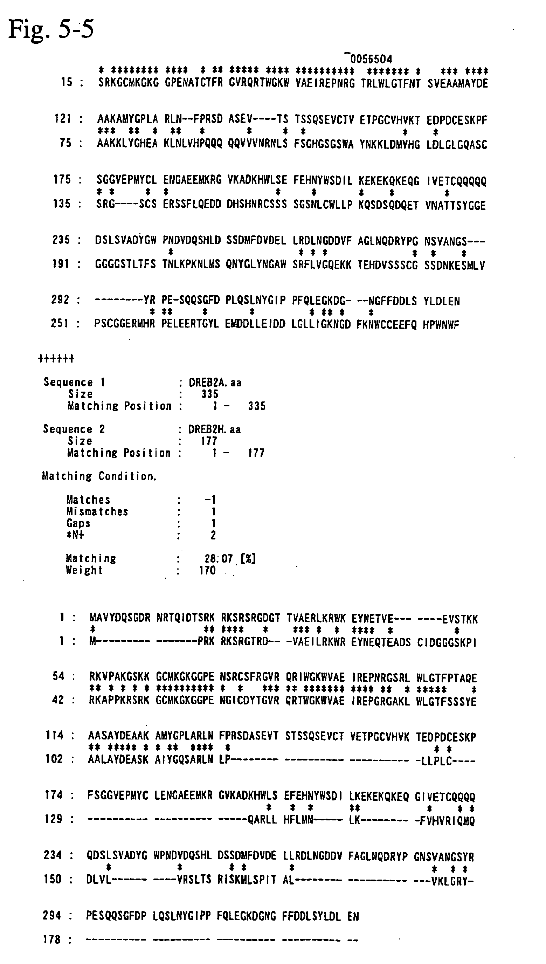

Fig. 5-5 shows 1 to 1 alignment, common sequences and homology % at the amino acid sequence level between DREB2A as a standard and each one of DREB2B to DREB2H (continued from Fig. 5-4).
Fig. 5-6 shows 1 to 1 alignment, common sequences and homology % at the amino acid sequence level between DREB2A as a standard and each one of DREB2B to DREB2H (continued from Fig. 5-5).
Fig. 5-7 shows 1 to 1 alignment, common sequences and homology % at the amino acid sequence level between DREB2A as a standard and each one of DREB2B to DREB2H (continued from Fig. 5-6).
Fig. 5-8 shows 1 to 1 alignment, common sequences and homology % at the amino acid sequence level between DREB2A as a standard and each one of DREB2B to DREB2H (continued from Fig. 5-7).
Fig. 5-9 shows 1 to 1 alignment, common sequences and homology % at the amino acid sequence level between DREB2A as a standard and each one of DREB2B to DREB2H (continued from Fig. 5-8).
Fig. 5-10 shows 1 to 1 alignment, common sequences and homology % at the amino acid sequence level between DREB2A as a standard and each one of DREB2B to DREB2H (continued from Fig. 5-9).
Fig. 5-11 shows 1 to 1 alignment, common sequences and homology % at the amino acid sequence level between DREB2A as a standard and each one of DREB2B to DREB2H (continued from Fig. 5-10).
Fig. 5-12 shows 1 to 1 alignment, common sequences and homology % at the amino acid sequence level between DREB2A as a standard and each one of DREB2B to DREB2H (continued from Fig. 5-11).
Fig. 5-13 shows 1 to 1 alignment, common sequences and homology % at the amino acid sequence level between DREB2A as a standard and each one of DREB2B to DREB2H (continued from Fig. 5-12).
Fig. 5-14 shows 1 to 1 alignment, common sequences and homology % at the amino acid sequence level between DREB2A as a standard and each one of DREB2B to DREB2H (continued from Fig. 5-13).
Fig. 5-15 shows 1 to 1 alignment, common sequences and homology % at the amino acid sequence level between DREB2A as a standard and each one of DREB2B to DREB2H (continued from Fig. 5-14).
Fig. 5-16 shows 1 to 1 alignment, common sequences and homology % at the amino acid sequence level between DREB2A as a standard and each one of DREB2B to DREB2H (continued from Fig. 5-15).
Fig. 5-17 shows 1 to 1 alignment, common sequences and homology % at the amino acid sequence level between DREB2A as a standard and each one of DREB2B to DREB2H (continued from Fig. 5-16).
Fig. 5-18 shows 1 to 1 alignment, common sequences and homology % at the amino acid sequence level between DREB2A as a standard and each one of DREB2B to DREB2H (continued from Fig. 5-17).
Fig. 5-19 shows 1 to 1 alignment, common sequences and homology % at the amino acid sequence level between DREB2A as a standard and each one of DREB2B to DREB2H (continued from Fig. 5-18).
Fig. 5-20 shows 1 to 1 alignment, common sequences and homology % at the amino acid sequence level between DREB2A as a standard and each one of DREB2B to DREB2H (continued from Fig. 5-19).

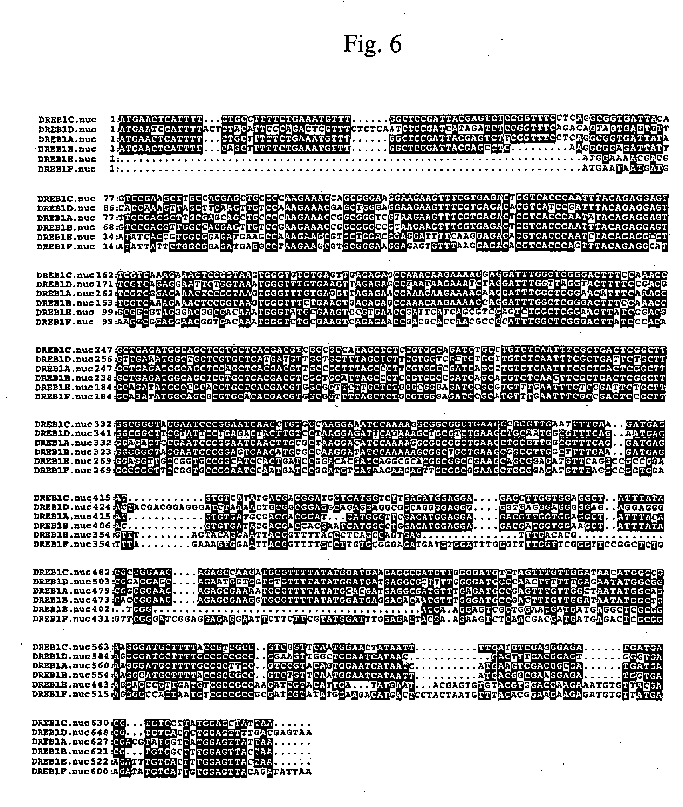

Fig. 6 shows alignment at the nucleotide sequence level between DREB1A as a standard and each one of DREB1B to DREB1F.

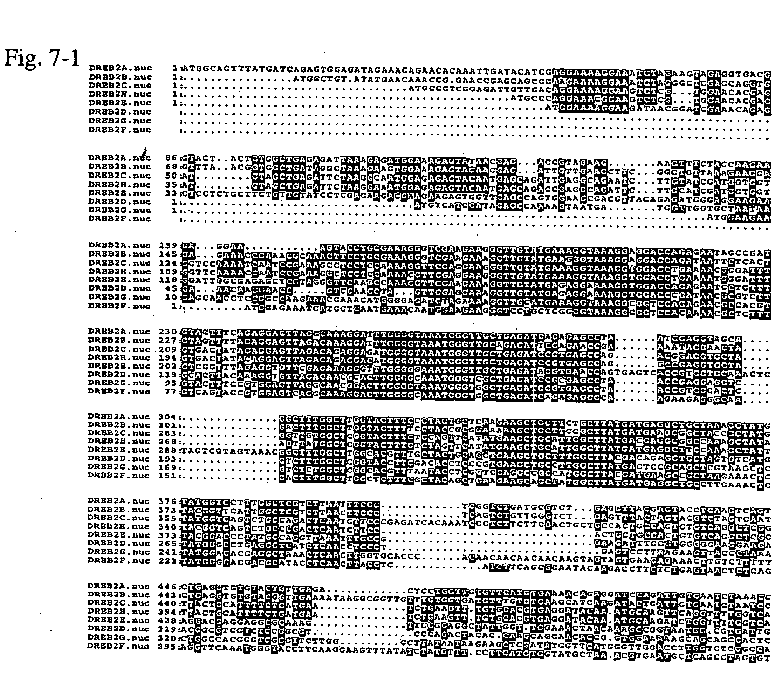

Fig. 7-1 shows alignment at the nucleotide sequence level between DREB2A as a standard and each one of DREB2B to DREB2H (to position 518 of DREB2A).

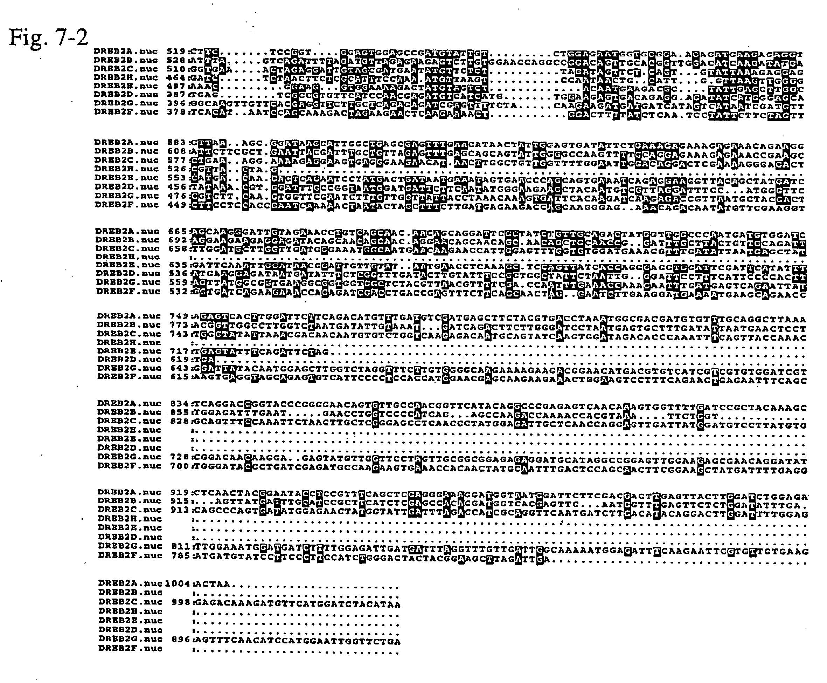

Fig. 7-2 shows alignment at the nucleotide sequence level between DREB2A as a standard and each one of DREB2B to DREB2H (from position 519 of DREB2A).

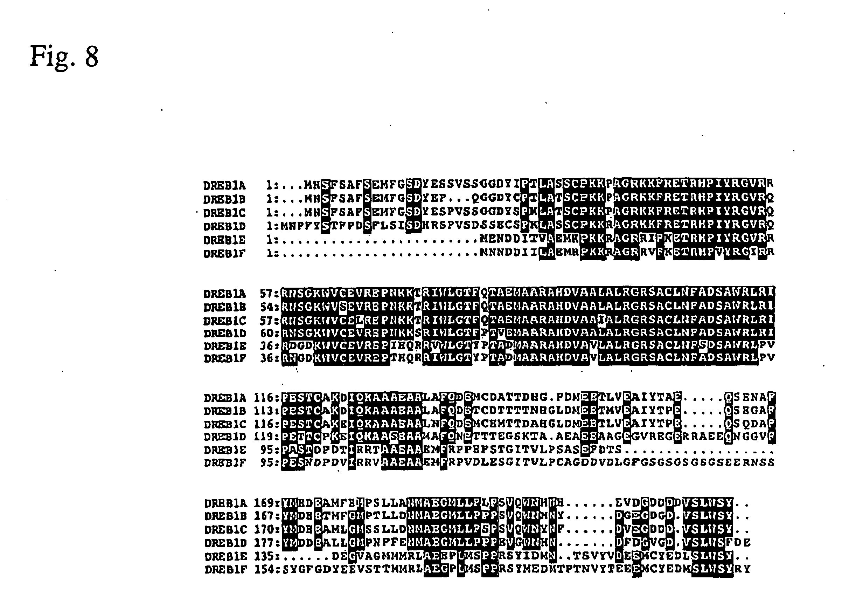

Fig. 8 shows alignment at the amino acid sequence level between DREB1A as a standard and each one of DREB 1 B to DREB1F.

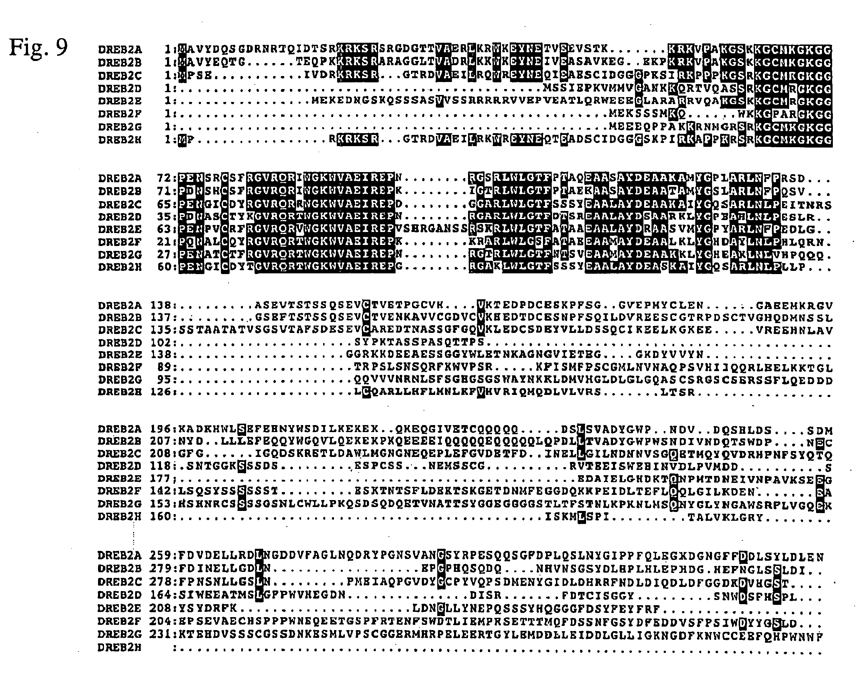

Fig. 9 shows alignment at the amino acid sequence level between DREB2A as a standard and each one of DREB2B to DREB2H.

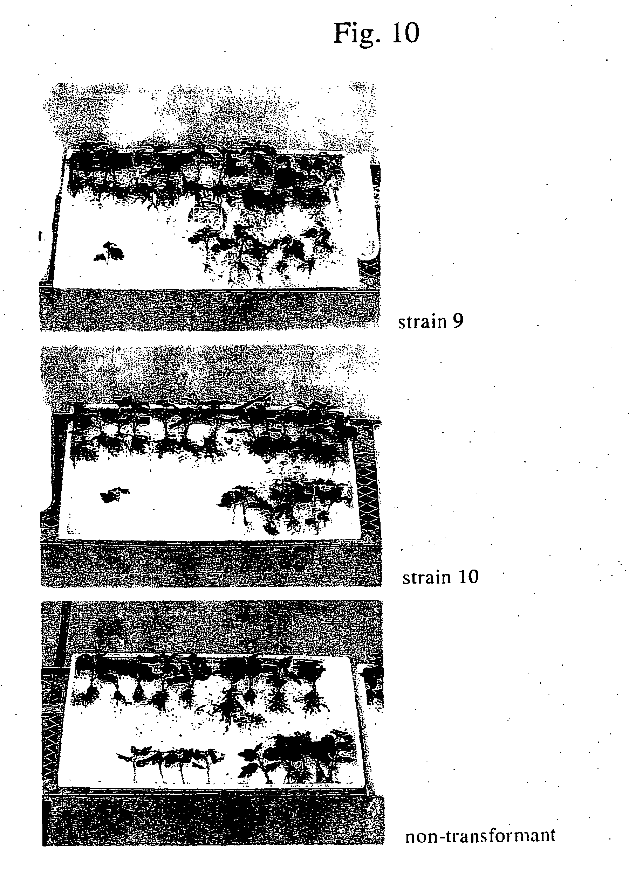

Fig. 10 shows photographs showing the rooting ability of non-transformants, and lines 9 and 10 in the rooting ability test upon production with scions.

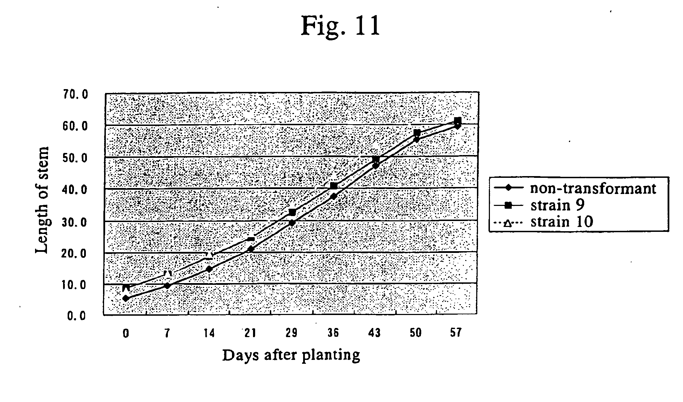

Fig. 11 is a graph showing the stem lengths of non-transformants, and lines 9 and 10 after planting.

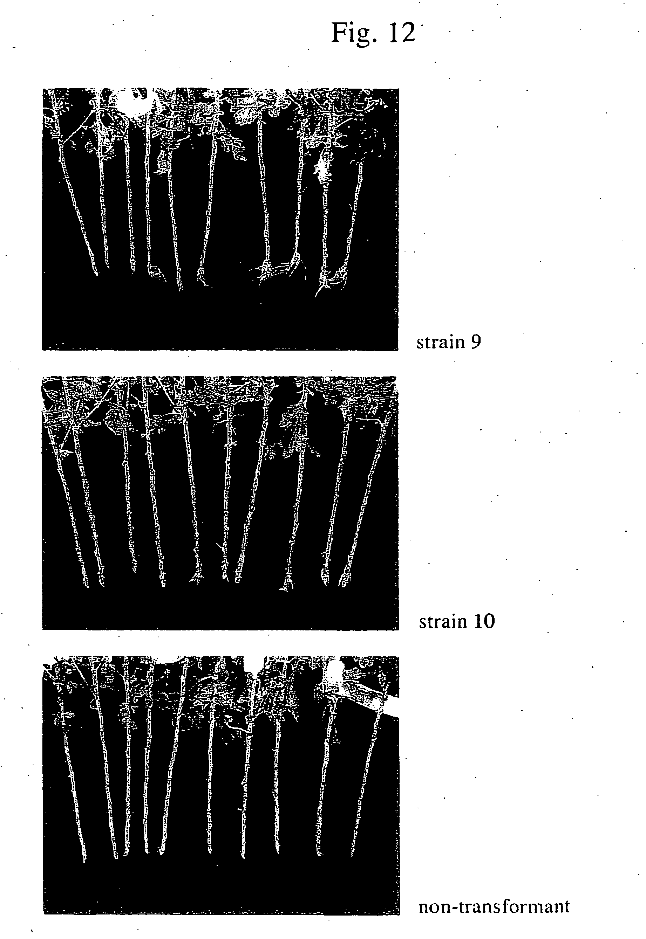

Fig. 12 shows photographs showing the vicinity of the cut ends of non-transformants, and lines 9 and 10 on day 22 after the start of a vase life test.

### DETAILED DESCRIPTION OF THE INVENTION

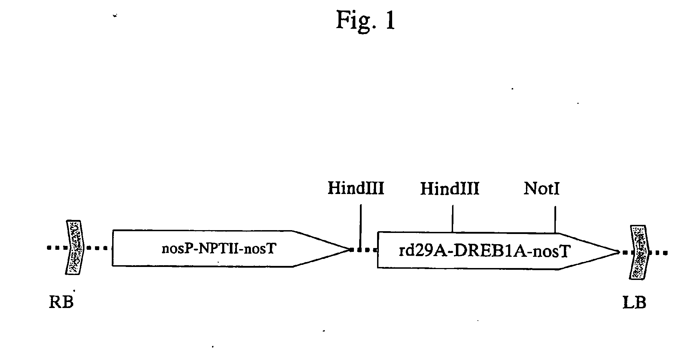

The method of producing a transformed plant of the present invention involves introducing a gene (also referred to as a stress-resistance gene in this specification) wherein a DNA (also referred to as DREB gene) encoding a transcription factor that has functions to bind to a dehydration responsive element (DRE) contained in a stress-responsive promoter and activate the transcription of a gene located downstream of the DRE is ligated downstream of the stress-responsive promoter. The transformed plant has enhanced efficiency of propagation by cutting as a result of improving the rooting efficiency, and has improved vase life (prolonged life of cut flowers). As an example, a gene having a structure wherein the rd29A promoter is used is shown (Fig. 1).

### (1) DREB gene

The DNA used in the method of the present invention encoding a transcription factor having functions to bind to a dehydration responsive element (DRE) and activate the transcription of a gene located downstream of the DRE includes DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene and DREB2H gene, and they can be used as appropriate. The DREB1A gene can be obtained by amplifying the cDNA region of the DREB1A gene [Kazuko Yamaguchi-Shinozaki and Kazuo Shinozaki: Plant Cell 6 : 251-264 (1994)] by performing a reverse transcription polymerase chain reaction (also referred to as RT-PCR). An example of a template mRNA that can be used for PCR herein is mRNA that is prepared from Arabidopsis plants inoculated and grown on solid media such as MS media [Murashige and Skoog: Physiol. Plant. 15: 473-497 (1962)] under aseptic conditions, and then exposed to dehydration stress (e.g., putting the plants under drought).

Furthermore, these genes are disclosed in JP Patent No. 3178672, and can be obtained according to the description given in this publication. In addition, the nucleotide sequences of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene are shown respectively in SEQ ID NOS: 1, 3, 5, 7, 9, 11, 13, 15, 17, 19, 21, 23, 25, and 27. In addition, the amino acid sequences of the proteins respectively encoded by DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene are respectively shown in SEQ ID NOS: 2, 4, 6, 8, 10, 12, 14, 16, 18, 20, 22, 24, 26, and 28. Furthermore, a recombinant vector containing DREB1A or DREB2A gene has been introduced into the *Escherichia coli* K-12 strain, and *Escherichia coli* containing DREB1A gene and *Escherichia coli* containing DREB2A gene were respectively deposited under accession nos. FERM P-16936 and FERM P-16937 with the International Patent Organism Depositary, the National Institute of Advanced Industrial Science and Technology (Tsukuba Central 6, 1-1-1 Higashi, Tsukuba, Ibaraki, Japan) on August 11, 1998. Furthermore, 1 to 1 alignment, common sequences, and homology % at the nucleotide sequence level between DREB1A as a standard and each one of DREB1B to DREB1F are shown in Fig. 2; 1 to 1 alignment, common sequences and homology% at the amino acid sequence level between DREB1A as a standard and each one of DREB1B to DREB1F are shown in Fig. 3; 1 to 1 alignment, common sequences and homology% at the nucleotide sequence level between DREB2A as a standard and each one of DREB2B to DREB2H are shown in Fig. 4; 1 to 1 alignment, common sequences, and homology% at the amino acid sequence level between DREB2A as a standard and each one of DREB2B to DREB2H are shown in Fig. 5. For this alignment, GENETYX-MAC version 12.0.0 was used as analysis software. In addition, the analyses of the nucleotide sequences, amino acid sequences and the expression of DREB1D to DREB1F, and DREB2C to DREB2H are described in Biochem. Biophys. Res. Comm, 290: 998-1009 (2002). To obtain the DREB gene of the invention of this application, this literature can be referred to.

According to sequence comparison at the nucleotide sequence level among DREB1A to DREB1F genes in Fig. 2, the lowest homology between DREB1A and each one of DREB1B to DREB1F is 54.7%. In addition, among DREB1B to DREB1F, the lowest homology is 51.2% between DREB1D and DREB1E. Furthermore among DREB1A to DREB1F, many common sequences are present in a sequence region corresponding to a sequence ranging approximately from nucleotide positions 100 to 400 of DREB1A. The lowest homology of a region corresponding to a nucleotide sequence ranging from positions 100 to 400 of DREB1A is approximately 65% between DREB1D and DREB1E.

Therefore, a DNA that comprises a nucleotide sequence having 50% or more homology with the nucleotide sequence of any one of DREB1A to DREB1F and is of a gene of DREB 1 family is described as the DNA encoding a transcription factor having functions to bind to a dehydration responsive element (DRE) and activate the transcription of a gene located downstream of the DRE. Among these DNAs, in particular, a DNA having a nucleotide sequence region that shares high homology with a nucleotide sequence region ranging from positions 100 to 400 of DREB1A, or with a nucleotide sequence region corresponding to the nucleotide sequence region ranging from positions 100 to 400 of DREB1A when the nucleotide sequence of any one of DREB1B to DREB1F is aligned with the nucleotide sequence of DREB1A by the above method is described. Specifically, such a DNA having a region that shares at least 60% and preferably 65% or more homology with that of any one of DREB1A to DREB1F is described as the DNA encoding a transcription factor having functions to bind to a dehydration responsive element (DRE) and activate the transcription of a gene located downstream of the DRE. Furthermore, a DNA containing at least the above nucleotide sequence region is described as the DNA encoding a transcription factor having functions to bind to a dehydration responsive element (DRE) and activate the transcription of a gene located downstream of the DRE.

According to sequence comparison at the amino acid level among DREB 1 A to DREB1F proteins in Fig. 2, the lowest homology between DREB1A and each one of DREB1B to DREB1F is 43.9%. In addition, among DREB1B to DREB1F, the lowest homology is 41.9% between DREB1D and DREB1E.

Hence, a DNA encoding a protein that belongs to the DREB family and comprises an amino acid sequence having 40% or more homology with the amino acid sequence of any one of DREB1A to DREB1F is described as the DNA encoding a transcription factor having functions to bind to a dehydration responsive element (DRE) and activate the transcription of a gene located downstream of the DRE. Among these DNAs, in particular a DNA encoding a protein having an amino acid sequence region that shares high homology with an amino acid sequence region ranging approximately from amino acid positions 31 to 120 of DREB1A protein or with an amino acid sequence region corresponding to the amino acid sequence region ranging from amino acid positions 31 to 120 of DREB1A when the amino acid sequence of any one of DREB1B to DREB1F proteins is aligned with the amino acid sequence of DREB1A protein by the above method is described. Specifically, such a DNA encoding a protein having the region that shares at least 60% and preferably 70% or more homology with that of any one of DREB1A to DREB1F is described as the DNA encoding a transcription factor having functions to bind to a dehydration responsive element (DRE) and activate the transcription of a gene located downstream of the DRE. Furthermore, a DNA encoding a protein containing at least the above amino acid sequence region is described as the DNA encoding a transcription factor having functions to bind to a dehydration responsive element (DRE) and activate the transcription of a gene located downstream of the DRE. Furthermore, among the amino acid sequences of DREB1A to DREB1F proteins, an amino acid sequence (MAARAHDVA) ranging from positions 85 to 93 and an amino acid sequence (ALRGRSACLNF) ranging from positions 95 to 105 of DREB1A protein are common sequences of DREB1A to DREB1F proteins. A DNA encoding a protein having the entirety of both common sequences, or a sequence derived from the common sequences by substitution, deletion, or addition of 1 or several amino acids is also described as the DNA encoding a transcription factor having functions to bind to a dehydration responsive element (DRE) and activate the transcription of a gene located downstream of the DRE.

According to sequence comparison at the nucleotide sequence level among DREB2A to DREB2H genes in Fig. 4, the lowest homology between DREB2A and each one of DREB2B to DREB2H is 39.4%. In addition, among DREB2B to DREB2H, the lowest homology is 38.4% between DREB2G and DREB2H. Furthermore, among DREB2A to DREB2H, many common sequences are present in a sequence region ranging approximately from nucleotide positions 180 to 400.

Hence, a DNA that comprises a nucleotide sequence having 50% or more homology with the nucleotide sequence of any one of DREB2A to DREB2H and is of a gene of DREB2 family is described as the DNA encoding a transcription factor having functions to bind to a dehydration responsive element (DRE) and activate the transcription of a gene located downstream of the DRE. Among these DNAs, in particular a DNA having a nucleotide sequence region that shares high homology with a nucleotide sequence region ranging from positions 180 to 400 of DREB2A or with a nucleotide sequence region corresponding to the nucleotide sequence region ranging from positions 180 to 400 of DREB2A when the nucleotide sequence of any one of DREB2B to DREB2H is aligned with the nucleotide sequence of DREB2A by the above method is described. Specifically, such a DNA having a region that shares at least 40% and preferably 50% or more homology with that of any one of DREB2A to DREB2H is described as the DNA encoding a transcription factor having functions to bind to a dehydration responsive element (DRE) and activate the transcription of a gene located downstream of the DRE. Furthermore, a DNA containing at least the above nucleotide sequence region is described as the DNA encoding a transcription factor having functions to bind to a dehydration responsive element (DRE) and activate the transcription of a gene located downstream of the DRE.

According to sequence comparison at the amino acid sequence level among DREB2A to DREB2H proteins in Fig. 5, the lowest homology between DREB2A and each one of DREB2B to DREB2H is 26.1 %. In addition, among DREB2B to DREB2H, the lowest homology is 21.5% between DREB2F and DREB2G.

Hence, a DNA encoding a protein belonging to the DREB2 family comprising an amino acid sequence having 20% or more homology with the amino acid sequence of any one of DREB2A to DREB2H is described as the DNA encoding a transcription factor having functions to bind to a dehydration responsive element (DRE) and activate the transcription of a gene located downstream of the DRE. Among these DNAs, in particular a DNA encoding a protein having an amino acid sequence region that shares high homology with an amino acid sequence region ranging approximately from amino acid positions 61 to 130 of DREB2A protein, or with an amino acid sequence region corresponding to the amino acid sequence region ranging from amino acid positions 61 to 130 of DREB2A when the amino acid sequence of any one of DREB2B to DREB2H proteins is aligned with the amino acid sequence of DREB2A protein by the above method is described. Specifically, such a DNA encoding a protein having a region that shares at least 20% and preferably 30% or more homology with that of any one of DREB2A to DREB2H is described as the DNA encoding a transcription factor having functions to bind to a dehydration responsive element (DRE) and activate the transcription of a gene located downstream of the DRE. Furthermore, a DNA encoding a protein containing at least the above amino acid sequence region is also described as the DNA encoding a transcription factor having functions to bind to a dehydration responsive element (DRE) and activate the transcription of a gene located downstream of the DRE. Furthermore, among the amino acid sequences of DREB2A to DREB2H proteins, an amino acid sequence (WGKWVAEIREP) ranging from positions 88 to 98 of DREB2A protein is a common sequence of DREB2A to DREB2H proteins. A DNA encoding a protein having the entire common sequence region or a sequence derived from the common sequence by substitution, deletion, or addition of 1 or several amino acids is also described as the DNA encoding a transcription factor having functions to bind to a dehydration responsive element (DRE) and activate the transcription of a gene located downstream of the DRE.

In addition, "family" means molecules belonging to a group of molecules relating to DREB1A to F and DREB2A to H molecular-systematically, and having a specific homology at the amino acid sequence level therewith, and includes those other than DREB1A to F and DREB2A to H.

Furthermore, Fig. 6 shows alignment at the nucleotide sequence level between DREB1A as a standard and each one of DREB1B to DREB1F, Fig. 7 shows alignment at the nucleotide sequence level between DREB2A as a standard and each one of DREB2B to DREB2H, Fig. 8 shows alignment at the amino acid sequence level between DREB1A as a standard and each one of DREB1B to DREB1F, and Fig. 9 shows alignment at the amino acid sequence level between DREB2A as a standard and each one of DREB2B to DREB2H. A DNA comprising any one of the DNAs hybridizing under stringent conditions to DNAs that consist of each common nucleotide sequence when the above DREB1A or DREB2A is used as a standard, a degenerate mutant of the sequence, a sequence having 80% or more homology with such sequence, and a DNA complementary to the sequence can be used as the DNA encoding a transcription factor having functions to bind to a dehydration responsive element (DRE) and activate the transcription of a gene located downstream of the DRE. Furthermore, a DNA encoding a protein having an amino acid sequence of any one of the common amino acid sequences when the above DREB1A or DREB2A is used as a standard, or an amino acid sequence derived from the common amino acid sequence by substitution, deletion, addition, or insertion of one or several amino acids, can also be used as the DNA encoding a transcription factor having functions to bind to a dehydration responsive element (DRE) and activate the transcription of a gene located downstream of the DRE.

Common sequences at the amino acid level among DREB1A to 1F, common sequences at the amino acid level among DREB2A to 2H, common sequences at the nucleotide level among DREB I A to 1F, and common sequences at the nucleotide sequence level among DREB2A to 2H are shown below.

### *DREB1A to 1F amino acid level:

In DREB1A, an amino acid at position 30 is A, amino acids at positions 34 to 36 are P, K, and K, respectively, amino acids at positions 38 to 40 are A, G and R, respectively, an amino acid at position 43 is F, amino acids at positions 45 to 49 are E, T, R, H, and P, respectively, amino acids at positions 51 to 53 are V, R and G, respectively, an amino acid at position 55 is R, an amino acid at position 57 is R, amino acids at positions 61 to 63 are K, W, and V, respectively, an amino acid at position 65 is E, amino acids at positions 67 to 69 are R, E, and P, respectively, an amino acid at position 74 is R, amino acids at positions 76 to 79 are W, L, G and T, respectively, an amino acid at position 82 is T, amino acids at positions 85 to 93 are M, A, A, R, A, H, D, V, and A, respectively, amino acids at positions 96 to 106 are A, L, R, G, R, S, A, C, L, N, and F, respectively, amino acids at positions 108 to 113 are D, S, A, W, R, and L, respectively, an amino acid at position 116 is P, an amino acid at position 124 is I, an amino acid at position 128 is A, amino acids at positions 130 to 132 are E, A, and A, respectively, an amino acid at position 135 is F, amino acids at positions 186 and 187 are A and E, respectively, an amino acid at position 190 is L, an amino acid at position 194 is P, and amino acids at positions 212 to 215 are S, L, W, and S, respectively.

### *DREB2A to 2H amino acid level:

In DREB2A, amino acids at positions 63 and 64 are K and G, respectively, amino acids at positions 68 to 71 are G, K, G, and G, respectively, an amino acid at position 72 is P, an amino acid at position 74 is N, amino acid at position 77 is C, amino acids at positions 81 to 85 are G, V, R, O, and R, respectively, amino acids at positions 87 to 97 are W, G, K, W, V, A, E, I, R, E, and P, respectively, amino acids at positions 103 to 106 are L, W, L, and G, respectively, an amino acid at position 108 is F, amino acids at positions 114 and 115 are A and A, respectively, amino acids at positions 117 to 119 are A, Y, and D, respectively, an amino acid at position 121 is A, amino acids at positions 126 and 127 are Y and G, respectively, an amino acid at position 130 is A, and amino acids at positions 132 and 133 are L and N, respectively.

### *DREB1A to 1F nucleotide level:

In DREB1A, a nucleotide at position 71 is A, a nucleotide at position 82 is A, a nucleotide at position 86 is T, nucleotides at positions 88 and 89 are G and C, respectively, a nucleotide at position 94 is A, both nucleotides at positions 100 and 101 are C, nucleotides at positions 103 to 107 are A, A, G, A, and A, respectively, a nucleotide at position 109 is C, nucleotides at positions 112 and 113 are G and C, respectively, both nucleotides at positions 115 and 116 are G, a nucleotide at position 119 is G, a nucleotide at position 121 is A, both nucleotides at positions 127 and 128 are T, nucleotides at positions 133 to 137 are G, A, G, A, and C, respectively, nucleotides at positions 139 to 143 are C, G, T, C, and A, respectively, both nucleotides at positions 145 and 146 are C, a nucleotide at position 149 is T, nucleotides at positions 151 to 158 are T, A, C, A, G, A, G, and G, respectively, a nucleotide at position 161 is T, a nucleotide at position 164 is G, a nucleotide at position 166 is C, nucleotides at positions 169 and 170 are A and G, respectively, a nucleotide at position 173 is A, a nucleotide at position 178 is G, both nucleotides at positions 181 and 182 are A, nucleotides at positions 184 to 188 are T, G, G, G, and T, respectively, a nucleotide at position 190 is T, nucleotides at positions 193 and 194 are G and A, respectively, a nucleotide at position 197 is T, a nucleotide at position 200 is G, nucleotides at positions 202 and 203 are G and A, respectively, both nucleotides at positions 205 and 206 are C, a nucleotide at position 208 is A, a nucleotide at position 212 is A, a nucleotide at position 215 is A, a nucleotide at position 221 is G, a nucleotide at position 224 is T, nucleotides at positions 226 to 228 are T, G, and G, respectively, a nucleotide at position 230 is T, both nucleotides at positions 232 and 233 are G, nucleotides at positions 235 and 236 are A and C, respectively, a nucleotide at position 238 is T, a nucleotide at position 241 is C, nucleotides at positions 244 and 245 are A and C, respectively, a nucleotide at position 247 is G, nucleotides at positions 250 and 251 are G and A, respectively, nucleotides at positions 253 to 257 are A, T, G, G, and C, respectively, nucleotides at positions 259 and 260 are G and C, respectively, nucleotides at positions 262 and 263 are C and G, respectively, respectively, nucleotides at positions 262 and 263 are C and G, respectively, nucleotides at positions 265 and 266 are G and C, respectively, nucleotides at positions 268 and 269 are C and A, respectively, nucleotides at positions 271 and 272 are G and A, respectively, nucleotides at positions 274 and 275 are G and T, respectively, nucleotides at positions 277 and 278 are G and C, respectively, a nucleotide at position 280 is G, a nucleotide at position 284 is T, nucleotides at positions 286 and 287 are G and C, respectively, nucleotides at positions 289 and 290 are C and T, respectively, nucleotides at positions 292 and 293 are C and G, respectively, both nucleotides at positions 295 and 296 are G, a nucleotide at position 299 is G, nucleotides at positions 301 and 302 are T and C, respectively, nucleotides at positions 304 and 305 are G and C, respectively, nucleotides at positions 307 to 309 are T, G, and T, respectively, a nucleotide at position 311 is T, both nucleotides at positions 313 and 314 are A, nucleotides at positions 316 to 318 are T, T, and C, respectively, a nucleotide at position 320 is C, nucleotides at positions 322 and 323 are G and A, respectively, nucleotides at positions 325 and 326 are T and C, respectively, nucleotides at positions 328 to 333 are G, C, T, T, G, and G, respectively, a nucleotide at position 335 is G, a nucleotide at position 338 is T, a nucleotide at position 340 is C, a nucleotide at position 344 is T, both nucleotides at positions 346 and 347 are C, a nucleotide at position 349 is G, a nucleotide at position 353 is C, a nucleotide at position 355 is A, a nucleotide at position 362 is C, a nucleotide at position 365 is A, nucleotides at positions 370 and 371 are A and T, respectively, nucleotides at positions 382 and 383 are G and C, respectively, a nucleotide at position 386 is C, nucleotides at positions 388 to 392 are G, A, A, G, and C, respectively, nucleotides at positions 394 and 395 are G and C, respectively, a nucleotide at position 399 is G, both nucleotides at positions 403 and 404 are T, a nucleotide at position 412 is G, nucleotides at positions 428 and 429 are C and G, respectively, a nucleotide at position 439 is G, a nucleotide at position 445 is G, a nucleotide at position 462 is G, both nucleotides at positions 483 and 484 are G, a nucleotide at position 529 is G, a nucleotide at position 533 is T, a nucleotide at position 536 is C, a nucleotide at position 545 is T, a nucleotide at position 550 is A, a nucleotide at position 554 is T, nucleotides at positions 556 and 557 are G and C, respectively, nucleotides at positions 559 and 560 are G and A, respectively, a nucleotide at position 562 is G, a nucleotide at position 569 is T, a nucleotide at position 572 is T, nucleotides at positions 575 and 576 are C and G, respectively, both nucleotides at positions 580 and 581 are C, a nucleotide at position 582 is G, nucleotides at positions 586 and 587 are G and T, respectively, a nucleotide at position 593 is T, nucleotides at positions 599 and 600 are G and A, respectively, a nucleotide at position 602 is A, a nucleotide at position 608 is A, nucleotides at positions 613 and 614 are G and A, respectively, a nucleotide at position 616 is G, a nucleotide at position 619 is G, nucleotides at positions 625 and 626 are G and A, respectively, a nucleotide at position 628 is G, a nucleotide at position 632 is T, nucleotides at positions 634 and 635 are T and C, respectively, a nucleotide at position 638 is T, nucleotides at positions 640 to 644 are T, G, G, A, and G, respectively, and a nucleotide at position 646 is T.

### *DREB2A to 2H nucleotide level:

In DREB2A, a nucleotide at position 181 is T, a nucleotide at position 184 is A, both nucleotides at positions 187 and 188 are A, nucleotides at positions 190 to 192 are G, G, and T, respectively, both nucleotides at positions 202 and 203 are G, nucleotides at positions 205 to 209 are A, A, A, G, and G, respectively, both nucleotides at positions 211 and 212 are G, both nucleotides at positions 214 and 215 are C, a nucleotide at position 218 is A, both nucleotides at positions 220 and 221 are A, a nucleotide at position 229 is T, a nucleotide at position 230 is G, a nucleotide at position 235 is T, both nucleotides at positions 241 and 242 are G, nucleotides at positions 244 and 245 are G and T, respectively, a nucleotide at position 248 is G, nucleotides at positions 250 and 251 are C and A, respectively, a nucleotide at position 254 is G, nucleotides at positions 259 to 263 are T, G, G, G, and G, respectively, nucleotides at positions 265 to 272 are A, A, A, T, G, G, G, and T, respectively, nucleotides at positions 274 and 275 are G and C, respectively, nucleotides at positions 277 to 281 are G, A, G, A, and T, respectively, a nucleotide at position 284 is G, nucleotides at positions 286 and 287 are G and A, respectively, both nucleotides at positions 289 and 290 are C, a nucleotide at position 299 is G, a nucleotide at position 308 is T, nucleotides at positions 310 to 314 are T, G, G, C, and T, respectively, both nucleotides at positions 316 and 317 are G, a nucleotide at position 320 is C, both nucleotides at positions 322 and 323 are T, a nucleotide at position 328 is A, a nucleotide at position 332 is C, a nucleotide at position 338 is A, nucleotides at positions 340 and 341 are G and C, respectively, nucleotides at positions 343 and 344 are G and C, respectively, nucleotides at positions 349 to 353 are G, C, T, T, and A, respectively, nucleotides at positions 355 and 356 are G and A, respectively, nucleotides at positions 361 and 362 are G and C, respectively, a nucleotide at position 365 is C, a nucleotide at position 374 is T, nucleotides at positions 376 and 377 are T and A, respectively, both nucleotides at positions 379 and 380 are G, nucleotides at positions 388 and 389 are G and C, respectively, a nucleotide at position 395 is T, both nucleotides at positions 397 and 398 are A, a nucleotide at position 401 is A, a nucleotide at position 554 is A, and a nucleotide at position 572 is T.

As long as a protein comprising an amino acid sequence encoding each of the above various genes has functions to bind to the DRE so as to activate the transcription of a gene located downstream of the DRE, a mutant gene other than those of the DREB1 or DREB2 family encoding a protein that comprises an amino acid sequence derived from the above amino acid sequence by a mutation such as deletion, substitution, or addition of at least 1 or more amino acids (plurality of amino acids, or several amino acids) is described as a gene equivalent to each of the above genes.

For example, a gene encoding a protein that comprises an amino acid sequence derived from one of these amino acid sequences by substitution of at least 1, preferably 1 to 160, more preferably 1 to 40, further more preferably 1 to 20, and most preferably 1 to 5 amino acids with (an)other amino acid(s) is also described, as long as the protein has functions to bind to the DRE and activate the transcription of a gene located downstream of the DRE.

Moreover, a DNA that is capable of hybridizing under stringent conditions to a DNA complementary to the DNA of each of the above various genes can also be used in the present invention as a gene equivalent to each of the above genes, as long as a protein encoded by the DNA has functions to bind to the DRE so as to activate the transcription of a gene located downstream of the DRE. Such stringent conditions comprise, for example, sodium concentration between 10 mM and 300 mM, or preferably between 20 mM and 100 mM, and temperatures between 25°C and 70°C, or preferably between 42°C and 55°C [Molecular Cloning (edited by Sambrook et al., (1989) Cold Spring Harbor Lab. Press, New York)].

Moreover, a mutant gene can be prepared according to a known technique such as the Kunkel method or the Gapped duplex method, or a method according thereto using, for example, a kit for mutagenesis (e.g., Mutant-K (TAKARA) and Mutant-G (TAKARA)) utilizing the site-directed mutagenesis method, or a LA PCR in vitro Mutagenesis series kit (TAKARA). Regarding the above mutagenesis methods, it is clear that persons skilled in the art can produce the above mutant genes without any special difficulties by referring to the nucleotide sequence of DREB gene to perform selection and the procedures according to the description in literature such as Molecular Cloning (edited by Sambrook et al. (1989) 15 Site-directed Mutagenesis of Cloned DNA, 15.3 to 15.113, Cold Spring Harbor Lab. Press, New York). Furthermore, regarding techniques (site-directed mutagenesis), by which substitution, deletion, insertion, or addition of one or more (1 or several or more nucleotides) nucleotides is artificially performed based on the nucleotide sequence of DREB gene, persons skilled in the art can obtain and utilize a mutant according to techniques described in Proc. Natl. Acad. Sci. U.S.A. 81 (1984) 5662-5666; WO85/00817, Nature 316 (1985) 601-605, Gene 34 (1985) 315-323; Nucleic Acids Res. 13 (1985) 4431-4442; Proc. Natl. Acad. Sci. U.S.A. 79 (1982) 6409-6413; Science 224 (1984) 1431-1433; or the like.

Furthermore, the DREB gene described herein also includes a nucleotide sequence (mutant) having 80% or more, preferably 90% or more, more preferably 94% or more, and most preferably 99% or more homology with the nucleotide sequence of each of the above DREB genes or each common nucleotide sequence thereof, as long as the mutant has functions to bind to the DRE and activate the transcription of a gene located downstream of the DRE. Here, such numerical values of homologies are calculated based on default parameter settings (initial settings) using a program for comparing nucleotide sequences, such as GENETYX-MAC version 12.0.0.

If such a mutant of a DNA comprising the nucleotide sequence of the DREB gene or a part thereof has activity to bind to the DRE and activate the transcription of a gene located downstream of the DRE, the mutant may be used in the present invention, and the strength of the activity is not specifically limited. Preferably, each mutant substantially has activity equivalent to the activity of the DNA comprising the nucleotide sequence or the part thereof to bind to the DRE and activate the transcription of a gene located downstream of the DRE. Here, the phrase, "substantially having activity equivalent to that of binding to the DRE and activating the transcription of a gene located downstream of the DRE" means that in the actual embodiment where the activity is utilized, activity is maintained to a degree such that almost the same use as that of the DNA or the part thereof is possible under the same conditions. In addition, the activity used herein means activity of, for example, plant cells or plants, preferably the cells or the plants of dicotyledon, more preferably the cells or the plants of chrysanthemum, and most preferably the cells or the plants of Lineker (*Chrysanthemum morifolium* cv. Lineker or *Dendranthema grandiflorum* cv. Lineker) of chrysanthemum cultivars. These activities can be measured according to the method disclosed in JP Patent No. 3178672.

Once the nucleotide sequence of DREB gene is determined, DREB gene can then be obtained by chemical synthesis, PCR using the cDNA or the genomic DNA of this gene as a template, or hybridization using a DNA fragment having the nucleotide sequence as a probe.

DREB gene is a gene encoding a protein that activates transcription. Thus, in a plant having the gene introduced therein, the thus expressed DREB protein acts so as to activate various genes, and the plant's own growth may be suppressed by the resulting increases in energy consumption or activation of metabolism. To prevent this from occurring, it is conceivable that a stress-responsive promoter be ligated upstream of DREB gene so as to cause the expression of DREB gene only when stress is provided. Examples of such a promoter are as follows:
rd29A gene promoter [Yamaguchi-Shinozaki, K. et al.: Plant Cell, 6: 251-264 (1994)], rd29B gene promoter [Yamaguchi-Shinozaki, K. et al.: Plant Cell, 6: 251-264 (1994)], rd17 gene promoter [Iwasaki, T. et al.: Plant Physiol., 115: 1287 (1997)], rd22 gene promoter [Iwasaki, T. et al.: Mol. Gen. Genet., 247: 391-398(1995)], DREB1A gene promoter [Shinwari, Z. K,. et al.: Biochem. Biophys. Res. Com. 250: 161-170 (1998)], cor6.6 gene promoter [Wang, H. et al.: Plant Mol. Biol. 28: 619-634 (1995)], cor15a gene promoter [Baker, S. S. et al.: Plant Mol. Biol. 24: 701-713 (1994)], erd1 gene promoter [Nakashima K. et al.: Plant J. 12: 851-861(1997)], and kin1 gene promoter [Wang, H. et al.: Plant Mol. Biol. 28: 605-617 (1995)].

However, as long as a promoter is stress responsive and functions within a plant cell or a plant, it is not limited to the above promoters. In addition, these promoters can be obtained by a PCR amplification reaction using primers designed based on the nucleotide sequence of a DNA containing the promoter and the genomic DNA as a template. Specifically, a promoter can be obtained by amplifying by polymerase chain reaction (PCR) the promoter region (a region from the translation initiation point of rd29A gene -215 to -145) [Kazuko Yamaguchi-Shinozaki and Kazuo Shinozaki: Plant Cell 6: 251-264 (1994)] of the rd29A gene, which is one type of dehydration-stress-resistance gene. An example of a template DNA that can be used for PCR is a genomic DNA of Arabidopsis, but use is not limited thereto.

An example of a gene used in the present invention wherein DREB gene is ligated to a stress-responsive promoter is rd29A-DREB1A. This gene is derived from a plant plasmid pBI29AP: DREB1A described in Example 5 of JP Patent No. 3178672, and is a stress-resistance gene that has also been reported by Kasuga et al's report [Nature Biotech., 17 287-291(1999)].

Also for such a promoter, in a manner similar to the case for the above DREB genes, various mutants can be used, as long as they possess promoter activity. Such a mutant can be prepared by persons skilled in the art without any special difficulties by referring to the nucleotide sequences described in literature concerning the various above promoters, as described also for the above DREB genes. Whether or not the mutant obtained as described above has activity as a promoter and whether or not the mutant substantially retains the promoter activity of a DNA containing the promoter or a part thereof can be confirmed by ligating useful DREB genes for expression within host cells according to the descriptions of the following examples, and then carrying out various forms of bioassay (e.g., in terms of salinity resistance, rooting ability, and prolonged life of cut flowers). Such methods can be appropriately conducted by persons skilled in the art.

Therefore, the above various stress-responsive promoters and various DREB genes can be appropriately combined, selected, and used according to the purpose of use in various plant cells or plants, so that activity can be confirmed.

Furthermore, a terminator ordering to terminate transcription can also be ligated downstream of DREB gene, if necessary. Examples of a terminator include a terminator derived from the cauliflower mosaic virus and a nopaline synthase gene terminator. However, examples of a terminator are not limited thereto, as long as they are known to function within a plant.

Furthermore, an intron sequence having a function to enhance gene expression, such as the intron of alcohol dehydrogenase (Adhl) of maize [Genes & Development 1: 1183-1200 (1987)], can be introduced between a promoter sequence and DREB gene, if necessary.

### (2) DNA strand for production of transformed plant

To produce the transformed plant in the method of the present invention, a DNA strand, which comprises the DNA as described herein wherein a stress-responsive promoter and DREB gene are linked, is used. In a specific form of the DNA strand, for example, the DNA having a stress-responsive promoter ligated to DREB gene may be inserted as a component into a plasmid or a phage DNA.

The DNA strand can further contain components such as a translation enhancer, a translation termination codon, and a terminator. As a translation enhancer, a translation termination codon, or a terminator, those known can be appropriately combined and used. Examples of a translation enhancer of viral origin include the sequences of tobacco mosaic virus, alfalfa mosaic virus RNA 4, bromo mosaic virus RNA 3, potato virus X, and tobacco etch virus [Gallie et al., Nuc. Acids Res., 15 (1987) 8693-8711]. Moreover, examples of a translation enhancer of plant origin include a sequence derived from β-1,3 glucanase (Glu) of soybean [written by Isao Ishida and Norihiko Misawa, edited by Kodansha Scientific, Cell Technology, Introduction to Experimental Protocols (Saibo-ko-gaku jikken so-sa nyumon), KODANSHA, p.119, 1992], and a sequence derived from a ferredoxin-binding subunit (PsaDb) of tobacco [Yamamoto et al., J. Biol. Chem., 270 (1995) 12466-12470]. Examples of a translation termination codon include sequences such as TAA, TAG, and TGA [described in the above-mentioned Molecular Cloning]. Examples of a terminator include the terminator of nos gene and the terminator of ocs gene [Annu. Rev. Plant Physiol. Plant Mol. Biol., 44 (1993) 985-994, "Plant genetic transformation and gene expression; a laboratory manual" as mentioned above]. Furthermore, it has been reported that activity can be enhanced by lining up and linking several 35S enhancer regions identified as transcription enhancers in a promoter [Plant Cell, 1 (1989) 141-150]. These regions can also be used as a part of the DNA strand. Each of these various components is preferably incorporated operably into the DNA strand so that each component can function depending on the properties thereof. Persons skilled in the art can appropriately carry out such operations.

The above DNA strand can be easily produced by persons skilled in the art using techniques generally used in the field of genetic engineering. Moreover, the DNA strand is not limited to those isolated from natural supply sources, and may be an artificial construct, as long as it has the above-mentioned structure. The DNA strand can be obtained by synthesizing it according to a known and generally used method of synthesizing nucleic acids.

### (3) Transformation of plant

By the transformation of a host using the gene obtained in (1) above, and the culture or cultivation of the obtained transformant, a protein regulating the transcription of a gene located downstream of a stress-responsive element can be expressed, and a transformed plant having improved propagation efficiency of plant seedlings and improved vase life can be prepared.

The above DNA strand after transformation can be present in microorganisms (particularly, bacteria), phage particles, or plants, while being inserted in a plasmid, a phage, or a genomic DNA. Here, examples of bacteria typically include *Escherichia coli* and Agrobacterium, but are not limited thereto.

In a preferred embodiment of the present invention, the DNA strand described herein is introduced in a plant in a form wherein the DNA disclosed herein (promoter), a translation enhancer, a structural gene DNA, a translation termination codon, a terminator, and the like are integrally bound and this integrated combination thereof is inserted in the genome, so that the structural gene for the expression of a protein can be stably expressed in the plant.

Preferred examples of a host include cells of monocotyledons such as rice, wheat, corn, onions, lilies, and orchids, and cells of dicotyledons such as soybean, rapeseeds, tomatoes, potatoes, chrysanthemums, roses, carnations, petunias, baby's-breath, and cyclamens. Particularly preferred specific examples include the plant cells of 3 important cut flowers with large worldwide production amounts, turn volumes, and amounts of consumption: chrysanthemums, carnations and roses. Also particularly preferred are the plant cells of clones such as petunias whose production amounts, turn volumes, and amounts of consumption are growing drastically across the globe in recent years. In addition, examples of a specific plant material include vegetative points, shoot primordia, meristematic tissues, laminae, stem pieces, root pieces, tuber pieces, petiole pieces, protoplasts, calli, anthers, pollens, pollen tubes, flower stalk pieces, scape pieces, petals, and sepals.

As a method of introducing a foreign gene into a host, various methods that have been previously reported and established can be appropriately utilized. Preferred examples of such a method include a biological method using, for example, a virus or the Ti plasmid or the Ri plasmid of Agrobacterium as a vector, and a physical method involving introduction of a gene by electroporation, polyethylene glycol, particle gun, microinjection [the aforementioned "Plant genetic transformation and gene expression; a laboratory manual"], silicon nitride whisker, silicon carbide whisker [Euphytica 85 (1995) 75-80; In Vitro Cell. Dev. Biol. 31 (1995) 101-104; Plant Science 132 (1998) 31-43] or the like. Persons skilled in the art can appropriately select and use the introduction method.

Furthermore, by the regeneration of a plant cell transformed with the DNA strand as described herein, a transformed plant wherein the introduced gene is expressed within the cell can be produced. Persons skilled in the art can easily conduct such a procedure by a generally known method of regenerating plants from plant cells. Regarding regeneration of plants from plant cells, for example, see literature such as [Manuals for Plant Cell Culture (Shokubutsu saibo-baiyo manual)], and [edited and written by Yasuyuki Yamada, Kodansha Scientific, 1984].

In general, a gene introduced into a plant is incorporated into the genome of a host plant. At this time, a phenomenon referred to as position effect is observed, wherein a different position on the genome to which a gene is introduced leads to a different expression of the transgene. The transformant wherein a transgene is expressed more strongly than others can be selected by assaying mRNA levels expressed in the host plant by the Northern method using the DNA fragment of the transgene as a probe.

The incorporation of a target gene into a transformed plant, into which the gene used in the present invention has been introduced, can be confirmed by extracting DNA from these cells and tissues according to any standard method, and detecting the introduced gene using the known PCR method or Southern analysis.

### (4) Transformed plant obtainable by the method of the present invention

The method of the present invention provides a transformed plant, which contains a gene wherein a DNA encoding a protein that binds to a stress-responsive element contained in a stress-responsive promoter and regulates the transcription of a gene located downstream of the element is ligated downstream of the stress-responsive promoter, and has improved rooting efficiency and/or prolonged vase life. Examples of a plant include monocotyledons such as rice, wheat, corn, onions, lilies, and orchids, and dicotyledons such as soybean, rapeseeds, tomatoes, potatoes, chrysanthemums, roses, carnations, petunias, baby's-breath and cyclamens. Particularly preferred specific examples include 3 important cut flowers with large worldwide production amounts, turn volumes and amounts of consumption: chrysanthemums, carnations and roses. Also particularly preferred are the clones such as petunias whose production amounts, turn volumes and amounts of consumption are growing drastically across the globe. The method of the present invention also provides the scions of the transformed plants of the above plants having improved propagation efficiency and rooting efficiency compared with those of non-transformed plants, and the cut flowers of the transformed plants of the above plants having improved vase life (prolonged life of cut flowers) compared with those of non-transformed plants. Here "scions" mean branches, treetops, stems, leaves, and the like that are cut from plants and then planted for cutting. "Cut flowers" mean flowers cut from plants with branches and stems uncut.

### (5) Scion propagation efficiency test and vase life test

The transformed plant obtained by the method of the present invention has improved efficiency of propagation using scions, rooting efficiency, and vase life (prolonged life of cut flowers) compared with the case of non-transformed plants.

The efficiency of propagation using scions, rooting efficiency, and vase life (prolonged life of cut flowers) of a transformed plant can be evaluated by measuring efficiencies under the same conditions as those employed for plant production. For example, the efficiency of propagation using scions or the rooting efficiency of chrysanthemums can be evaluated by planting scions in soil for scions and examining the growing conditions 2 to 4 weeks later, and the growth of the same can be evaluated by potting the plants and measuring the stem lengths or the like. Vase life can be evaluated by carrying out approximately 4 weeks of long-day cultivation after potting, followed by approximately 8 weeks of short-day cultivation, so as to cause the plants to flower, cutting chrysanthemums, allowing the plants to stand in the dark for 1 day, arranging them in water, and then observing their conditions thereafter. For the general cultivation methods for chrysanthemums, see "revised version of New Techniques for Cut Flower Cultivation, Chrysanthemum" ("Kiribanasaibai-no-shingijutsu, Kaitei, Kiku," edited by Keiichi Funakoshi, SEIBUNDO SHINKOSHA, 1989).

### EFFECT OF THE INVENTION

As shown in Examples, a plant that has been transformed using a gene (stress-resistance gene) wherein a DNA encoding a protein that binds to a dehydration responsive element (DRE) and regulates the transcription of a gene located downstream of the DRE is ligated downstream of a stress-responsive promoter, has improved rooting efficiency and/or prolonged vase life compared with those of non-transformed plants. In addition, the transformed plant grows well after rooting. Hence, the method of introducing DREB gene into a plant of the present invention is useful in developing a plant having enhanced efficiency of propagation by cutting, enhanced rooting efficiency, and prolonged vase life.

### DETAILED DESCRIPTION OF THE PREFERRED EMBODIMENTS

The present invention will be described by examples below, but the present invention is not specifically limited by these examples.

### [Example 1] Preparation of Chrysanthemum plant expressing DREB1A gene

The rd29A-DREB1A expression vector described in Kasuga et al's report [Nature Biotech., 17 (1999) 287-291] is shown in Fig. 1. This vector was introduced into the *Agrobacterium tumefaciens* AGL0 strain by the electroporation method. The *Agrobacterium tumefaciens* AGL0 strain containing rd29A-DREB1A was inoculated into 3 ml of the following YEB-Km medium. After 16 hours of culture at 28°C in the dark, cells were collected by centrifugation, and were then suspended in 10 ml of the following medium for infection. The suspension was used as a solution for infection. The medium compositions of the YEB-Km medium and the medium for infection are as follows.
YEB-Km medium: 5 g/l beef extract, 1 g/l yeast extract, 5 g/l peptone, 5 g/l sucrose, 2 mM magnesium sulfate (pH 7.2), and 50 mg/l kanamycin (Km)
Medium for infection: inorganic salt and vitamins in a half concentration of a MS [Murashige & Skoog, Physiol. Plant., 15 (1962) 473-497] medium, 15 g/l sucrose, 10 g/l glucose, and 10 mM MES (pH 5.4)

The leaves of germ-free Lineker plants, *Chrysanthemum morifolium* cv. Lineker or *Dendranthema grandiflorum* cv. Lineker, which were Chrysanthemum cultivars, were cut 5 to 7 mm square, and then immersed for 10 minutes in the solution for infection with the agrobacteria, into which the rd29A-DREB1A expression vector had been introduced. After excessive solution for infection had been wiped off on filter paper, transplantation into the following co-culture medium was performed, followed by culture at 25°C in the dark. After 3 days of culture, cultured cells were transplanted onto the following selection medium, and then cultured for 3 weeks, thereby obtaining Km-resistant calli. Culture was conducted on the selection medium under conditions of 25°C, 16 hours of illumination (light density: 32 µE/m²s)/8 hours of no illumination.

Co-culture medium: MS medium with inorganic salt and vitamins, 30g/l sucrose, 1 mg/l naphthalenacetic acid, 2 mg/l benzyladenine, 8 g/l agar, 5 mM MES (pH 5.8), and 200 µM acetosyringone
Selection medium: MS medium with inorganic salt and vitamins, 30 g/l sucrose, 1 mg/l naphthalenacetic acid, 2 mg/l benzyladenine, 8 g/l agar, 5 mM MES (pH 5.8), 25 mg/l kanamycin (Km), and 300 mg/l cefotaxime

Plants were regenerated from the obtained Km-resistant calli in the selection media containing Km. Furthermore, the plants were grown on media for promoting rooting that had been prepared by removing plant-growth-regulating substances (naphthalenacetic acid and benzyladenine) from the selection media in order to promote rooting.

Individual plants containing DREB gene were detected from the plants that had grown by performing PCR, and then it was confirmed that the plants that had regenerated were transformants. As primers for specifically amplifying a characteristic sequence of DREB gene, GAGTCTTCGGTTTCCTCA (SEQ ID NO: 29) and CGATACGTCGTCATCATC (SEQ ID NO: 30) were used. PCR reaction was performed under conditions of heating at 94°C for 5 minutes; 30 cycles of 94°C (30 seconds), -55°C (1 minute), and -72°C (1 minute); and was finally conducted reaction at 72°C for 10 minutes. In this reaction, Taq polymerase (manufactured by TAKARA SHUZO) was used as an enzyme.

Thus, 13 lines of chrysanthemum having the gene introduced therein were obtained.

### [Example 2] Salinity tolerance test

The apical buds that had developed 2 to 3 leaves of all the Lineker non-transformants and the Lineker transformants obtained in Example 1 were placed on the following growth media (in vitro) variously supplemented with 0.1, 0.2, and 0.4 M NaCl. Two weeks later, rooting was observed. With 0.2 M NaCl, rooting became unobservable in those buds to which no rd29A-DREB1A gene had been introduced, but rooting was observed in all of buds to which DREB gene had been introduced, excluding a line 14. Even with 0.4 M NaCl, rooting was observed in a line 9. The results for the non-transformants, and lines 9 and 10, are shown in Table 1.

Growth medium: MS medium with inorganic salt and vitamins, 30 g/l sucrose, and 5 mM MES (pH 5.8)

**Table 1 Salt tolerance test**

| Line No. | Added salt concentration (M) | | | |
|---|---|---|---|---|
| | 0 | 0.1 | 0.2 | 0.4 |
| 9 | + | + | + | + |
| 10 | + | + | + | - |
| Non-transformant | + | + | - | - |

### [Example 3] Propagation using scions and the following growth test

The Lineker non-transformants and lines 9 and 10 of the Lineker transformants obtained in Example 1 were acclimatized in a greenhouse, thereby producing mother plants to obtain scions. Twenty scions were obtained from each line, planted in sufficiently-moistened soil for rooting (Akadama soil : Kanuma soil = 1:1), covered with moisture-retaining covers having air permeability, and then cultivated within a greenhouse. Twenty-one days later, plants were harvested so as not to damage the roots from the soil for rooting, and then rooting conditions were observed. The plants were classified in descending order from high to low rooting levels (high, moderate, low, and none (no rooting was observed)), and the number of scions was recorded. The results are shown Table 2 below and in Fig. 10. Surprisingly, rooting ability was significantly improved in lines 9 and 10 to which the rd29A-DREB1A gene had been introduced, compared with that of the Lineker non-transformants.

**Table 2 Rooting ability test upon scion production**

| Line No. | Rooting conditions (number of scions) | | | | Total |
|---|---|---|---|---|---|
| | High | Moderate | Low | None | |
| 9 | 4 | 10 | 5 | 1 | 20 |
| 10 | 6 | 7 | 6 | 1 | 20 |
| Non-transformant | 1 | 8 | 7 | 4 | 20 |

Moreover, 18 to 20 scions were separately obtained by a method similar to the above method. Ten scions showing good rooting (high and moderate according to the above classification) were selected from the scions, and then planted in vinyl pots. Stem length was measured and recorded to study the following growth, and the results are shown in Fig. 11. As shown this figure, compared with the Lineker non-transformants, lines 9 and 10 to which rd29A-DREB1A gene had been introduced showed not only good rooting ability, but also good growth thereafter.

### [Example 4] Vase life test

Ten individual plants of the Lineker non-transformants and the same of the lines 9 and 10 of the Lineker transformants obtained in Example 3 were then cultivated with long-day conditions (a light period of 18 hours and a dark period of 6 hours) for 4 weeks, and then cultivated with short-day conditions (a light period of 10 hours and a dark period of 14 hours) to cause them to flower. After they had developed 4 to 5 flowers on top, the above ground portions were cut. Cut flowers were arranged in buckets containing tap water, and then stored in a cool and dark place for 2 hours and 30 minutes. Subsequently, the cut flowers were allowed to stand in corrugated cardboard containers for delivery for 17 hours at room temperature, and then arranged in tap water. Vase life test was then conducted. Under conditions employed herein, the cut flowers were allowed to stand in a place where indoor fluorescent lamps were kept on for 11 hours and 30 minutes, while exchanging tap water used for arranging the cut flowers every 2 to 3 days.

Approximately 2 weeks after the start of the vase life test, no differences were found between the Lineker non-transformants and the Lineker transformants. However, 16 days later, rooting from stems several centimeters above the cut end was observed in both transformed lines. Twenty-two days later, rooting could be observed in most plants of the transformed lines, whereas no rooting was observed in the non-transformed lines (Fig. 12 and Table 3). Thereafter, compared with plants showing no rooting, it was observed that plants showing rooting were clearly exhibiting good plant conditions (in terms of vigor and wilting in flowers, stems, and leaves) and had prolonged vase life (Table 4).

**Table 3 Rooting conditions upon vase life test**

| Line No. | Number of plants showing rooting | | | | Total |
|---|---|---|---|---|---|
| | Days after the start of test (day) | 1 | 16 | 22 | |
| 9 | | 0 | 8 | 8 | 10 |
| 10 | | 0 | 2 | 9 | 10 |
| Non-transformant | | 0 | 0 | 0 | 10 |

**Table 4 Cut flower conditions on day 22 after the start of vase life test (Number of plants)**

| Line No. | Flower conditions * 1 | | Stem/Leaf conditions *2 | | Total |
|---|---|---|---|---|---|
| | Good | Poor | Good | Poor | |
| 9 | 8 | 2 | 8 | 2 | 10 |
| 10 | 9 | 1 | 9 | 1 | 10 |
| Non-transformant | 0 | 10 | 0 | 10 | 10 |

| | | | | | |
|---|---|---|---|---|---|
| All the plants exhibiting good conditions had rooted. | | | | | |

### Sequence Listing Free Text

SEQ ID NO: 29: primer
SEQ ID NO: 30: primer

## Claims

1. A method of improving rooting efficiency of a plant and/or prolonging vase life comprising transforming a plant
using a gene wherein a DNA encoding a protein that binds to a stress-responsive element contained in a stress-responsive promoter and activates the transcription of a gene located downstream of the element is ligated downstream of the stress-responsive promoter, and
wherein the DNA encoding a protein that binds to a stress-responsive element and activates the transcription of a gene located downstream of the element is at least one gene selected from the group consisting of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene.

2. The method of claim 1, wherein the DNA encoding a protein that binds to a stress-responsive element and regulates the transcription of a gene located downstream of the element is at least one DNA selected from the group consisting of:
(a) a DNA comprising a nucleotide sequence having at least 80% or more homology with the nucleotide sequence of a DNA of at least one of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene, and encoding a protein having activity to bind to a stress-responsive element and regulate the transcription of a gene located downstream of the element; and
(b) a DNA hybridizing under stringent conditions to a DNA complementary to a DNA of at least one of DREB1A gene, DREB1B gene, DREB1C gene, DREB1D gene, DREB1E gene, DREB1F gene, DREB2A gene, DREB2B gene, DREB2C gene, DREB2D gene, DREB2E gene, DREB2F gene, DREB2G gene, and DREB2H gene, and encoding a protein having activity to bind to a stress-responsive element and regulate the transcription of a gene located downstream of the element.

3. The method of claim 1 or 2, wherein the stress-responsive promoter is at least one promoter selected from the group consisting of rd29A gene promoter, rd29B gene promoter, rd17 gene promoter, rd22 gene promoter, DREB1A gene promoter, cor6.6 gene promoter, cor15a gene promoter, erd 1 gene promoter, and kin 1 gene promoter.

4. The method of any one of claims 1 to 3, wherein the DNA of a stress-responsive promoter is at least one DNA selected from the group consisting of:
(a) a DNA comprising a nucleotide sequence having at least 80% or more homology with the nucleotide sequence of a DNA of at least one of rd29A gene promoter, rd29B gene promoter, rd17 gene promoter, rd22 gene promoter, DREB1A gene promoter, cor6.6 gene promoter, cor15a gene promoter, erd1 gene promoter, and kin1 gene promoter, and having activity as the DNA of the stress-responsive promoter; and
(b) a DNA hybridizing under stringent conditions to a DNA complementary to a DNA of at least one of rd29A gene promoter, rd29B gene promoter, rd17 gene promoter, rd22 gene promoter, DREB1A gene promoter, cor6.6 gene promoter, cor15a gene promoter, erd1 gene promoter, and kin1 gene promoter, and having activity as the DNA of the stress-responsive promoter.

## Patentansprüche

1. Verfahren zur Verbesserung der Bewurzelungseffizienz einer Pflanze und/oder zur Verlängerung der Haltbarkeit in der Vase, umfassend die Transformation einer Pflanze unter Verwendung eines Gens, worin eine DNA, die ein Protein kodiert, das an ein in einem Stress-abhängigen Promotor enthaltenes Stress-abhängiges Element bindet und die Transkription eines Gens aktiviert, das stromabwärts des Elements angeordnet ist, stromabwärts von dem Stress-abhängigen Promotor ligiert ist und
wobei die DNA, die ein Protein kodiert, das an ein Stress-abhängiges Element bindet und die Transkription eines Gens aktiviert, das stromabwärts des Elements angeordnet ist, mindestens ein Gen ist, das ausgewählt ist aus der Gruppe bestehend aus DREB1A-Gen, DREB1B-Gen, DREB1C-Gen DREB1D-Gen, DREB1E-Gen, DREB1F-Gen, DREB2A-Gen, DREB2B-Gen, DREB2C-Gen, DREB2D-Gen, DREB2E-Gen, DREB2F-Gen, DREB2G-Gen und DREB2H-Gen.

2. Verfahren gemäß Anspruch 1, wobei die DNA, die ein Protein kodiert, das an ein Stress-abhängiges Element bindet und die Transkription eines Gens aktiviert, das stromabwärts des Element angeordnet ist, mindestens eine DNA ist, die ausgewählt ist aus der Gruppe bestehend aus:
(a) einer DNA, umfassend eine Nukleotidsequenz, die mindestens 80 % oder mehr Homologie zur Nukleotidsequenz einer DNA von mindestens einem der Gene DREB1A-Gen, DREB1B-Gen, DREB1C-Gen DREB1D-Gen, DREB1E-Gen, DREB1F-Gen, DREB2A-Gen, DREB2B-Gen, DREB2C-Gen, DREB2D-Gen, DREB2E-Gen, DREB2F-Gen, DREB2G-Gen und DREB2H-Gen aufweist und ein Protein kodiert, das Aktivität zur Bindung an ein Stress-abhängiges Element besitzt und die Transkription eines Gens reguliert, das stromabwärts des Elements angeordnet ist, und
(b) einer DNA, die unter stringenten Bedingungen mit einer DNA hybridisiert, die komplementär zu einer DNA von mindestens einem der Gene DREB1A-Gen, DREB1B-Gen, DREB1C-Gen DREB1D-Gen, DREB1E-Gen, DREB1F-Gen, DREB2A-Gen, DREB2B-Gen, DREB2C-Gen, DREB2D-Gen, DREB2E-Gen, DREB2F-Gen, DREB2G-Gen und DREB2H-Gen ist und ein Protein kodiert, dass Aktivität zur Bindung an ein Stress-abhängiges Element besitzt und die Transkription eines Gens reguliert, das stromabwärts des Elements angeordnet ist.

3. Verfahren gemäß Anspruch 1 oder.2, wobei der Stressabhängige Promotor mindestens ein Promotor ist, der ausgewählt ist aus der Gruppe bestehend aus rd29A-Genpromotor, rd29B-Genpromotor, rd17-Genpromotor, rd22-Genpromotor, DREB1A-Genpromotor, cor6.6-Genpromotor, cor15a-Genpromotor, erdl-Genpromotor und kinl-Genpromotor.

4. Verfahren gemäß einem der Ansprüche 1 bis 3, wobei die DNA eines Stress-abhängigen Promotors mindestens eine DNA ist, die ausgewählt ist aus der Gruppe bestehend aus
(a) einer DNA, umfassend eine Nukleotidsequenz, die mindestens 80 % oder mehr Homologie zur Nukleotidsequenz einer DNA von mindestens einem der Genpromotoren rd29A-Genpromotor, rd29B-Genpromotor, rd17-Genpromotor, rd22-Genpromotor, DREB1A-Genpromotor, cor6.6-Genpromotor, cor15a-Genpromotor, erdl-Genpromotor und kinl-Genpromotor aufweist und Aktivität als die DNA des Stress-abhängigen Promotors besitzt und
(b) einer DNA, die unter stringenten Bedingungen mit einer DNA hybridisiert, die komplementär zu einer DNA von mindestens einem der Genpromotoren rd29A-Genpromotor, rd29B-Genpromotor, rd17-Genpromotor, rd22-Genpromotor, DREB1A-Genpromotor, cor6.6-Genpromotor, cor15a-Genpromotor, erdl-Genpromotor und kinl-Genpromotor ist und Aktivität als die DNA des Stress-abhängigen Promotors besitzt.

## Revendications

1. Procédé d'amélioration de l'efficacité de l'enracinement d'une plante et/ou de prolongation de la durée de vie en vase comprenant la transformation d'une plante
en utilisant un gène dans lequel un ADN codant pour une protéine qui se lie à un élément de réponse au stress contenu dans un promoteur sensible au stress et active la transcription d'un gène situé en aval de l'élément est ligaturé en aval du promoteur sensible au stress, et
dans lequel l'ADN codant pour une protéine qui se lie à un élément de réponse au stress et active la transcription d'un gène situé en aval de l'élément est au moins un gène choisi dans le groupe constitué du gène DREB1A, du gène DREB1B, du gène DREB1C, du gène DREB1D, du gène DREB1E, du gène DREB1F, du gène DREB2A, du gène DREB2B, du gène DREB2C, du gène DREB2D, du gène DREB2E, du gène DREB2F, du gène DREB2G, et du gène DREB2H.

2. Procédé selon la revendication 1, dans lequel l'ADN codant pour une protéine qui se lie à un élément de réponse en stress et régule la transcription d'un gène situé en aval de l'élément est au moins un ADN choisi dans le groupe constitué de :
(a) un ADN comprenant une séquence nucléotidique ayant au moins 80 % d'homologie ou plus avec la séquence nucléotidique d'un ADN d'au moins l'un du gène DREB1A, du gène DREB1B, du gène DREB1C, du gène DREB1D, du gène DREB1E, du gène DREB1F, du gène DREB2A, du gène DREB2B, du gène DREB2C, du gène DREB2D, du gène DREB2E, du gène DREB2F, du gène DREB2G, et du gène DREB2H, et codant pour une protéine ayant une activité pour se lier à un élément de réponse au stress et régule la transcription d'un gène situé en aval de l'élément ; et
(b) un ADN s'hybridant dans des conditions stringentes à un ADN complémentaire d'un ADN d'au moins l'un du gène DREB1A, du gène DREB1B, du gène DREB1C, du gène DREB1D, du gène DREB1E, du gène DREB1F, du gène DREB2A, du gène DREB2B, du gène DREB2C, du gène DREB2D, du gène DREB2E, du gène DREB2F, du gène DREB2G, et du gène DREB2H, et codant pour une protéine ayant une activité pour se lier à un élément de réponse au stress et régule la transcription d'un gène situé en aval de l'élément.

3. Procédé selon la revendication 1 ou 2, dans lequel le promoteur sensible au stress est au moins un promoteur choisi dans le groupe constitué du promoteur du gène rd29A, du promoteur du gène rd29B, du promoteur du gène rd17, du promoteur du gène rd22, du promoteur du gène DREB1A, du promoteur du gène cor6.6, du promoteur du gène cor15a, du promoteur du gène erd 1, et du promoteur du gène kin 1.

4. Procédé selon l'une quelconque des revendications 1 à 3, dans lequel l'ADN d'un promoteur sensible au stress est au moins un ADN choisi dans le groupe constitué de :
(a) un ADN comprenant une séquence nucléotidique ayant au moins 80 % d'homologie ou plus avec la séquence nucléotidique d'un ADN d'au moins l'un du promoteur du gène rd29A, du promoteur du gène rd29B, du promoteur du gène rd17, du promoteur du gène rd22, du promoteur du gène DREB1A, du promoteur du gène cor6.6, du promoteur du gène cor15a, du promoteur du gène erd 1, et du promoteur du gène kin 1, et ayant une activité comme l'ADN du promoteur sensible au stress ; et
(b) un ADN s'hybridant dans des conditions stringentes à un ADN complémentaire d'un ADN d'au moins l'un du promoteur du gène rd29A, du promoteur du gène rd29B, du promoteur du gène rd17, du promoteur du gène rd22, du promoteur du gène DREB1A, du promoteur du gène cor6.6, du promoteur du gène cor15a, du promoteur du gène erd 1, et du promoteur du gène kin 1, et ayant une activité comme l'ADN du promoteur sensible au stress.
